# EUROPEAN PATENT APPLICATION

(11) **EP 3 124 487 A1**
(43) Date of publication of application: **01.02.2017**
(21) Application number: 15768768.2
(22) Date of filing: 23.03.2015
(51) Int. Cl.: C07D 471/04, A61K 31/4439, A61K 31/4709, A61K 31/502, A61K 31/52, A61P 1/04, A61P 7/06, A61P 11/06, A61P 17/00, A61P 17/06, A61P 21/04, A61P 37/00, A61P 37/06, A61P 37/08, A61P 43/00, C07D 487/04, C07D 519/00

(54) **HETEROCYCLIC COMPOUND**

(30) Priority: 24.03.2014 JP 2014060927
(71) Applicant: Takeda Pharmaceutical Company Limited, Osaka-shi, Osaka 541-0045 (JP)
(72) Inventor: TSUKAMOTO, Tetsuya, Izumiotsu-shi Osaka 595-0025 (JP); NAKADA, Yoshihisa, Fujisawa-shi Kanagawa 251-0012 (JP); MOCHIZUKI, Michiyo, Fujisawa-shi Kanagawa 251-0012 (JP); TAKAI, Takafumi, Fujisawa-shi Kanagawa 251-0012 (JP); YUKAWA, Tomoya, Fujisawa-shi Kanagawa 251-0012 (JP); SHIOKAWA, Zenyu, Fujisawa-shi Kanagawa 251-0012 (JP); KATOH, Taisuke, Fujisawa-shi Kanagawa 251-0012 (JP); SETOH, Masaki, Fujisawa-shi Kanagawa 251-0012 (JP); SATO, Ayumu, Fujisawa-shi Kanagawa 251-0012 (JP); YUKAWA, Takafumi, Fujisawa-shi Kanagawa 251-0012 (JP)
(74) Representative: Held, Stephan
(86) International application number: PCT/JP2015/058778
(87) International publication number: WO 2015/146929

(57) **Abstract**

Provided is a compound having a superior PKC inhibitory action, and useful as a prophylactic or therapeutic agent for immune diseases, inflammatory diseases and the like, or a salt thereof. A compound represented by the formula (I): 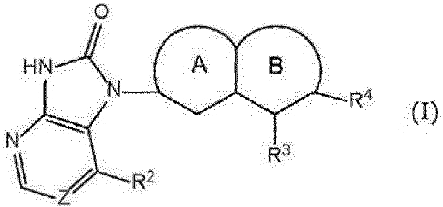 wherein each symbol is as described in the DESCRIPTION, or a salt thereof has a superior PKC inhibitory action, and is useful as a prophylactic or therapeutic agent for immune diseases, inflammatory diseases and the like.

## Description

### [Technical Field]

The present invention relates to a heterocyclic compound having a protein kinase C (sometimes to be abbreviated as "PKC" in the present specification) inhibitory action, and useful for the treatment of immune diseases, inflammatory diseases or the like, a pharmaceutical composition containing same or the like.

### (Background of the Invention)

In the immune system, T cell plays an important role as a part of the acquired immune mechanism to eliminate foreign antigens such as virus, bacterium or the like and cancer. On the other hand, it is known that excess activation of T cell is involved in the onset of autoimmune diseases and causes harmful reaction on the human body such as rejection on transplantation or the like. Therefore, T cell is considered a promising treatment target for these diseases (non-patent document 1). This is also evident from the fact that calcineurin inhibitors (Tacrolimus and Cyclosporin A) which are the medicaments that suppress activation of T cell, have been approved as a prophylactic or therapeutic drug for rejection in organ transplantation such as kidney transplantation, liver transplantation, heart transplantation, lung transplantation, pancreas transplantation, small intestine transplantation or the like, and diseases such as rejection in bone marrow transplantation, graft-versus-host disease, Behcet's disease, plaque psoriasis, pustular psoriasis, psoriatic erythroderma, psoriasis arthropica, aplastic anemia, pure red cell aplasia, nephrotic syndrome, systemic myasthenia gravis, atopic dermatitis, ulcerative colitis or the like in Japan (non-patent documents 2 and 3).

It is known that PKC family consists of at least 12 kinds of subtypes and is expressed in various tissues. Since the PKC family is expressed in immunocompetent cells such as T cell, B cell, macrophage or the like, and plays an important role in various immune reactions, its involvement in immune diseases and inflammatory diseases is assumed (non-patent document 4).

In the family, PKC-θ is a phosphorylating enzyme selectively expressed in T cell and plays an important role in the activation of T cell, since it transmits stimulation on T cell receptors into the cell.

Inhibition of PKC-θ is known to suppress activation of T cell (non-patent document 5), and PKC-θ-deficient mouse has been reported to be resistant to multiple sclerosis model, inflammatory bowel disease model, graft versus host disease model, rheumatoid arthritis model and asthma model (non-patent documents 6 - 8).

From the foregoing, a PKC (e.g., PKC-θ) inhibitor becomes a prophylactic or therapeutic drug for rejection in organ transplantation such as kidney transplantation, liver transplantation, heart transplantation, lung transplantation, pancreas transplantation, small intestine transplantation or the like, rejection in bone marrow transplantation, and various immune diseases and inflammatory diseases such as graft-versus-host disease, Behcet's disease, plaque psoriasis, pustular psoriasis, psoriatic erythroderma, psoriasis arthropica, aplastic anemia, pure red cell aplasia, nephrotic syndrome, systemic myasthenia gravis, atopic dermatitis, ulcerative colitis, asthma or the like.

Examples of the compound having a structure similar to the compound described in the present specification include the following compounds.

### (1) A compound represented by the following formula:

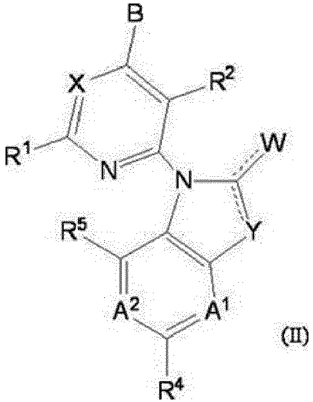

wherein
R¹ is -NR⁹R¹⁰-, -NR⁹COR⁹-, -S(O)ₙR¹²- etc. (R⁹, R¹⁰ and R¹² are each independently C₃₋₆ cycloalkyl, C₄₋₇ cycloalkylalkyl, n is 0-2), cyano, C₁₋₄ haloalkyl or the like, or C₁₋₆ alkyl, C₃₋₆ cycloalkyl etc. (each is optionally substituted by -NR⁹R¹⁰-,-NR⁹COR⁹-, -S(O)ₙR¹²- etc.);
A¹ and A² are each independently N or CR⁵;
W is =O, =S, -H or (-H, -H);
X is N or CR¹;
Y is -C(R^{Y})₂-, -N-R^{Y}- etc. (R^{Y} is H or the like);
R² is H, halogen, cyano, C₁₋₄ alkyl, C₃₋₆ cycloalkyl or the like; B is R³, NHR³ or the like;
R³ is C₁₋₁₀ alkyl or the like;
R⁴ is H, -OR¹⁰, -COR⁹, halogen, C₁₋₆ alkyl, C₃₋₆ cycloalkyl or the like; and
R⁵ is H, halogen, C₁₋₁₀ alkyl, C₂₋₁₀ alkenyl, C₃₋₆ cycloalkyl or the like (patent document 1).

### (2) A compound represented by the following formula:

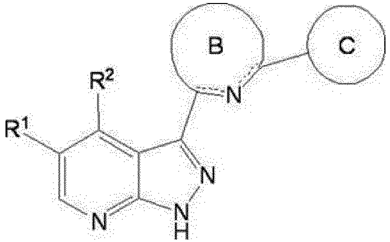

wherein
B is optionally substituted 6-membered aromatic heterocycle or the like;
C is optionally substituted 3-8-membered monocycle (having 0-3 hetero atoms), optionally substituted C₈₋₁₂ bicyclic ring (having 0-5 hetero atoms); and
R¹ and R² are each independently H, halogen, -OR' (R' is H, C₁₋₆ alkyl (optionally substituted by -OR, -N(R)₂, cyano, oxo etc.)), cyano, C₁₋₁₀ aliphatic compound (optionally substituted by halogen, -OR, -N(R)₂, cyano etc.) or the like (patent document 2).

### (3) A compound represented by the following formula:

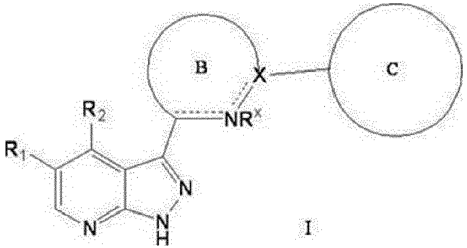

wherein
B is optionally substituted 5-membered aromatic heterocycle or the like;
R¹ and R² are each independently H, halogen, -OR', cyano, C₁₋₁₀ aliphatic compound (optionally substituted by halogen, -OR, - N(R)₂, -C(O)R, cyano, oxo etc.), C₃₋₈ alicyclic compound (optionally substituted by halogen, -OR, -N(R)₂, cyano, oxo, C₁₋₆ alkyl (optionally substituted by halogen, -OR, -N(R)₂, -C(O)R, cyano, oxo etc.) etc.) or the like;
X is C or N;
R^{X} is absent or H;
C is optionally substituted 3-8-membered monocycle (having 0-3 hetero atoms), optionally substituted C₈₋₁₂ bicyclic ring (having 0-5 hetero atoms);
R is H or C₁₋₆ alkyl; and
R' is H or C₁₋₆ alkyl (optionally substituted by halogen, -OR,-N(R)₂, -C(O)R, cyano, oxo etc.) (patent document 3).

### (4) A compound represented by the following formula:

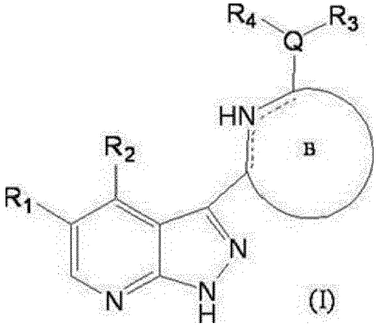

wherein
B is optionally substituted 6-membered aromatic heterocycle or the like;
R¹ and R² are each independently H, halogen, -OR', cyano, C₁₋₁₀ aliphatic compound (optionally substituted by halogen, -OR, - N(R)₂, -C(O)R, cyano, oxo etc.), C₃₋₈ alicyclic compound (optionally substituted by halogen, -OR, -N(R)₂, cyano, oxo, C₁₋₆ alkyl etc.) or the like;
R³ is absent, or H, C₁₋₆ alkyl (optionally substituted by halogen, -OR, -N(R)₂, -C(O)R, cyano, oxo etc.);
R⁴ is optionally substituted C₁₋₁₀ aliphatic compound, optionally substituted 3-8-membered monocycle (having 0-3 hetero atoms), optionally substituted C₈₋₁₂ bicyclic ring (having 0-5 hetero atoms);
R is H or C₁₋₆ alkyl;
R' is H or C₁₋₆ alkyl (optionally substituted by halogen, -OR,-N(R)₂, -C(O)R, cyano, oxo etc.); and
Q is N, O or S (patent document 4).

### (5) A compound represented by the following formula:

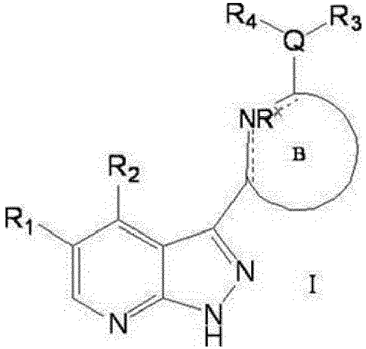

wherein
B is optionally substituted 5-membered aromatic heterocycle or the like;
R₁ and R₂ are each independently H, halogen, cyano, -OR', C₁₋₁₀ aliphatic compound (optionally substituted by halogen, -OR, - N(R)₂, -C(O)R, cyano, oxo etc.), C₃₋₈ alicyclic compound (optionally substituted by halogen, -OR, -N(R)₂, cyano, oxo, C₁₋₆ alkyl (optionally substituted by halogen, -OR, -N(R)₂, -C(O)R, cyano, oxo etc.));
R³ is absent, or H, C₁₋₆ alkyl (optionally substituted by halogen, -OR, -N(R)₂, -C(O)R, cyano, oxo etc.);
R₄ is optionally substituted C₁₋₁₀ aliphatic compound, optionally substituted 3-8-membered monocycle (having 0-3 hetero atoms), optionally substituted C₈₋₁₂ bicyclic ring (having 0-5 hetero atoms);
Q is N, O or S; and
R^{X} is absent or H (patent document 5).

### (6) A compound represented by the following formula:

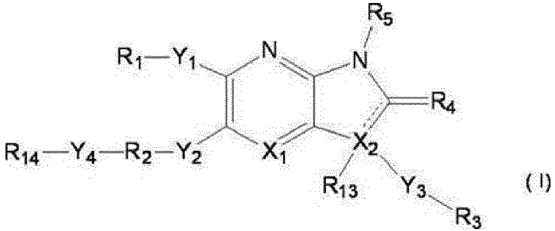

wherein
X₁ is CH or N;
X₂ is C or N;
Y₁ is -(CR₆ₐR_{6b})ₘ-Z₁-(CR₇ₐR_{7b})ₙ-;
Y₂ is -(CR₈ₐR_{8b})ₚ-Z₂-(CR₉ₐR_{9b})_{q}-;
Y₃ is -(CR₁₀ₐR_{10b})ᵣ-Z₃-(CR₁₁ₐR_{11b})ₛ-;
Y₄ is -(CH₂)ₜ-Z₄-:
   Z₁-Z₃ are each independently bond, O, S, C(O) or the like;
   Z₄ is bond, O or N;
   m, n, p, q, r and s are each independently 0-6;
   t is 0-2;
   R₆ₐ-R_{11b} are each independently bond, H, -OH, C₁₋₃ alkoxy or C₁₋₃ alkyl;
   R₁, R₂ and R₃ are each independently H, halogen, alkoxy, cyano, optionally substituted cycloalkyl, optionally substituted aryl, optionally substituted heterocycloalkyl, optionally substituted heteroaryl;
   R₄ is H, O or the like;
   R₅ and R₁₃ are each independently H, halogen, OH, lower alkyl or the like; and
   R₁₄ is absent, or optionally substituted lower cycloalkyl, optionally substituted phenyl or the like (patent document 6).

### (7) A compound represented by the following formula:

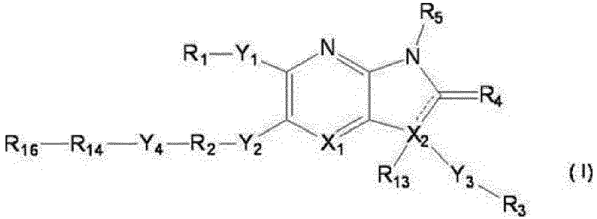

wherein
X₁ is CH or N;
X₂ is CR₁₃ or N ;
Y₁ is -(CR₆ₐR_{6b})ₘ-Z₁-(CR₇ₐR_{7b})ₙ-;
Y₂ is -(CR₈ₐR_{8b})ₚ-Z₂-(CR₉ₐR_{9b})_{q}-;
Y₃ is -(CR₁₀ₐR_{10b})ᵣ-Z₃-(CR₁₁ₐR_{11b})ₛ-;
Y₄ is -(CH₂)ₜ-Z₄-;
Z₁-Z₃ are each independently bond, O, S, C(O) or the like;
Z₄ is bond, O or N;
m, n, p, q, r and s are each independently 0-6;
t is 0-2;
R₆ₐ-R_{11b} are each independently bond, H, -OH, C₁₋₃ alkoxy or C₁₋₃ alkyl or the like;
R₁, R₂ and R₃ are each independently H, halogen, optionally substituted alkoxy, cyano, optionally substituted lower cycloalkyl, optionally substituted aryl, optionally substituted heterocycloalkyl, optionally substituted heteroaryl or the like;
R₄ is H, O, S, halogen, cyano, lower alkyl, lower alkenyl, aryl, lower heteroaryl or the like;
R₅ and R₁₃ are each independently H, halogen, lower alkyl, lower alkene or the like;
when X₂ is N, R₁₃ is absent;
R₁₄ is absent, or lower cycloalkyl, phenyl (each optionally substituted by C₁₋₃ alkyl etc.) or the like; and R₁₆ is lower alkyl, carbonyl, heteroaryl or the like (patent document 7).

### (8) A compound represented by the following formula:

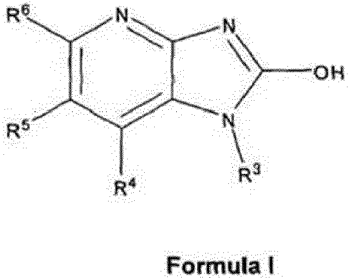

wherein
R³ is optionally substituted alkyl, optionally substituted cycloalkyl, optionally substituted aryl, optionally substituted heteroaryl or optionally substituted heterocycloalkyl;
R⁴ is optionally substituted aryl or optionally substituted heteroaryl;
R⁵ is H, halo, OH, optionally substituted alkyl, optionally substituted amino, optionally substituted alkenyl, optionally substituted alkynyl, optionally substituted cycloalkyl, optionally substituted aryl, optionally substituted heteroaryl, optionally substituted alkoxy, sulfanyl or sulfinyl; and
R⁶ is H, halo, OH, lower alkyl or lower haloalkyl (patent document 8).

### [Document List]

### Patent Documents

patent document 1: WO 02/092090
patent document 2: WO 2010/011772
patent document 3: WO 2010/011768
patent document 4: WO 2010/011756
patent document 5: WO 2010/011762
patent document 6: WO 2010/068483
patent document 7: WO 2011/149950
patent document 8: WO 2009/099594

### [non-patent document]

non-patent document 1: Immunotherapy. 2011 Jul; 3(7):853-70
non-patent document 2: Prograf Capsules package insert, revised in June 2012 (30th edition)
non-patent document 3: Neoral package insert, revised in August 2012 (18th edition)
non-patent document 4: Biochem J. 2003 Dec 15; 376(Pt 3):545-52
non-patent document 5: Front Immunol. 2012; 3:273
non-patent document 6: Autoimmunity. 2006 Sep; 39(6):469-78
non-patent document 7: J Clin Invest. 2009 Dec; 119(12):3774-86
non-patent document 8: J Immunol. 2006 Aug 1; 177(3):1886-93

### [SUMMARY OF THE INVENTION]

### [Problems to be Solved by the Invention]

An object of the present invention is to provide a compound having a superior PKC inhibitory activity, and useful as a prophylactic or therapeutic agent for immune disease, inflammatory diseases and the like.

### [Means of Solving the Problems]

The present inventors have conducted intensive studies in an attempt to solve the above-mentioned problem and found that compound represented by the following formula or a salt thereof has a superior PKC inhibitory activity, which resulted in the completion of the present invention.

Accordingly, the present invention relates to
[1] a compound represented by the formula (I): 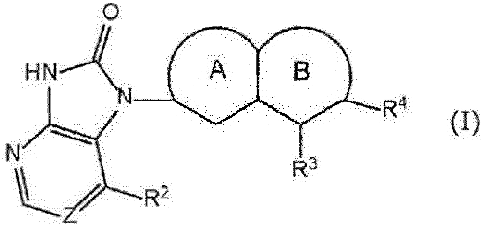 wherein
   Z is N or CR¹;
   R¹ is a hydrogen atom or a substituent;
   R² is a substituent;
   R³ is a hydrogen atom or a substituent;
   R⁴ is a hydrogen atom or a substituent, or optionally forms an optionally substituted ring together with R³;
   ring A is an optionally further substituted benzene ring, or an optionally further substituted 5-membered or 6-membered aromatic heterocycle; and
   ring B is an optionally further substituted 5-membered or 6-membered aromatic heterocycle, or a salt thereof (hereinafter sometimes to be referred to as compound (I));
[2] the compound of [1], wherein Z is N or CR¹;
   R¹ is a hydrogen atom or a halogen atom;
   R² is an optionally substituted C₁₋₆ alkyl group, an optionally substituted C₁₋₆ alkoxy group, an optionally substituted C₃₋₁₀ cycloalkyl group, an optionally substituted C₃₋₁₀ cycloalkyloxy group, an optionally substituted C₆₋₁₄ aryl group, an optionally substituted C₆₋₁₄ aryloxy group, an optionally substituted nonaromatic heterocyclic group, an optionally substituted non-aromatic heterocyclyl-oxy group or an optionally substituted mono- or di-C₁₋₆ alkylamino group;
   R³ is a hydrogen atom, a halogen atom, a cyano group, a hydroxy group, an optionally substituted C₁₋₆ alkyl group, an optionally substituted C₂₋₆ alkenyl group, an optionally substituted C₃₋₁₀ cycloalkyl group, an optionally substituted C₁₋₆ alkoxy group, an optionally substituted C₃₋₁₀ cycloalkyloxy group, an optionally substituted amino group, an optionally substituted carbamoyl group, an optionally substituted nonaromatic heterocyclic group or an optionally substituted 7-to 10-membered crosslinked heterocyclic group;
   R⁴ is a hydrogen atom, a halogen atom, an optionally substituted C₁₋₆ alkyl group, an optionally substituted C₃₋₁₀ cycloalkyl group, an optionally substituted C₆₋₁₄ aryl group, an optionally substituted C₁₋₆ alkoxy group, an optionally substituted C₁₋₆ alkyl-carbonylamino group, an optionally substituted carbamoyl group, an optionally substituted nonaromatic heterocyclic group or an optionally substituted aromatic heterocyclic group; or
   R⁴ and R³ are joined to form a non-aromatic heterocycle or an aromatic heterocycle, each of which is optionally substituted by 1 to 3 substituents selected from an optionally substituted C₁₋₆ alkyl group, an oxo group, an optionally substituted C₁₋₆ alkyl-carbonyl group and an optionally substituted C₁₋₆ alkylsulfonyl group;
   ring A is an optionally further substituted benzene ring, or a pyridine ring, a pyrazine ring or a pyridazine ring, each of which is optionally further substituted; and
   ring B is a furan ring, an imidazole ring, a pyrazole ring, a pyridine ring, a pyrimidine ring, a pyridazine ring or a pyrazine ring, each of which is optionally further substituted;
   or a salt thereof;
[3] the compound of [2], wherein Z is N or CR¹;
   R¹ is a hydrogen atom, a fluorine atom or a bromine atom;
   R² is
   (1) a C₁₋₆ alkyl group,
   (2) a C₁₋₆ alkoxy group optionally substituted by 1 - 5 substituents selected from a halogen atom, a hydroxy group, a C₁₋₆ alkoxy group, a C₃₋₁₀ cycloalkyl group, a C₁₋₆ alkyl-carbonyloxy group and a tri-C₁₋₆ alkylsilyl-oxy group,
   (3) a C₃₋₁₀ cycloalkyl group,
   (4) a C₃₋₁₀ cycloalkyloxy group optionally substituted by 1 - 5 substituents selected from a di-C₁₋₆ alkylamino group and a nonaromatic heterocyclic group,
   (5) a C₆₋₁₄ aryl group,
   (6) a C₆₋₁₄ aryloxy group,
   (7) a nonaromatic heterocyclic group,
   (8) a non-aromatic heterocyclyl-oxy group optionally substituted by a C₁₋₆ alkyl group, or
   (9) a mono- or di-C₁₋₆ alkylamino group optionally substituted by a C₆₋₁₄ aryl group;
   R³ is
   (1) a hydrogen atom,
   (2) a halogen atom,
   (3) a cyano group,
   (4) a hydroxy group,
   (5) a C₁₋₆ alkyl group optionally substituted by 1 to 3 substituents selected from a halogen atom, a C₃₋₁₀ cycloalkyl group and an amino group,
   (6) a C₂₋₆ alkenyl group,
   (7) a C₃₋₁₀ cycloalkyl group,
   (8) a C₁₋₆ alkoxy group optionally substituted by 1 to 3 substituents selected from a halogen atom, a C₁₋₆ alkoxy group and an amino group,
   (9) a C₃₋₁₀ cycloalkyloxy group,
   (10) an amino group,
   (11) a mono- or di-C₁₋₆ alkylamino group optionally substituted by a mono- or di-C₁₋₆ alkylamino group,
   (12) a (C₁₋₆ alkyl)(3- to 14-membered non-aromatic heterocyclyl)amino group,
   (13) a mono- or di-C₇₋₁₆ aralkylamino group,
   (14) a carbamoyl group,
   (15) a nonaromatic heterocyclic group optionally substituted by 1 to 3 substituents selected from a hydroxy group, an oxo group, a C₁₋₆ alkyl group optionally substituted by a hydroxy group, a C₆₋₁₄ aryl group, a mono- or di-C₁₋₆ alkylamino group and a C₁₋₆ alkoxy-carbonyl group, or
   (16) a 7- to 10-membered crosslinked heterocyclic group;
   R⁴ is
   (1) a hydrogen atom,
   (2) a halogen atom,
   (3) a C₁₋₆ alkyl group,
   (4) a C₃₋₁₀ cycloalkyl group,
   (5) a C₆₋₁₄ aryl group optionally substituted by 1 to 3 halogen atoms,
   (6) a C₁₋₆ alkoxy group optionally substituted by 1 to 3 substituents selected from a halogen atom and a C₁₋₆ alkoxy group,
   (7) a C₁₋₆ alkyl-carbonylamino group,
   (8) a carbamoyl group,
   (9) a nonaromatic heterocyclic group optionally substituted by an oxo group, or
   (10) an aromatic heterocyclic group optionally substituted by 1 to 3 substituents selected from (i) a C₁₋₆ alkyl group optionally substituted by 1 to 3 substituents selected from a C₃₋₁₀ cycloalkyl group and an aromatic heterocyclic group, and (ii) a nonaromatic heterocyclic group; or
   R⁴ and R³ are joined to form
   (1) a non-aromatic heterocycle optionally substituted by 1 to 3 substituents selected from a C₁₋₆ alkyl group optionally substituted by a C₆₋₁₄ aryl group, an oxo group, a C₁₋₆ alkyl-carbonyl group and a C₁₋₆ alkylsulfonyl group, or
   (2) an aromatic heterocycle optionally substituted by 1 to 3 substituents selected from a C₁₋₆ alkyl group optionally substituted by a hydroxy group;
   ring A is a benzene ring optionally further substituted by 1 to 3 substituents selected from a halogen atom, a C₁₋₆ alkyl group and a C₃₋₁₀ cycloalkyl group, or a pyridine ring, a pyrazine ring or a pyridazine ring; and
   ring B is a pyridine ring optionally further substituted by 1 or 2 substituents selected from a halogen atom, a hydroxy group, a C₁₋₆ alkyl group, a C₁₋₆ alkoxy group and an amino group, or a furan ring, an imidazole ring, a pyrazole ring, a pyrimidine ring, a pyridazine ring or a pyrazine ring;
   or a salt thereof;
[4] 7-ethoxy-1-(4-methoxyquinolin-6-yl)-1,3-dihydro-2H-imidazo[4,5-b]pyridin-2-one or a salt thereof;
[5] 7-ethoxy-1-(8-fluoro-2,3-dihydro[1,4]dioxino[2,3-c]quinolin-9-yl)-1,3-dihydro-2H-imidazo[4,5-b]pyridin-2-one or a salt thereof;
[6] 7-ethoxy-1-(4-methoxy-3-(1-methyl-1H-pyrazol-4-yl)quinolin-6-yl)-1,3-dihydro-2H-imidazo[4,5-b]pyridin-2-one or a salt thereof;
[7] a medicament comprising the compound of [1] or a salt thereof;
[8] the medicament of [7], which is a protein kinase C inhibitor;
[9] the medicament of [7], which is a prophylactic or therapeutic agent for immune diseases and/or inflammatory diseases;
[10] the medicament of [7], which is a prophylactic or therapeutic agent for an inflammatory bowel disease, psoriasis or atopic dermatitis;
[11] the compound of [1] or a salt thereof for use in the prophylaxis or treatment of immune diseases and/or inflammatory diseases;
[12] the compound of [1] or a salt thereof for use in the prophylaxis or treatment of an inflammatory bowel disease, psoriasis or atopic dermatitis;
[13] a method of inhibiting protein kinase C in a mammal, comprising administering an effective amount of the compound of [1] or a salt thereof to the mammal;
[14] a method for the prophylaxis or treatment of an immune disease and/or an inflammatory disease in a mammal, comprising administering an effective amount of the compound of [1] or a salt thereof to the mammal;
[15] a method for the prophylaxis or treatment of an inflammatory bowel disease, psoriasis or atopic dermatitis in a mammal, comprising administering an effective amount of the compound of [1] or a salt thereof to the mammal;
[16] use of the compound of [1] or a salt thereof in the production of a prophylactic or therapeutic agent for an immune disease and/or an inflammatory disease;
[17] use of the compound of [1] or a salt thereof in the production of a prophylactic or therapeutic agent for an inflammatory bowel disease, psoriasis or atopic dermatitis; and the like.

### [Effect of the Invention]

Compound (I) has a superior PKC (e.g., PKC-θ) inhibitory activity, and is useful as a prophylactic or therapeutic agent for immune diseases, inflammatory diseases and the like.

### (Detailed Description of the Invention)

The definition of each substituent used in the present specification is described in detail in the following. Unless otherwise specified, each substituent has the following definition.

In the present specification, examples of the "halogen atom" include fluorine, chlorine, bromine and iodine.

In the present specification, examples of the "C₁₋₆ alkyl group" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl and 2-ethylbutyl.

In the present specification, examples of the "optionally halogenated C₁₋₆ alkyl group" include a C₁₋₆ alkyl group optionally having 1 to 7, preferably 1 to 5, halogen atoms. Specific examples thereof include methyl, chloromethyl, difluoromethyl, trichloromethyl, trifluoromethyl, ethyl, 2-bromoethyl, 2,2,2-trifluoroethyl, tetrafluoroethyl, pentafluoroethyl, propyl, 2,2-difluoropropyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl and 6,6,6-trifluorohexyl.

In the present specification, examples of the "C₂₋₆ alkenyl group" include ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl and 5-hexenyl.

In the present specification, examples of the "C₂₋₆ alkynyl group" include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and 4-methyl-2-pentynyl.

In the present specification, examples of the "C₃₋₁₀ cycloalkyl group" include cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl and adamantyl.

In the present specification, examples of the "optionally halogenated C₃₋₁₀ cycloalkyl group" include a C₃₋₁₀ cycloalkyl group optionally having 1 to 7, preferably 1 to 5, halogen atoms. Specific examples thereof include cyclopropyl, 2,2-difluorocyclopropyl, 2,3-difluorocyclopropyl, cyclobutyl, difluorocyclobutyl, cyclopentyl, cyclohexyl, cycloheptyl and cyclooctyl.

In the present specification, examples of the "C₃₋₁₀ cycloalkenyl group" include cyclopropenyl, cyclobutenyl, cyclopentenyl, cyclohexenyl, cycloheptenyl and cyclooctenyl.

In the present specification, examples of the "C₆₋₁₄ aryl group" include phenyl, 1-naphthyl, 2-naphthyl, 1-anthryl, 2-anthryl and 9-anthryl.

In the present specification, examples of the "C₇₋₁₆ aralkyl group" include benzyl, phenethyl, naphthylmethyl and phenylpropyl.

In the present specification, examples of the "C₁₋₆ alkoxy group" include methoxy, ethoxy, propoxy, isopropoxy, butoxy, isobutoxy, sec-butoxy, tert-butoxy, pentyloxy and hexyloxy.

In the present specification, examples of the "optionally halogenated C₁₋₆ alkoxy group" include a C₁₋₆ alkoxy group optionally having 1 to 7, preferably 1 to 5, halogen atoms. Specific examples thereof include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy and hexyloxy.

In the present specification, examples of the "C₃₋₁₀ cycloalkyloxy group" include cyclopropyloxy, cyclobutyloxy, cyclopentyloxy, cyclohexyloxy, cycloheptyloxy and cyclooctyloxy.

In the present specification, examples of the "C₁₋₆ alkylthio group" include methylthio, ethylthio, propylthio, isopropylthio, butylthio, sec-butylthio, tert-butylthio, pentylthio and hexylthio.

In the present specification, examples of the "optionally halogenated C₁₋₆ alkylthio group" include a C₁₋₆ alkylthio group optionally having 1 to 7, preferably 1 to 5, halogen atoms. Specific examples thereof include methylthio, difluoromethylthio, trifluoromethylthio, ethylthio, propylthio, isopropylthio, butylthio, 4,4,4-trifluorobutylthio, pentylthio and hexylthio.

In the present specification, examples of the "C₁₋₆ alkyl-carbonyl group" include acetyl, propanoyl, butanoyl, 2-methylpropanoyl, pentanoyl, 3-methylbutanoyl, 2-methylbutanoyl, 2,2-dimethylpropanoyl, hexanoyl and heptanoyl.

In the present specification, examples of the "optionally halogenated C₁₋₆ alkyl-carbonyl group" include a C₁₋₆ alkyl-carbonyl group optionally having 1 to 7, preferably 1 to 5, halogen atoms. Specific examples thereof include acetyl, chloroacetyl, trifluoroacetyl, trichloroacetyl, propanoyl, butanoyl, pentanoyl and hexanoyl.

In the present specification, examples of the "C₁₋₆ alkoxy-carbonyl group" include methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl and hexyloxycarbonyl.

In the present specification, examples of the "C₆₋₁₄ aryl-carbonyl group" include benzoyl, 1-naphthoyl and 2-naphthoyl. In the present specification, examples of the "C₇₋₁₆ aralkyl-carbonyl group" include phenylacetyl and phenylpropionyl.

In the present specification, examples of the "5- to 14-membered aromatic heterocyclylcarbonyl group" include nicotinoyl, isonicotinoyl, thenoyl and furoyl.

In the present specification, examples of the "3- to 14-membered non-aromatic heterocyclylcarbonyl group" include morpholinylcarbonyl, piperidinylcarbonyl and
pyrrolidinylcarbonyl.

In the present specification, examples of the "mono- or di-C₁₋₆ alkyl-carbamoyl group" include methylcarbamoyl, ethylcarbamoyl, dimethylcarbamoyl, diethylcarbamoyl and N-ethyl-N-methylcarbamoyl.

In the present specification, examples of the "mono- or di-C₇₋₁₆ aralkyl-carbamoyl group" include benzylcarbamoyl and phenethylcarbamoyl.

In the present specification, examples of the "C₁₋₆ alkylsulfonyl group" include methylsulfonyl, ethylsulfonyl, propylsulfonyl, isopropylsulfonyl, butylsulfonyl, sec-butylsulfonyl and tert-butylsulfonyl.

In the present specification, examples of the "optionally halogenated C₁₋₆ alkylsulfonyl group" include a C₁₋₆ alkylsulfonyl group optionally having 1 to 7, preferably 1 to 5, halogen atoms. Specific examples thereof include
methylsulfonyl, difluoromethylsulfonyl, trifluoromethylsulfonyl, ethylsulfonyl, propylsulfonyl, isopropylsulfonyl, butylsulfonyl, 4,4,4-trifluorobutylsulfonyl, pentylsulfonyl and hexylsulfonyl.

In the present specification, examples of the "C₆₋₁₄ arylsulfonyl group" include phenylsulfonyl, 1-naphthylsulfonyl and 2-naphthylsulfonyl.

In the present specification, examples of the "substituent" include a halogen atom, a cyano group, a nitro group, an optionally substituted hydrocarbon group, an optionally substituted heterocyclic group, an acyl group, an optionally substituted amino group, an optionally substituted carbamoyl group, an optionally substituted thiocarbamoyl group, an optionally substituted sulfamoyl group, an optionally substituted hydroxy group, an optionally substituted sulfanyl (SH) group and an optionally substituted silyl group.

In the present specification, examples of the "hydrocarbon group" (including "hydrocarbon group" of "optionally substituted hydrocarbon group") include a C₁₋₆ alkyl group, a C₂₋₆ alkenyl group, a C₂₋₆ alkynyl group, a C₃₋₁₀ cycloalkyl group, a C₃₋₁₀ cycloalkenyl group, a C₆₋₁₄ aryl group and a C₇₋₁₆ aralkyl group.

In the present specification, examples of the "optionally substituted hydrocarbon group" include a hydrocarbon group optionally having substituent(s) selected from the following substituent group A.

### [substituent group A]

(1) a halogen atom,
(2) a nitro group,
(3) a cyano group,
(4) an oxo group,
(5) a hydroxy group,
(6) an optionally halogenated C₁₋₆ alkoxy group,
(7) a C₆₋₁₄ aryloxy group (e.g., phenoxy, naphthoxy),
(8) a C₇₋₁₆ aralkyloxy group (e.g., benzyloxy),
(9) a 5- to 14-membered aromatic heterocyclyloxy group (e.g., pyridyloxy),
(10) a 3- to 14-membered non-aromatic heterocyclyloxy group (e.g., morpholinyloxy, piperidinyloxy),
(11) a C₁₋₆ alkyl-carbonyloxy group (e.g., acetoxy, propanoyloxy),
(12) a C₆₋₁₄ aryl-carbonyloxy group (e.g., benzoyloxy, 1-naphthoyloxy, 2-naphthoyloxy),
(13) a C₁₋₆ alkoxy-carbonyloxy group (e.g., methoxycarbonyloxy, ethoxycarbonyloxy, propoxycarbonyloxy, butoxycarbonyloxy),
(14) a mono- or di-C₁₋₆ alkyl-carbamoyloxy group (e.g., methylcarbamoyloxy, ethylcarbamoyloxy, dimethylcarbamoyloxy, diethylcarbamoyloxy),
(15) a C₆₋₁₄ aryl-carbamoyloxy group (e.g., phenylcarbamoyloxy, naphthylcarbamoyloxy),
(16) a 5- to 14-membered aromatic heterocyclylcarbonyloxy group (e.g., nicotinoyloxy),
(17) a 3- to 14-membered non-aromatic heterocyclylcarbonyloxy group (e.g., morpholinylcarbonyloxy, piperidinylcarbonyloxy),
(18) an optionally halogenated C₁₋₆ alkylsulfonyloxy group (e.g., methylsulfonyloxy, trifluoromethylsulfonyloxy),
(19) a C₆₋₁₄ arylsulfonyloxy group optionally substituted by a C₁₋₆ alkyl group (e.g., phenylsulfonyloxy, toluenesulfonyloxy),
(20) an optionally halogenated C₁₋₆ alkylthio group,
(21) a 5- to 14-membered aromatic heterocyclic group,
(22) a 3- to 14-membered non-aromatic heterocyclic group,
(23) a formyl group,
(24) a carboxy group,
(25) an optionally halogenated C₁₋₆ alkyl-carbonyl group,
(26) a C₆₋₁₄ aryl-carbonyl group,
(27) a 5- to 14-membered aromatic heterocyclylcarbonyl group,
(28) a 3- to 14-membered non-aromatic heterocyclylcarbonyl group,
(29) a C₁₋₆ alkoxy-carbonyl group,
(30) a C₆₋₁₄ aryloxy-carbonyl group (e.g., phenyloxycarbonyl, 1-naphthyloxycarbonyl, 2-naphthyloxycarbonyl),
(31) a C₇₋₁₆ aralkyloxy-carbonyl group (e.g., benzyloxycarbonyl, phenethyloxycarbonyl),
(32) a carbamoyl group,
(33) a thiocarbamoyl group,
(34) a mono- or di-C₁₋₆ alkyl-carbamoyl group,
(35) a C₆₋₁₄ aryl-carbamoyl group (e.g., phenylcarbamoyl),
(36) a 5- to 14-membered aromatic heterocyclylcarbamoyl group (e.g., pyridylcarbamoyl, thienylcarbamoyl),
(37) a 3- to 14-membered non-aromatic heterocyclylcarbamoyl group (e.g., morpholinylcarbamoyl, piperidinylcarbamoyl),
(38) an optionally halogenated C₁₋₆ alkylsulfonyl group,
(39) a C₆₋₁₄ arylsulfonyl group,
(40) a 5- to 14-membered aromatic heterocyclylsulfonyl group (e.g., pyridylsulfonyl, thienylsulfonyl),
(41) an optionally halogenated C₁₋₆ alkylsulfinyl group,
(42) a C₆₋₁₄ arylsulfinyl group (e.g., phenylsulfinyl, 1-naphthylsulfinyl, 2-naphthylsulfinyl),
(43) a 5- to 14-membered aromatic heterocyclylsulfinyl group (e.g., pyridylsulfinyl, thienylsulfinyl),
(44) an amino group,
(45) a mono- or di-C₁₋₆ alkylamino group (e.g., methylamino, ethylamino, propylamino, isopropylamino, butylamino, dimethylamino, diethylamino, dipropylamino, dibutylamino, N-ethyl-N-methylamino),
(46) a mono- or di-C₆₋₁₄ arylamino group (e.g., phenylamino),
(47) a 5- to 14-membered aromatic heterocyclylamino group (e.g., pyridylamino),
(48) a C₇₋₁₆ aralkylamino group (e.g., benzylamino),
(49) a formylamino group,
(50) a C₁₋₆ alkyl-carbonylamino group (e.g., acetylamino, propanoylamino, butanoylamino),
(51) a (C₁₋₆ alkyl) (C₁₋₆ alkyl-carbonyl) amino group (e.g., N-acetyl-N-methylamino),
(52) a C₆₋₁₄ aryl-carbonylamino group (e.g., phenylcarbonylamino, naphthylcarbonylamino),
(53) a C₁₋₆ alkoxy-carbonylamino group (e.g.,
   methoxycarbonylamino, ethoxycarbonylamino, propoxycarbonylamino, butoxycarbonylamino, tert-butoxycarbonylamino),
(54) a C₇₋₁₆ aralkyloxy-carbonylamino group (e.g.,
   benzyloxycarbonylamino),
(55) a C₁₋₆ alkylsulfonylamino group (e.g., methylsulfonylamino, ethylsulfonylamino),
(56) a C₆₋₁₄ arylsulfonylamino group optionally substituted by a C₁₋₆ alkyl group (e.g., phenylsulfonylamino,
   toluenesulfonylamino),
(57) an optionally halogenated C₁₋₆ alkyl group,
(58) a C₂₋₆ alkenyl group,
(59) a C₂₋₆ alkynyl group,
(60) a C₃₋₁₀ cycloalkyl group,
(61) a C₃₋₁₀ cycloalkenyl group and
(62) a C₆₋₁₄ aryl group.

The number of the above-mentioned substituents in the "optionally substituted hydrocarbon group" is, for example, 1 to 5, preferably 1 to 3. When the number of the substituents is two or more, the respective substituents may be the same or different.

In the present specification, examples of the "heterocyclic group" (including "heterocyclic group" of "optionally substituted heterocyclic group") include (i) an aromatic heterocyclic group, (ii) a non-aromatic heterocyclic group and (iii) a 7- to 10-membered bridged heterocyclic group, each containing, as a ring-constituting atom besides carbon atom, 1 to 4 hetero atoms selected from a nitrogen atom, a sulfur atom and an oxygen atom.

In the present specification, examples of the "aromatic heterocyclic group" (including "5- to 14-membered aromatic heterocyclic group") include a 5- to 14-membered (preferably 5-to 10-membered) aromatic heterocyclic group containing, as a ring-constituting atom besides carbon atom, 1 to 4 hetero atoms selected from a nitrogen atom, a sulfur atom and an oxygen atom.

Preferable examples of the "aromatic heterocyclic group" include 5- or 6-membered monocyclic aromatic heterocyclic groups such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, triazinyl and the like; and
8- to 14-membered fused polycyclic (preferably bi or tricyclic) aromatic heterocyclic groups such as benzothiophenyl, benzofuranyl, benzimidazolyl, benzoxazolyl, benzisoxazolyl, benzothiazolyl, benzisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acridinyl, phenazinyl, phenothiazinyl, phenoxazinyl and the like.

In the present specification, examples of the "non-aromatic heterocyclic group" (including "3- to 14-membered non-aromatic heterocyclic group") include a 3- to 14-membered (preferably 4- to 10-membered) non-aromatic heterocyclic group containing, as a ring-constituting atom besides carbon atom, 1 to 4 hetero atoms selected from a nitrogen atom, a sulfur atom and an oxygen atom.

Preferable examples of the "non-aromatic heterocyclic group" include 3- to 8-membered monocyclic non-aromatic heterocyclic groups such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisooxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, diazocanyl and the like; and
9- to 14-membered fused polycyclic (preferably bi or tricyclic) non-aromatic heterocyclic groups such as dihydrobenzofuranyl, dihydrobenzimidazolyl, dihydrobenzoxazolyl, dihydrobenzothiazolyl, dihydrobenzisothiazolyl, dihydronaphtho[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzazepinyl, tetrahydroquinoxalinyl, tetrahydrophenanthridinyl, hexahydrophenothiazinyl, hexahydrophenoxazinyl, tetrahydrophthalazinyl, tetrahydronaphthyridinyl, tetrahydroquinazolinyl, tetrahydrocinnolinyl, tetrahydrocarbazolyl, tetrahydro-β-carbolinyl, tetrahydroacrydinyl, tetrahydrophenazinyl, tetrahydrothioxanthenyl, octahydroisoquinolyl and the like.

In the present specification, preferable examples of the "7- to 10-membered bridged heterocyclic group" include quinuclidinyl and 7-azabicyclo[2.2.1]heptanyl.

In the present specification, examples of the "nitrogen-containing heterocyclic group" include the "heterocyclic group" containing at least one nitrogen atom as a ring-constituting atom.

In the present specification, examples of the "optionally substituted heterocyclic group" include a heterocyclic group optionally having substituent(s) selected from the aforementioned substituent group A.

The number of the substituents in the "optionally substituted heterocyclic group" is, for example, 1 to 3. When the number of the substituents is two or more, the respective substituents may be the same or different.

In the present specification, examples of the "acyl group" include a formyl group, a carboxy group, a carbamoyl group, a thiocarbamoyl group, a sulfino group, a sulfo group, a sulfamoyl group and a phosphono group, each optionally having "1 or 2 substituents selected from a C₁₋₆ alkyl group, a C₂₋₆ alkenyl group, a C₃₋₁₀ cycloalkyl group, a C₃₋₁₀ cycloalkenyl group, a C₆₋₁₄ aryl group, a C₇₋₁₆ aralkyl group, a 5- to 14-membered aromatic heterocyclic group and a 3- to 14-membered non-aromatic heterocyclic group, each of which optionally has 1 to 3 substituents selected from a halogen atom, an optionally halogenated C₁₋₆ alkoxy group, a hydroxy group, a nitro group, a cyano group, an amino group and a carbamoyl group".

Examples of the "acyl group" also include a hydrocarbon-sulfonyl group, a heterocyclylsulfonyl group, a hydrocarbon-sulfinyl group and a heterocyclylsulfinyl group.

Here, the hydrocarbon-sulfonyl group means a hydrocarbon group-bonded sulfonyl group, the heterocyclylsulfonyl group means a heterocyclic group-bonded sulfonyl group, the hydrocarbon-sulfinyl group means a hydrocarbon group-bonded sulfinyl group and the heterocyclylsulfinyl group means a heterocyclic group-bonded sulfinyl group.

Preferable examples of the "acyl group" include a formyl group, a carboxy group, a C₁₋₆ alkyl-carbonyl group, a C₂₋₆ alkenyl-carbonyl group (e.g., crotonoyl), a C₃₋₁₀ cycloalkylcarbonyl group (e.g., cyclobutanecarbonyl, cyclopentanecarbonyl, cyclohexanecarbonyl, cycloheptanecarbonyl), a C₃₋₁₀ cycloalkenyl-carbonyl group (e.g., 2-cyclohexenecarbonyl), a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a C₆₋₁₄ aryloxy-carbonyl group (e.g., phenyloxycarbonyl, naphthyloxycarbonyl), a C₇₋₁₆ aralkyloxy-carbonyl group (e.g., benzyloxycarbonyl, phenethyloxycarbonyl), a carbamoyl group, a mono- or di-C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl), a mono- or di-C₆₋₁₄ aryl-carbamoyl group (e.g., phenylcarbamoyl), a mono- or di-C₇₋₁₆ aralkyl-carbamoyl group, a 5- to 14-membered aromatic heterocyclylcarbamoyl group (e.g., pyridylcarbamoyl), a thiocarbamoyl group, a mono- or di-C₁₋₆ alkyl-thiocarbamoyl group (e.g., methylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkylthiocarbamoyl group (e.g., benzylthiocarbamoyl, phenethylthiocarbamoyl), a 5- to 14-membered aromatic heterocyclylthiocarbamoyl group (e.g., pyridylthiocarbamoyl), a sulfino group, a C₁₋₆ alkylsulfinyl group (e.g., methylsulfinyl, ethylsulfinyl), a sulfo group, a C₁₋₆ alkylsulfonyl group, a C₆₋₁₄ arylsulfonyl group, a phosphono group and a mono- or di-C₁₋₆ alkylphosphono group (e.g., dimethylphosphono, diethylphosphono, diisopropylphosphono, dibutylphosphono).

In the present specification, examples of the "optionally substituted amino group" include an amino group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from substituent group A".

Preferable examples of the optionally substituted amino group include an amino group, a mono- or di-(optionally halogenated C₁₋₆ alkyl)amino group (e.g., methylamino, trifluoromethylamino, dimethylamino, ethylamino, diethylamino, propylamino, dibutylamino), a mono- or di-C₂₋₆ alkenylamino group (e.g., diallylamino), a mono- or di-C₃₋₁₀ cycloalkylamino group (e.g., cyclopropylamino, cyclohexylamino), a mono- or di-C₆₋₁₄ arylamino group (e.g., phenylamino), a mono- or di-C₇₋₁₆ aralkylamino group (e.g., benzylamino, dibenzylamino), a mono- or di-(optionally halogenated C₁₋₆ alkyl)-carbonylamino group (e.g., acetylamino, propionylamino), a mono- or di-C₆₋₁₄ aryl-carbonylamino group (e.g., benzoylamino), a mono- or di-C₇₋₁₆ aralkyl-carbonylamino group (e.g., benzylcarbonylamino), a mono- or di-5- to 14-membered aromatic
heterocyclylcarbonylamino group (e.g., nicotinoylamino, isonicotinoylamino), a mono- or di-3- to 14-membered non-aromatic heterocyclylcarbonylamino group (e.g., piperidinylcarbonylamino), a mono- or di-C₁₋₆ alkoxycarbonylamino group (e.g., tert-butoxycarbonylamino), a 5- to 14-membered aromatic heterocyclylamino group (e.g., pyridylamino), a carbamoylamino group, a (mono- or di-C₁₋₆ alkyl-carbamoyl)amino group (e.g., methylcarbamoylamino), a (mono- or di-C₇₋₁₆ aralkyl-carbamoyl) amino group (e.g., benzylcarbamoylamino), a C₁₋₆ alkylsulfonylamino group (e.g., methylsulfonylamino, ethylsulfonylamino), a C₆₋₁₄ arylsulfonylamino group (e.g., phenylsulfonylamino), a (C₁₋₆ alkyl)(C₁₋₆ alkyl-carbonyl)amino group (e.g., N-acetyl-N-methylamino) and a (C₁₋₆ alkyl) (C₆₋₁₄ aryl-carbonyl) amino group (e.g., N-benzoyl-N-methylamino).

In the present specification, examples of the "optionally substituted carbamoyl group" include a carbamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5-to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted carbamoyl group include a carbamoyl group, a mono- or di-C₁₋₆ alkyl-carbamoyl group, a mono- or di-C₂₋₆ alkenyl-carbamoyl group (e.g., diallylcarbamoyl), a mono- or di-C₃₋₁₀ cycloalkyl-carbamoyl group (e.g., cyclopropylcarbamoyl,
cyclohexylcarbamoyl), a mono- or di-C₆₋₁₄ aryl-carbamoyl group (e.g., phenylcarbamoyl), a mono- or di-C₇₋₁₆ aralkyl-carbamoyl group, a mono- or di-C₁₋₆ alkyl-carbonyl-carbamoyl group (e.g., acetylcarbamoyl, propionylcarbamoyl), a mono- or di-C₆₋₁₄ aryl-carbonyl-carbamoyl group (e.g., benzoylcarbamoyl) and a 5- to 14-membered aromatic heterocyclylcarbamoyl group (e.g., pyridylcarbamoyl).

In the present specification, examples of the "optionally substituted thiocarbamoyl group" include a thiocarbamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5-to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted thiocarbamoyl group include a thiocarbamoyl group, a mono- or di-C₁₋₆ alkyl-thiocarbamoyl group (e.g., methylthiocarbamoyl, ethylthiocarbamoyl, dimethylthiocarbamoyl, diethylthiocarbamoyl, N-ethyl-N-methylthiocarbamoyl), a mono- or di-C₂₋₆ alkenyl-thiocarbamoyl group (e.g., diallylthiocarbamoyl), a mono- or di-C₃₋₁₀ cycloalkyl-thiocarbamoyl group (e.g., cyclopropylthiocarbamoyl, cyclohexylthiocarbamoyl), a mono- or di-C₆₋₁₄ aryl-thiocarbamoyl group (e.g., phenylthiocarbamoyl), a mono- or di-C₇₋₁₆ aralkyl-thiocarbamoyl group (e.g., benzylthiocarbamoyl, phenethylthiocarbamoyl), a mono- or di-C₁₋₆ alkyl-carbonyl-thiocarbamoyl group (e.g., acetylthiocarbamoyl, propionylthiocarbamoyl), a mono- or di-C₆₋₁₄ aryl-carbonyl-thiocarbamoyl group (e.g., benzoylthiocarbamoyl) and a 5- to 14-membered aromatic heterocyclylthiocarbamoyl group (e.g., pyridylthiocarbamoyl).

In the present specification, examples of the "optionally substituted sulfamoyl group" include a sulfamoyl group optionally having "1 or 2 substituents selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5-to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group and a mono- or di-C₇₋₁₆ aralkyl-carbamoyl group, each of which optionally has 1 to 3 substituents selected from substituent group A".

Preferable examples of the optionally substituted sulfamoyl group include a sulfamoyl group, a mono- or di-C₁₋₆ alkyl-sulfamoyl group (e.g., methylsulfamoyl, ethylsulfamoyl, dimethylsulfamoyl, diethylsulfamoyl, N-ethyl-N-methylsulfamoyl), a mono- or di-C₂₋₆ alkenyl-sulfamoyl group (e.g., diallylsulfamoyl), a mono- or di-C₃₋₁₀ cycloalkyl-sulfamoyl group (e.g., cyclopropylsulfamoyl, cyclohexylsulfamoyl), a mono- or di-C₆₋₁₄ aryl-sulfamoyl group (e.g., phenylsulfamoyl), a mono- or di-C₇₋₁₆ aralkyl-sulfamoyl group (e.g.,
benzylsulfamoyl, phenethylsulfamoyl), a mono- or di-C₁₋₆ alkyl-carbonyl-sulfamoyl group (e.g., acetylsulfamoyl, propionylsulfamoyl), a mono- or di-C₆₋₁₄ aryl-carbonyl-sulfamoyl group (e.g., benzoylsulfamoyl) and a 5- to 14-membered aromatic heterocyclylsulfamoyl group (e.g., pyridylsulfamoyl).

In the present specification, examples of the "optionally substituted hydroxy group" include a hydroxyl group optionally having "a substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group, a C₇₋₁₆ aralkyl-carbonyl group, a 5- to 14-membered aromatic heterocyclylcarbonyl group, a 3- to 14-membered non-aromatic heterocyclylcarbonyl group, a C₁₋₆ alkoxy-carbonyl group, a 5- to 14-membered aromatic heterocyclic group, a carbamoyl group, a mono- or di-C₁₋₆ alkyl-carbamoyl group, a mono- or di-C₇₋₁₆ aralkyl-carbamoyl group, a C₁₋₆ alkylsulfonyl group and a C₆₋₁₄ arylsulfonyl group, each of which optionally has 1 to 3 substituents selected from substituent group A".

Preferable examples of the optionally substituted hydroxy group include a hydroxy group, a C₁₋₆ alkoxy group, a C₂₋₆ alkenyloxy group (e.g., allyloxy, 2-butenyloxy, 2-pentenyloxy, 3-hexenyloxy), a C₃₋₁₀ cycloalkyloxy group (e.g., cyclohexyloxy), a C₆₋₁₄ aryloxy group (e.g., phenoxy, naphthyloxy), a C₇₋₁₆ aralkyloxy group (e.g., benzyloxy, phenethyloxy), a C₁₋₆ alkyl-carbonyloxy group (e.g., acetyloxy, propionyloxy, butyryloxy, isobutyryloxy, pivaloyloxy), a C₆₋₁₄ aryl-carbonyloxy group (e.g., benzoyloxy), a C₇₋₁₆ aralkyl-carbonyloxy group (e.g., benzylcarbonyloxy), a 5- to 14-membered aromatic heterocyclylcarbonyloxy group (e.g., nicotinoyloxy), a 3- to 14-membered non-aromatic heterocyclylcarbonyloxy group (e.g., piperidinylcarbonyloxy), a C₁₋₆ alkoxy-carbonyloxy group (e.g., tert-butoxycarbonyloxy), a 5- to 14-membered aromatic heterocyclyloxy group (e.g., pyridyloxy), a carbamoyloxy group, a C₁₋₆ alkyl-carbamoyloxy group (e.g., methylcarbamoyloxy), a C₇₋₁₆ aralkyl-carbamoyloxy group (e.g., benzylcarbamoyloxy), a C₁₋₆ alkylsulfonyloxy group (e.g., methylsulfonyloxy, ethylsulfonyloxy) and a C₆₋₁₄ arylsulfonyloxy group (e.g., phenylsulfonyloxy).

In the present specification, examples of the "optionally substituted sulfanyl group" include a sulfanyl group optionally having "a substituent selected from a C₁₋₆ alkyl group, a C₂₋₆ alkenyl group, a C₃₋₁₀ cycloalkyl group, a C₆₋₁₄ aryl group, a C₇₋₁₆ aralkyl group, a C₁₋₆ alkyl-carbonyl group, a C₆₋₁₄ aryl-carbonyl group and a 5- to 14-membered aromatic heterocyclic group, each of which optionally has 1 to 3 substituents selected from substituent group A" and a halogenated sulfanyl group.

Preferable examples of the optionally substituted sulfanyl group include a sulfanyl (-SH) group, a C₁₋₆ alkylthio group, a C₂₋₆ alkenylthio group (e.g., allylthio, 2-butenylthio, 2-pentenylthio, 3-hexenylthio), a C₃₋₁₀ cycloalkylthio group (e.g., cyclohexylthio), a C₆₋₁₄ arylthio group (e.g., phenylthio, naphthylthio), a C₇₋₁₆ aralkylthio group (e.g., benzylthio, phenethylthio), a C₁₋₆ alkyl-carbonylthio group (e.g., acetylthio, propionylthio, butyrylthio, isobutyrylthio, pivaloylthio), a C₆₋₁₄ aryl-carbonylthio group (e.g., benzoylthio), a 5- to 14-membered aromatic heterocyclylthio group (e.g., pyridylthio) and a halogenated thio group (e.g., pentafluorothio).

In the present specification, examples of the "optionally substituted silyl group" include a silyl group optionally having "1 to 3 substituents selected from a C₁₋₆ alkyl group, a C₂₋₆ alkenyl group, a C₃₋₁₀ cycloalkyl group, a C₆₋₁₄ aryl group and a C₇₋₁₆ aralkyl group, each of which optionally has 1 to 3 substituents selected from substituent group A".

Preferable examples of the optionally substituted silyl group include a tri-C₁₋₆ alkylsilyl group (e.g., trimethylsilyl, tert-butyl(dimethyl)silyl).

In the present specification, examples of the "hydrocarbon ring" include a C₆₋₁₄ aromatic hydrocarbon ring, C₃₋₁₀ cycloalkane and C₃₋₁₀ cycloalkene.

In the present specification, examples of the "C₆₋₁₄ aromatic hydrocarbon ring" include benzene and naphthalene.

In the present specification, examples of the "C₃₋₁₀ cycloalkane" include cyclopropane, cyclobutane, cyclopentane, cyclohexane, cycloheptane and cyclooctane.

In the present specification, examples of the "C₃₋₁₀ cycloalkene" include cyclopropene, cyclobutene, cyclopentene, cyclohexene, cycloheptene and cyclooctene.

In the present specification, examples of the "heterocycle" include an aromatic heterocycle and a non-aromatic heterocycle, each containing, as a ring-constituting atom besides carbon atom, 1 to 4 hetero atoms selected from a nitrogen atom, a sulfur atom and an oxygen atom.

In the present specification, examples of the "aromatic heterocycle" include a 5- to 14-membered (preferably 5- to 10-membered) aromatic heterocycle containing, as a ring-constituting atom besides carbon atom, 1 to 4 hetero atoms selected from a nitrogen atom, a sulfur atom and an oxygen atom. Preferable examples of the "aromatic heterocycle" include 5- or 6-membered monocyclic aromatic heterocycles such as thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, triazine and the like; and 8- to 14-membered fused polycyclic (preferably bi or tricyclic) aromatic heterocycles such as benzothiophene, benzofuran, benzimidazole, benzoxazole, benzisoxazole, benzothiazole, benzisothiazole, benzotriazole, imidazopyridine, thienopyridine, furopyridine, pyrrolopyridine, pyrazolopyridine, oxazolopyridine, thiazolopyridine, imidazopyrazine, imidazopyrimidine, thienopyrimidine, furopyrimidine, pyrrolopyrimidine, pyrazolopyrimidine, oxazolopyrimidine, thiazolopyrimidine, pyrazolopyrimidine, pyrazolotriazine, naphtho[2,3-b]thiophene, phenoxathiin, indole, isoindole, 1H-indazole, purine, isoquinoline, quinoline, phthalazine, naphthyridine, quinoxaline, quinazoline, cinnoline, carbazole, β-carboline, phenanthridine, acridine, phenazine, phenothiazine, phenoxazine and the like.

In the present specification, examples of the "non-aromatic heterocycle" include a 3- to 14-membered (preferably 4- to 10-membered) non-aromatic heterocycle containing, as a ring-constituting atom besides carbon atom, 1 to 4 hetero atoms selected from a nitrogen atom, a sulfur atom and an oxygen atom. Preferable examples of the "non-aromatic heterocycle" include 3- to 8-membered monocyclic non-aromatic heterocycles such as aziridine, oxirane, thiirane, azetidine, oxetane, thietane, tetrahydrothiophene, tetrahydrofuran, pyrroline, pyrrolidine, imidazoline, imidazolidine, oxazoline, oxazolidine, pyrazoline, pyrazolidine, thiazoline, thiazolidine, tetrahydroisothiazole, tetrahydrooxazole, tetrahydroisoxazole, piperidine, piperazine, tetrahydropyridine, dihydropyridine, dihydrothiopyran, tetrahydropyrimidine, tetrahydropyridazine, dihydropyran, tetrahydropyran, tetrahydrothiopyran, morpholine, thiomorpholine, azepanine, diazepane, azepine, azocane, diazocane, oxepane and the like; and
9- to 14-membered fused polycyclic (preferably bi or tricyclic) non-aromatic heterocycles such as dihydrobenzofuran, dihydrobenzimidazole, dihydrobenzoxazole, dihydrobenzothiazole, dihydrobenzisothiazole, dihydronaphtho[2,3-b]thiophene, tetrahydroisoquinoline, tetrahydroquinoline, 4H-quinolizine, indoline, isoindoline, tetrahydrothieno[2,3-c]pyridine, tetrahydrobenzazepine, tetrahydroquinoxaline,
tetrahydrophenanthridine, hexahydrophenothiazine,
hexahydrophenoxazine, tetrahydrophthalazine, tetrahydronaphthyridine, tetrahydroquinazoline, tetrahydrocinnoline, tetrahydrocarbazole, tetrahydro-β-carboline, tetrahydroacridine, tetrahydrophenazine, tetrahydrothioxanthene, octahydroisoquinoline and the like.

In the present specification, examples of the "nitrogen-containing heterocycle" include the "heterocycle" containing at least one nitrogen atom as a ring-constituting atom.

In the present specification, as the "5-membered or 6-membered aromatic heterocycle" of the "optionally further substituted 5-membered or 6-membered aromatic heterocycle", 5-membered or 6-membered ones from the above-mentioned "aromatic heterocycle" can be mentioned and, as the substituent thereof, the above-mentioned "substituent" can be mentioned.

In the present specification, as the "ring" of the "optionally substituted ring", the above-mentioned "hydrocarbon ring" and "heterocycle" can be mentioned and, as the substituent thereof, the above-mentioned "substituent" can be mentioned.

The definition of each symbol in the formula (I) is described below.

Z is N or CR¹, and R¹ is a hydrogen atom or a substituent.

As the "substituent" for R¹, a halogen atom (e.g., fluorine atom, bromine atom) can be mentioned.

Z is preferably N or CR¹ (R¹ is a hydrogen atom or a halogen atom (e.g., fluorine atom, bromine atom)), more preferably, N or CR¹ (R¹ is a hydrogen atom, a fluorine atom or a bromine atom), still more preferably, CR¹ (R¹ is a hydrogen atom).

R² is a substituent.

As the "substituent" for R², an optionally substituted C₁₋₆ alkyl group (e.g., methyl, ethyl, n-propyl), an optionally substituted C₁₋₆ alkoxy group (e.g., methoxy, ethoxy, n-propoxy, isopropoxy, n-butoxy, sec-butoxy, n-pentoxy), an optionally substituted C₃₋₁₀ cycloalkyl group (e.g., cyclopropyl), an optionally substituted C₃₋₁₀ cycloalkyloxy group (e.g., cyclobutyloxy, cyclohexyloxy), an optionally substituted C₆₋₁₄ aryl group (e.g., phenyl), an optionally substituted C₆₋₁₄ aryloxy group (e.g., phenoxy), an optionally substituted nonaromatic heterocyclic group (e.g., tetrahydropyranyl), an optionally substituted non-aromatic heterocyclyl-oxy group (e.g., piperidinyloxy, tetrahydropyranyloxy), and an optionally substituted mono- or di-C₁₋₆ alkylamino group (e.g., ethyl(methyl)amino) can be mentioned.

R² is preferably
(1) a C₁₋₆ alkyl group (e.g., methyl, ethyl, n-propyl),
(2) an optionally substituted C₁₋₆ alkoxy group (e.g., methoxy, ethoxy, n-propoxy, isopropoxy, n-butoxy, sec-butoxy, n-pentoxy),
(3) a C₃₋₁₀ cycloalkyl group (e.g., cyclopropyl),
(4) an optionally substituted C₃₋₁₀ cycloalkyloxy group (e.g., cyclobutyloxy, cyclohexyloxy),
(5) a C₆₋₁₄ aryl group (e.g., phenyl),
(6) a C₆₋₁₄ aryloxy group (e.g., phenoxy),
(7) a nonaromatic heterocyclic group (e.g., tetrahydropyranyl),
(8) an optionally substituted non-aromatic heterocyclyloxy group (e.g., piperidinyloxy, tetrahydropyranyloxy), or
(9) an optionally substituted mono- or di-C₁₋₆ alkylamino group (e.g., ethyl(methyl)amino), more preferably,
   (1) a C₁₋₆ alkyl group (e.g., methyl, ethyl, n-propyl),
   (2) a C₁₋₆ alkoxy group (e.g., methoxy, ethoxy, n-propoxy, isopropoxy, n-butoxy, sec-butoxy, n-pentoxy) optionally substituted by 1-5 substituents selected from deuterium, a halogen atom (e.g., fluorine atom), a hydroxy group, a C₁₋₆ alkoxy group (e.g., methoxy), a C₃₋₁₀ cycloalkyl group (e.g., cyclopropyl), a C₁₋₆ alkyl-carbonyloxy group (e.g., acetyloxy) and a tri-C₁₋₆ alkylsilyl-oxy group (e.g., tert-butyl(dimethyl)silyloxy),
   (3) a C₃₋₁₀ cycloalkyl group (e.g., cyclopropyl),
   (4) a C₃₋₁₀ cycloalkyloxy group (e.g., cyclobutyloxy, cyclohexyloxy) optionally substituted by 1 - 5 substituents selected from a di-C₁₋₆ alkylamino group (e.g., dimethylamino) and a nonaromatic heterocyclic group (e.g., morpholinyl),
   (5) a C₆₋₁₄ aryl group (e.g., phenyl),
   (6) a C₆₋₁₄ aryloxy group (e.g., phenoxy),
   (7) a nonaromatic heterocyclic group (e.g., tetrahydropyranyl),
   (8) a non-aromatic heterocyclyl-oxy group (e.g., piperidinyloxy, tetrahydropyranyloxy) optionally substituted by a C₁₋₆ alkyl group (e.g., methyl), or
   (9) a mono- or di-C₁₋₆ alkylamino group (e.g., ethyl (methyl) amino) optionally substituted by a C₆₋₁₄ aryl group (e.g., phenyl).

In another embodiment of the present invention, R² is more preferably
(1) a C₁₋₆ alkyl group (e.g., methyl, ethyl, n-propyl),
(2) a C₁₋₆ alkoxy group (e.g., methoxy, ethoxy, n-propoxy, isopropoxy, n-butoxy, sec-butoxy, n-pentoxy) optionally substituted by 1 - 5 substituents selected from a halogen atom (e.g., fluorine atom), a hydroxy group, a C₁₋₆ alkoxy group (e.g., methoxy), a C₃₋₁₀ cycloalkyl group (e.g., cyclopropyl), a C₁₋₆ alkyl-carbonyloxy group (e.g., acetyloxy) and a tri-C₁₋₆ alkylsilyl-oxy group (e.g., tert-butyl(dimethyl)silyloxy),
(3) a C₃₋₁₀ cycloalkyl group (e.g., cyclopropyl),
(4) a C₃₋₁₀ cycloalkyloxy group (e.g., cyclobutyloxy, cyclohexyloxy) optionally substituted by 1 - 5 substituents selected from a di-C₁₋₆ alkylamino group (e.g., dimethylamino) and a nonaromatic heterocyclic group (e.g., morpholinyl),
(5) a C₆₋₁₄ aryl group (e.g., phenyl),
(6) a C₆₋₁₄ aryloxy group (e.g., phenoxy),
(7) a nonaromatic heterocyclic group (e.g., tetrahydropyranyl),
(8) a non-aromatic heterocyclyl-oxy group (e.g., piperidinyloxy, tetrahydropyranyloxy) optionally substituted by a C₁₋₆ alkyl group (e.g., methyl), or
(9) a mono- or di-C₁₋₆ alkylamino group (e.g., ethyl (methyl) amino) optionally substituted by a C₆₋₁₄ aryl group (e.g., phenyl).

R³ is a hydrogen atom or a substituent.

As the "substituent" for R³, a halogen atom (e.g., chlorine atom), a cyano group, optionally substituted hydroxy group, an optionally substituted C₁₋₆ alkyl group (e.g., methyl, ethyl, isopropyl), an optionally substituted C₂₋₆ alkenyl group (e.g., allyl), an optionally substituted C₃₋₁₀ cycloalkyl group (e.g., cyclopropyl), an optionally substituted C₁₋₆ alkoxy group (e.g., methoxy, ethoxy, isopropoxy), an optionally substituted C₃₋₁₀ cycloalkyloxy group (e.g., cyclopropyloxy), an optionally substituted amino group (e.g., amino group, optionally substituted mono- or di-C₁₋₆ alkylamino group (e.g., methylamino, ethylamino, dimethylamino, ethyl(methyl)amino), an optionally substituted (C₁₋₆ alkyl)(3- to 14-membered non-aromatic heterocyclyl)amino group (e.g., methyl(piperidinyl)amino), optionally substituted mono- or di-C₇₋₁₆ aralkylamino group (e.g., benzylamino)), an optionally substituted carbamoyl group, an optionally substituted nonaromatic heterocyclic group (e.g., pyrrolidinyl, piperidinyl, piperazinyl, tetrahydropyridinyl, morpholinyl, 1,4-diazepanyl), and an optionally substituted 7-to 10-membered crosslinked heterocyclic group (e.g., 3,8-diazabicyclo[3.2.1]octanyl) can be mentioned.

R³ is preferably
(1) a hydrogen atom,
(2) a halogen atom (e.g., chlorine atom),
(3) a cyano group,
(4) a hydroxy group,
(5) an optionally substituted C₁₋₆ alkyl group (e.g., methyl, ethyl, isopropyl),
(6) a C₂₋₆ alkenyl group (e.g., allyl),
(7) a C₃₋₁₀ cycloalkyl group (e.g., cyclopropyl),
(8) an optionally substituted C₁₋₆ alkoxy group (e.g., methoxy, ethoxy, isopropoxy),
(9) a C₃₋₁₀ cycloalkyloxy group (e.g., cyclopropyloxy),
(10) an optionally substituted mono- or di-C₁₋₆ alkylamino group (e.g., methylamino, ethylamino, dimethylamino, ethyl(methyl)amino),
(11) a (C₁₋₆ alkyl) (3- to 14-membered non-aromatic heterocyclyl)amino group (e.g., methyl(piperidinyl)amino),
(12) a carbamoyl group,
(13) an optionally substituted nonaromatic heterocyclic group (e.g., piperazinyl, morpholinyl, 1,4-diazepanyl), or
(14) a 7- to 10-membered crosslinked heterocyclic group (e.g., 3,8-diazabicyclo[3.2.1]octanyl), more preferably,
   (1) a hydrogen atom,
   (2) a halogen atom (e.g., chlorine atom),
   (3) a cyano group,
   (4) a hydroxy group,
   (5) a C₁₋₆ alkyl group (e.g., methyl, ethyl, isopropyl) optionally substituted by 1 to 3 substituents selected from a halogen atom (e.g., fluorine atom), a C₃₋₁₀ cycloalkyl group (e.g., cyclopropyl) and an amino group,
   (6) a C₂₋₆ alkenyl group (e.g., allyl),
   (7) a C₃₋₁₀ cycloalkyl group (e.g., cyclopropyl),
   (8) a C₁₋₆ alkoxy group (e.g., methoxy, ethoxy, isopropoxy) optionally substituted by 1 to 3 substituents selected from deuterium, a halogen atom (e.g., fluorine atom), a C₁₋₆ alkoxy group (e.g., methoxy) and an amino group,
   (9) a C₃₋₁₀ cycloalkyloxy group (e.g., cyclopropyloxy),
   (10) a mono- or di-C₁₋₆ alkylamino group (e.g., methylamino, ethylamino, dimethylamino, ethyl(methyl)amino) optionally substituted by a mono- or di-C₁₋₆ alkylamino group (e.g., dimethylamino),
   (11) a (C₁₋₆ alkyl) (3- to 14-membered non-aromatic heterocyclyl)amino group (e.g., methyl(piperidinyl)amino),
   (12) a carbamoyl group,
   (13) a nonaromatic heterocyclic group (e.g., pyrrolidinyl, piperidinyl, piperazinyl, tetrahydropyridinyl, morpholinyl, 1,4-diazepanyl) optionally substituted by 1 to 3 substituents selected from a hydroxy group, an oxo group, a C₁₋₆ alkyl group (e.g., methyl, ethyl, isobutyl) optionally substituted by a hydroxy group, a C₆₋₁₄ aryl group (e.g., phenyl), a mono- or di-C₁₋₆ alkylamino group (e.g., dimethylamino) and a C₁₋₆ alkoxy-carbonyl group (e.g., tert-butoxycarbonyl), or
   (14) a 7- to 10-membered crosslinked heterocyclic group (e.g., 3,8-diazabicyclo[3.2.1]octanyl).

In another embodiment of the present invention, R³ is preferably a hydrogen atom, a halogen atom (e.g., chlorine atom), a cyano group, a hydroxy group, an optionally substituted C₁₋₆ alkyl group (e.g., methyl, ethyl, isopropyl), an optionally substituted C₂₋₆ alkenyl group (e.g., allyl), an optionally substituted C₃₋₁₀ cycloalkyl group (e.g., cyclopropyl), an optionally substituted C₁₋₆ alkoxy group (e.g., methoxy, ethoxy, isopropoxy), an optionally substituted C₃₋₁₀ cycloalkyloxy group (e.g., cyclopropyloxy), an optionally substituted amino group, an optionally substituted carbamoyl group, an optionally substituted nonaromatic heterocyclic group (e.g., piperazinyl, morpholinyl, 1,4-diazepanyl) or an optionally substituted 7- to 10-membered crosslinked heterocyclic group (e.g., 3,8-diazabicyclo[3.2.1]octanyl), more preferably,
(1) a hydrogen atom,
(2) a halogen atom (e.g., chlorine atom),
(3) a cyano group,
(4) a hydroxy group,
(5) an optionally substituted C₁₋₆ alkyl group (e.g., methyl, ethyl, isopropyl),
(6) a C₂₋₆ alkenyl group (e.g., allyl),
(7) a C₃₋₁₀ cycloalkyl group (e.g., cyclopropyl),
(8) an optionally substituted C₁₋₆ alkoxy group (e.g., methoxy, ethoxy, isopropoxy),
(9) a C₃₋₁₀ cycloalkyloxy group (e.g., cyclopropyloxy),
(10) an amino group,
(11) an optionally substituted mono- or di-C₁₋₆ alkylamino group (e.g., methylamino, ethylamino, dimethylamino, ethyl(methyl)amino),
(12) a (C₁₋₆ alkyl)(3- to 14-membered non-aromatic heterocyclyl)amino group (e.g., methyl(piperidinyl)amino),
(13) a mono- or di-C₇₋₁₆ aralkylamino group (e.g., benzylamino),
(14) a carbamoyl group,
(15) an optionally substituted nonaromatic heterocyclic group (e.g., piperazinyl, morpholinyl, 1,4-diazepanyl), or
(16) a 7- to 10-membered crosslinked heterocyclic group (e.g., 3,8-diazabicyclo[3.2.1]octanyl), still more preferably,
   (1) a hydrogen atom,
   (2) a halogen atom (e.g., chlorine atom),
   (3) a cyano group,
   (4) a hydroxy group,
   (5) a C₁₋₆ alkyl group (e.g., methyl, ethyl, isopropyl) optionally substituted by 1 to 3 substituents selected from a halogen atom (e.g., fluorine atom), a C₃₋₁₀ cycloalkyl group (e.g., cyclopropyl) and an amino group,
   (6) a C₂₋₆ alkenyl group (e.g., allyl),
   (7) a C₃₋₁₀ cycloalkyl group (e.g., cyclopropyl),
   (8) a C₁₋₆ alkoxy group (e.g., methoxy, ethoxy, isopropoxy) optionally substituted by 1 to 3 substituents selected from a halogen atom (e.g., fluorine atom), a C₁₋₆ alkoxy group (e.g., methoxy) and an amino group,
   (9) a C₃₋₁₀ cycloalkyloxy group (e.g., cyclopropyloxy),
   (10) an amino group,
   (11) a mono- or di-C₁₋₆ alkylamino group (e.g., methylamino, ethylamino, dimethylamino, ethyl(methyl)amino) optionally substituted by a mono- or di-C₁₋₆ alkylamino group (e.g., dimethylamino),
   (12) a (C₁₋₆ alkyl)(3- to 14-membered non-aromatic heterocyclyl)amino group (e.g., methyl(piperidinyl)amino),
   (13) a mono- or di-C₇₋₁₆ aralkylamino group (e.g., benzylamino),
   (14) a carbamoyl group,
   (15) a nonaromatic heterocyclic group (e.g., pyrrolidinyl, piperidinyl, piperazinyl, tetrahydropyridinyl, morpholinyl, 1,4-diazepanyl) optionally substituted by 1 to 3 substituents selected from a hydroxy group, an oxo group, a C₁₋₆ alkyl group (e.g., methyl, ethyl, isobutyl) optionally substituted by a hydroxy group, a C₆₋₁₄ aryl group (e.g., phenyl), a mono- or di-C₁₋₆ alkylamino group (e.g., dimethylamino) and a C₁₋₆ alkoxy-carbonyl group (e.g., tert-butoxycarbonyl), or
   (16) a 7- to 10-membered crosslinked heterocyclic group (e.g., 3,8-diazabicyclo[3.2.1]octanyl).

R⁴ is a hydrogen atom or a substituent.

As the "substituent" for R⁴, a halogen atom (e.g., fluorine atom, chlorine atom), an optionally substituted C₁₋₆ alkyl group (e.g., methyl), an optionally substituted C₃₋₁₀ cycloalkyl group (e.g., cyclopropyl), an optionally substituted C₆₋₁₄ aryl group (e.g., phenyl), an optionally substituted C₁₋₆ alkoxy group (e.g., methoxy, ethoxy), an optionally substituted C₁₋₆ alkyl-carbonylamino group (e.g., methylcarbonylamino), an optionally substituted carbamoyl group, an optionally substituted nonaromatic heterocyclic group (e.g., pyrrolidinyl, dihydropyranyl), and an optionally substituted aromatic heterocyclic group (e.g., furanyl, thienyl, pyrazolyl, thiazolyl, pyridyl, indazolyl, pyrazolopyridyl, isoquinolinyl) can be mentioned.

R⁴ is preferably
(1) a hydrogen atom,
(2) a halogen atom (e.g., chlorine atom),
(3) a C₁₋₆ alkyl group (e.g., methyl),
(4) an optionally substituted C₁₋₆ alkoxy group (e.g., methoxy, ethoxy),
(5) a C₁₋₆ alkyl-carbonylamino group (e.g., methylcarbonylamino),
(6) a carbamoyl group,
(7) an optionally substituted nonaromatic heterocyclic group (e.g., pyrrolidinyl), or
(8) an optionally substituted aromatic heterocyclic group (e.g., pyrazolyl), more preferably,
   (1) a hydrogen atom,
   (2) a halogen atom (e.g., chlorine atom),
   (3) a C₁₋₆ alkyl group (e.g., methyl),
   (4) a C₁₋₆ alkoxy group (e.g., methoxy, ethoxy) optionally substituted by 1 to 3 substituents selected from a halogen atom (e.g., fluorine atom) and a C₁₋₆ alkoxy group (e.g., methoxy),
   (5) a C₁₋₆ alkyl-carbonylamino group (e.g., methylcarbonylamino),
   (6) a carbamoyl group,
   (7) a nonaromatic heterocyclic group (e.g., pyrrolidinyl) optionally substituted by an oxo group, or
   (8) an aromatic heterocyclic group (e.g., pyrazolyl) optionally substituted by a C₁₋₆ alkyl group (e.g., methyl).

In another embodiment of the present invention, R⁴ is preferably a hydrogen atom, a halogen atom (e.g., fluorine atom, chlorine atom), an optionally substituted C₁₋₆ alkyl group (e.g., methyl), an optionally substituted C₃₋₁₀ cycloalkyl group (e.g., cyclopropyl), an optionally substituted C₆₋₁₄ aryl group (e.g., phenyl), an optionally substituted C₁₋₆ alkoxy group (e.g., methoxy, ethoxy), an optionally substituted C₁₋₆ alkyl-carbonylamino group (e.g., methylcarbonylamino), an optionally substituted carbamoyl group, an optionally substituted nonaromatic heterocyclic group (e.g., pyrrolidinyl, dihydropyranyl) or an optionally substituted aromatic heterocyclic group (e.g., furanyl, thienyl, pyrazolyl, thiazolyl, pyridyl, indazolyl, pyrazolopyridyl, isoquinolinyl), more preferably,
(1) a hydrogen atom,
(2) a halogen atom (e.g., fluorine atom, chlorine atom),
(3) a C₁₋₆ alkyl group (e.g., methyl),
(4) a C₃₋₁₀ cycloalkyl group (e.g., cyclopropyl),
(5) an optionally substituted C₆₋₁₄ aryl group (e.g., phenyl),
(6) an optionally substituted C₁₋₆ alkoxy group (e.g., methoxy, ethoxy),
(7) a C₁₋₆ alkyl-carbonylamino group (e.g., methylcarbonylamino),
(8) a carbamoyl group,
(9) an optionally substituted nonaromatic heterocyclic group (e.g., pyrrolidinyl, dihydropyranyl), or
(10) an optionally substituted aromatic heterocyclic group (e.g., furanyl, thienyl, pyrazolyl, thiazolyl, pyridyl, indazolyl, pyrazolopyridyl, isoquinolinyl), still more preferably,
   (1) a hydrogen atom,
   (2) a halogen atom (e.g., chlorine atom),
   (3) a C₁₋₆ alkyl group (e.g., methyl),
   (4) a C₃₋₁₀ cycloalkyl group (e.g., cyclopropyl),
   (5) a C₆₋₁₄ aryl group (e.g., phenyl) optionally substituted by 1 to 3 halogen atoms (e.g., fluorine atom),
   (6) a C₁₋₆ alkoxy group (e.g., methoxy, ethoxy) optionally substituted by 1 to 3 substituents selected from a halogen atom (e.g., fluorine atom) and a C₁₋₆ alkoxy group (e.g., methoxy),
   (7) a C₁₋₆ alkyl-carbonylamino group (e.g., methylcarbonylamino),
   (8) a carbamoyl group,
   (9) a nonaromatic heterocyclic group (e.g., pyrrolidinyl, dihydropyranyl) optionally substituted by an oxo group, or
   (10) an aromatic heterocyclic group (e.g., furanyl, thienyl, pyrazolyl, thiazolyl, pyridyl, indazolyl, pyrazolopyridyl, isoquinolinyl) optionally substituted by 1 to 3 substituents selected from (i) a C₁₋₆ alkyl group (e.g., methyl, ethyl) optionally substituted by 1 to 3 substituents selected from a C₃₋₁₀ cycloalkyl group (e.g., cyclopropyl) and an aromatic heterocyclic group (e.g., pyridyl) and (ii) a nonaromatic heterocyclic group (e.g., tetrahydropyranyl).

Alternatively, R⁴ and R³ are joined to form an optionally substituted ring.

As the "ring" of the "optionally substituted ring" jointly formed by R⁴ and R³, a non-aromatic heterocycle (e.g., dihydropyrrole ring, dihydrofuran ring, dihydrothiophene ring, tetrahydropyridine ring, 3,4-dihydro-2H-1,4-oxazine ring, 2,3-dihydro-1,4-dioxin ring, 2,3-dihydro-1,4-oxathiin ring, 6,7-dihydro-5H-1,4-dioxepine ring) and an aromatic heterocycle (e.g., furan ring, thiophene ring, imidazole ring) can be mentioned.

As the "substituent" of the "optionally substituted ring" jointly formed by R⁴ and R³, an optionally substituted C₁₋₆ alkyl group (e.g., methyl, ethyl), an oxo group, an optionally substituted C₁₋₆ alkyl-carbonyl group (e.g., methylcarbonyl) and an optionally substituted C₁₋₆ alkylsulfonyl group (e.g., methylsulfonyl) can be mentioned.

The "optionally substituted ring" jointly formed by R⁴ and R³ is preferably a non-aromatic heterocycle (e.g., dihydropyrrole ring, dihydrofuran ring, dihydrothiophene ring, tetrahydropyridine ring, 3,4-dihydro-2H-1,4-oxazine ring, 2,3-dihydro-1,4-dioxin ring, 2,3-dihydro-1,4-oxathiin ring, 6,7-dihydro-5H-1,4-dioxepine ring) or an aromatic heterocycle (e.g., furan ring, thiophene ring, imidazole ring), each of which is optionally substituted by 1 to 3 substituents selected from an optionally substituted C₁₋₆ alkyl group (e.g., methyl, ethyl), an oxo group, an optionally substituted C₁₋₆ alkyl-carbonyl group (e.g., methylcarbonyl) and an optionally substituted C₁₋₆ alkylsulfonyl group (e.g., methylsulfonyl), more preferably,
a non-aromatic heterocycle (e.g., dihydropyrrole ring, dihydrofuran ring, dihydrothiophene ring, tetrahydropyridine ring, 3,4-dihydro-2H-1,4-oxazine ring, 2,3-dihydro-1,4-dioxin ring, 2,3-dihydro-1,4-oxathiin ring, 6,7-dihydro-5H-1,4-dioxepine ring) or an aromatic heterocycle (e.g., furan ring, thiophene ring, imidazole ring), each of which is optionally substituted by 1 to 3 substituents selected from a C₁₋₆ alkyl group (e.g., methyl, ethyl) optionally substituted by a hydroxy group or a C₆₋₁₄ aryl group (e.g., phenyl), an oxo group, a C₁₋₆ alkyl-carbonyl group (e.g., methylcarbonyl) and a C₁₋₆ alkylsulfonyl group (e.g., methylsulfonyl), still more preferably,
(1) a non-aromatic heterocycle (e.g., dihydropyrrole ring, dihydrofuran ring, dihydrothiophene ring, tetrahydropyridine ring, 3,4-dihydro-2H-1,4-oxazine ring, 2,3-dihydro-1,4-dioxin ring, 2,3-dihydro-1,4-oxathiin ring, 6,7-dihydro-5H-1,4-dioxepine ring) optionally substituted by 1 to 3 substituents selected from a C₁₋₆ alkyl group (e.g., methyl, ethyl) optionally substituted by a C₆₋₁₄ aryl group (e.g., phenyl), an oxo group, a C₁₋₆ alkyl-carbonyl group (e.g., methylcarbonyl) and a C₁₋₆ alkylsulfonyl group (e.g., methylsulfonyl), or
(2) an aromatic heterocycle (e.g., furan ring, thiophene ring, imidazole ring) optionally substituted by 1 to 3 substituents selected from a C₁₋₆ alkyl group (e.g., methyl, ethyl) optionally substituted by a hydroxy group.

Ring A is an optionally further substituted benzene ring, or an optionally further substituted 5-membered or 6-membered aromatic heterocycle.

As the "5-membered or 6-membered aromatic heterocycle" of the "optionally further substituted 5-membered or 6-membered aromatic heterocycle" for ring A, a pyridine ring, a pyrazine ring and a pyridazine ring can be mentioned.

The "benzene ring" or "5-membered or 6-membered aromatic heterocycle" is optionally further substituted by 1 - 3 (preferably 1 or 2) substituents other than a 1,3-dihydro-2H-imidazo[4,5-b]pyridin-2-one ring group or a 7,9-dihydro-8H-purin-8-one ring group at substitutable position(s).

As such "substituent", a halogen atom (e.g., fluorine atom, chlorine atom), a C₁₋₆ alkyl group (e.g., methyl) and a C₃₋₁₀ cycloalkyl group (e.g., cyclopropyl) can be mentioned.

Ring A is preferably an optionally further substituted benzene ring, or a pyridine ring, a pyrazine ring or a pyridazine ring, each of which is optionally further substituted, more preferably, a benzene ring optionally substituted by 1-3 (preferably 1 or 2) substituents selected from a halogen atom (e.g., fluorine atom, chlorine atom), a C₁₋₆ alkyl group (e.g., methyl) and a C₃₋₁₀ cycloalkyl group (e.g., cyclopropyl), or a pyridine ring, a pyrazine ring or a pyridazine ring, each of which is optionally further substituted, still more preferably, a benzene ring optionally further substituted by 1-3 (preferably 1 or 2) substituents selected from a halogen atom (e.g., fluorine atom, chlorine atom), a C₁₋₆ alkyl group (e.g., methyl) and a C₃₋₁₀ cycloalkyl group (e.g., cyclopropyl), or a pyridine ring, a pyrazine ring or a pyridazine ring.

Ring B is an optionally further substituted 5-membered or 6-membered aromatic heterocycle.

As the "5-membered or 6-membered aromatic heterocycle" of the "optionally further substituted 5-membered or 6-membered aromatic heterocycle" for ring B, a furan ring, an imidazole ring, a pyrazole ring, a pyridine ring, a pyrimidine ring, a pyridazine ring and a pyrazine ring can be mentioned.

The "5-membered or 6-membered aromatic heterocycle" is optionally further substituted by 1 or 2 (preferably 1) substituents other than R³ and R⁴ at substitutable position(s).

As such "substituent", a halogen atom (e.g., chlorine atom), a hydroxy group, a C₁₋₆ alkyl group (e.g., methyl), a C₁₋₆ alkoxy group (e.g., methoxy), an optionally substituted amino group can be mentioned.

Ring B is preferably a furan ring, an imidazole ring, a pyrazole ring, a pyridine ring, a pyrimidine ring, a pyridazine ring or a pyrazine ring, each of which is optionally further substituted, more preferably, a furan ring, an imidazole ring, a pyrazole ring, a pyridine ring, a pyrimidine ring, a pyridazine ring or a pyrazine ring, each of which is optionally further substituted by 1 or 2 (preferably 1) substituents selected from a halogen atom (e.g., chlorine atom), a hydroxy group, a C₁₋₆ alkyl group (e.g., methyl) and an amino group, still more preferably, a pyridine ring optionally further substituted by 1 or 2 (preferably 1) substituents selected from a halogen atom (e.g., chlorine atom), a hydroxy group, a C₁₋₆ alkyl group (e.g., methyl) and an amino group, or a furan ring, an imidazole ring, a pyrazole ring, a pyrimidine ring, a pyridazine ring or a pyrazine ring.

In another embodiment of the present invention, ring B is preferably a furan ring, an imidazole ring, a pyrazole ring, a pyridine ring, a pyrimidine ring, a pyridazine ring or a pyrazine ring, each of which is optionally further substituted, more preferably, a furan ring, an imidazole ring, a pyrazole ring, a pyridine ring, a pyrimidine ring, a pyridazine ring or a pyrazine ring, each of which is optionally substituted by 1 or 2 (preferably 1) substituents selected from a halogen atom (e.g., chlorine atom), a hydroxy group, a C₁₋₆ alkyl group (e.g., methyl), a C₁₋₆ alkoxy group (e.g., methoxy) and an amino group, still more preferably, a pyridine ring optionally further substituted by 1 or 2 (preferably 1) substituents selected from a halogen atom (e.g., chlorine atom), a hydroxy group, a C₁₋₆ alkyl group (e.g., methyl), a C₁₋₆ alkoxy group (e.g., methoxy) and an amino group, or a furan ring, an imidazole ring, a pyrazole ring, a pyrimidine ring, a pyridazine ring or a pyrazine ring.

Preferable examples of compound (I) include the following compounds.

### [Compound I-1]

Compound (I) wherein
Z is N or CR¹ (R¹ is a hydrogen atom or a halogen atom (e.g., fluorine atom, bromine atom));
R² is an optionally substituted C₁₋₆ alkyl group (e.g., methyl, ethyl, n-propyl), an optionally substituted C₁₋₆ alkoxy group (e.g., methoxy, ethoxy, n-propoxy, isopropoxy, n-butoxy), an optionally substituted C₃₋₁₀ cycloalkyl group (e.g., cyclopropyl), an optionally substituted C₃₋₁₀ cycloalkyloxy group (e.g., cyclohexyloxy), an optionally substituted C₆₋₁₄ aryl group (e.g., phenyl), an optionally substituted C₆₋₁₄ aryloxy group (e.g., phenoxy), an optionally substituted nonaromatic heterocyclic group (e.g., tetrahydropyranyl), an optionally substituted non-aromatic heterocyclyl-oxy group (e.g., piperidinyloxy, tetrahydropyranyloxy) or an optionally substituted mono- or di-C₁₋₆ alkylamino group (e.g., ethyl(methyl)amino);
R³ is a hydrogen atom, a halogen atom (e.g., chlorine atom), a cyano group, optionally substituted hydroxy group, an optionally substituted C₁₋₆ alkyl group (e.g., methyl, ethyl, isopropyl), an optionally substituted C₂₋₆ alkenyl group (e.g., allyl), an optionally substituted C₃₋₁₀ cycloalkyl group (e.g., cyclopropyl), an optionally substituted C₁₋₆ alkoxy group (e.g., methoxy, ethoxy, isopropoxy), an optionally substituted C₃₋₁₀ cycloalkyloxy group (e.g., cyclopropyloxy), optionally substituted mono- or di-C₁₋₆ alkylamino group (e.g., methylamino, ethylamino, dimethylamino, ethyl(methyl)amino), optionally substituted (C₁₋₆ alkyl)(3- to 14-membered non-aromatic heterocyclyl)amino group (e.g., methyl(piperidinyl)amino), carbamoyl group, an optionally substituted nonaromatic heterocyclic group (e.g., pyrrolidinyl, piperidinyl, piperazinyl, tetrahydropyridinyl, morpholinyl, 1,4-diazepanyl) or an optionally substituted 7- to 10-membered crosslinked heterocyclic group (e.g., 3,8-diazabicyclo[3.2.1]octanyl);
R⁴ is a hydrogen atom, a halogen atom (e.g., chlorine atom), an optionally substituted C₁₋₆ alkyl group (e.g., methyl), an optionally substituted C₁₋₆ alkoxy group (e.g., methoxy, ethoxy), an optionally substituted C₁₋₆ alkyl-carbonylamino group (e.g., methylcarbonylamino), carbamoyl group, an optionally substituted nonaromatic heterocyclic group (e.g., pyrrolidinyl) or an optionally substituted aromatic heterocyclic group (e.g., pyrazolyl); or
R⁴ and R³ are joined to form a non-aromatic heterocycle (e.g., dihydropyrrole ring, dihydrofuran ring, dihydrothiophene ring, tetrahydropyridine ring, 3,4-dihydro-2H-1,4-oxazine ring, 2,3-dihydro-1,4-dioxin ring, 2,3-dihydro-1,4-oxathiin ring, 6,7-dihydro-5H-1,4-dioxepine ring) or an aromatic heterocycle (e.g., furan ring, thiophene ring, imidazole ring), each of which is optionally substituted by 1 to 3 substituents selected from an optionally substituted C₁₋₆ alkyl group (e.g., methyl, ethyl), an oxo group, an optionally substituted C₁₋₆ alkyl-carbonyl group (e.g., methylcarbonyl) and an optionally substituted C₁₋₆ alkylsulfonyl group (e.g., methylsulfonyl);
ring A is an optionally further substituted benzene ring, or a pyridine ring, a pyrazine ring or a pyridazine ring, each of which is optionally further substituted; and
ring B is a furan ring, an imidazole ring, a pyrazole ring, a pyridine ring, a pyrimidine ring, a pyridazine ring or a pyrazine ring, each of which is optionally further substituted.

### [Compound I-2]

Compound (I) wherein
Z is N or CR¹ (R¹ is a hydrogen atom, a fluorine atom or a bromine atom);
R² is
(1) a C₁₋₆ alkyl group (e.g., methyl, ethyl, n-propyl),
(2) an optionally substituted C₁₋₆ alkoxy group (e.g., methoxy, ethoxy, n-propoxy, isopropoxy, n-butoxy, sec-butoxy, n-pentoxy),
(3) a C₃₋₁₀ cycloalkyl group (e.g., cyclopropyl),
(4) an optionally substituted C₃₋₁₀ cycloalkyloxy group (e.g., cyclohexyloxy),
(5) a C₆₋₁₄ aryl group (e.g., phenyl),
(6) a C₆₋₁₄ aryloxy group (e.g., phenoxy),
(7) a nonaromatic heterocyclic group (e.g., tetrahydropyranyl),
(8) an optionally substituted non-aromatic heterocyclyl-oxy group (e.g., piperidinyloxy, tetrahydropyranyloxy), or
(9) an optionally substituted mono- or di-C₁₋₆ alkylamino group (e.g., ethyl(methyl)amino);
R³ is
(1) a hydrogen atom,
(2) a halogen atom (e.g., chlorine atom),
(3) a cyano group,
(4) a hydroxy group,
(5) an optionally substituted C₁₋₆ alkyl group (e.g., methyl, ethyl, isopropyl),
(6) a C₂₋₆ alkenyl group (e.g., allyl),
(7) a C₃₋₁₀ cycloalkyl group (e.g., cyclopropyl),
(8) an optionally substituted C₁₋₆ alkoxy group (e.g., methoxy, ethoxy, isopropoxy),
(9) a C₃₋₁₀ cycloalkyloxy group (e.g., cyclopropyloxy),
(10) an optionally substituted mono- or di-C₁₋₆ alkylamino group (e.g., methylamino, ethylamino, dimethylamino, ethyl(methyl)amino),
(11) a (C₁₋₆ alkyl) (3- to 14-membered non-aromatic heterocyclyl)amino group (e.g., methyl(piperidinyl)amino),
(12) a carbamoyl group,
(13) an optionally substituted nonaromatic heterocyclic group (e.g., piperazinyl, morpholinyl, 1,4-diazepanyl), or
(14) a 7- to 10-membered crosslinked heterocyclic group (e.g., 3,8-diazabicyclo[3.2.1]octanyl);
R⁴ is
(1) a hydrogen atom,
(2) a halogen atom (e.g., chlorine atom),
(3) a C₁₋₆ alkyl group (e.g., methyl),
(4) an optionally substituted C₁₋₆ alkoxy group (e.g., methoxy, ethoxy),
(5) a C₁₋₆ alkyl-carbonylamino group (e.g., methylcarbonylamino),
(6) a carbamoyl group,
(7) an optionally substituted nonaromatic heterocyclic group (e.g., pyrrolidinyl), or
(8) an optionally substituted by aromatic heterocyclic group (e.g., pyrazolyl); or
R⁴ and R³ are joined to form a non-aromatic heterocycle (e.g., dihydropyrrole ring, dihydrofuran ring, dihydrothiophene ring, tetrahydropyridine ring, 3,4-dihydro-2H-1,4-oxazine ring, 2,3-dihydro-1,4-dioxin ring, 2,3-dihydro-1,4-oxathiin ring, 6,7-dihydro-5H-1,4-dioxepine ring) or an aromatic heterocycle (e.g., furan ring, thiophene ring, imidazole ring), each of which is optionally substituted by 1 to 3 substituents selected from a C₁₋₆ alkyl group (e.g., methyl, ethyl) optionally substituted by a hydroxy group or a C₆₋₁₄ aryl group (e.g., phenyl), an oxo group, a C₁₋₆ alkyl-carbonyl group (e.g., methylcarbonyl) and a C₁₋₆ alkylsulfonyl group (e.g., methylsulfonyl);
ring A is a benzene ring optionally further substituted by 1 - 3 (preferably 1 or 2) substituents selected from a halogen atom (e.g., fluorine atom, chlorine atom), a C₁₋₆ alkyl group (e.g., methyl) and a C₃₋₁₀ cycloalkyl group (e.g., cyclopropyl), or a pyridine ring, a pyrazine ring or a pyridazine ring, each of which is optionally further substituted; and
ring B is a furan ring, an imidazole ring, a pyrazole ring, a pyridine ring, a pyrimidine ring, a pyridazine ring or a pyrazine ring, each of which is optionally further substituted by 1 or 2 (preferably 1) substituents selected from a halogen atom (e.g., chlorine atom), a hydroxy group, a C₁₋₆ alkyl group (e.g., methyl) and an amino group.

### [Compound I-3]

Compound (I) wherein
Z is CR¹ (R¹ is a hydrogen atom);
R² is
(1) a C₁₋₆ alkyl group (e.g., methyl, ethyl, n-propyl),
(2) a C₁₋₆ alkoxy group (e.g., methoxy, ethoxy, n-propoxy, isopropoxy, n-butoxy, sec-butoxy, n-pentoxy) optionally substituted by 1 - 5 substituents selected from deuterium, a halogen atom (e.g., fluorine atom), a hydroxy group, a C₁₋₆ alkoxy group (e.g., methoxy), a C₃₋₁₀ cycloalkyl group (e.g., cyclopropyl), a C₁₋₆ alkyl-carbonyloxy group (e.g., acetyloxy) and a tri-C₁₋₆ alkylsilyl-oxy group (e.g., tert-butyl(dimethyl)silyloxy),
(3) a C₃₋₁₀ cycloalkyl group (e.g., cyclopropyl),
(4) a C₃₋₁₀ cycloalkyloxy group (e.g., cyclohexyloxy) optionally substituted by a di-C₁₋₆ alkylamino group (e.g., dimethylamino),
(5) a C₆₋₁₄ aryl group (e.g., phenyl),
(6) a C₆₋₁₄ aryloxy group (e.g., phenoxy),
(7) a nonaromatic heterocyclic group (e.g., tetrahydropyranyl),
(8) a non-aromatic heterocyclyl-oxy group (e.g., piperidinyloxy, tetrahydropyranyloxy) optionally substituted by a C₁₋₆ alkyl group (e.g., methyl), or
(9) a mono- or di-C₁₋₆ alkylamino group (e.g., ethyl (methyl) amino) optionally substituted by a C₆₋₁₄ aryl group (e.g., phenyl);
R³ is
(1) a hydrogen atom,
(2) a halogen atom (e.g., chlorine atom),
(3) a cyano group,
(4) a hydroxy group,
(5) a C₁₋₆ alkyl group (e.g., methyl, ethyl, isopropyl) optionally substituted by 1 to 3 substituents selected from a halogen atom (e.g., fluorine atom), a C₃₋₁₀ cycloalkyl group (e.g., cyclopropyl) and an amino group,
(6) a C₂₋₆ alkenyl group (e.g., allyl),
(7) a C₃₋₁₀ cycloalkyl group (e.g., cyclopropyl),
(8) a C₁₋₆ alkoxy group (e.g., methoxy, ethoxy, isopropoxy) optionally substituted by 1 to 3 substituents selected from deuterium, a halogen atom (e.g., fluorine atom), a C₁₋₆ alkoxy group (e.g., methoxy) and an amino group,
(9) a C₃₋₁₀ cycloalkyloxy group (e.g., cyclopropyloxy),
(10) a mono- or di-C₁₋₆ alkylamino group (e.g., methylamino, ethylamino, dimethylamino, ethyl(methyl)amino) optionally substituted by a mono- or di-C₁₋₆ alkylamino group (e.g., dimethylamino),
(11) a (C₁₋₆ alkyl) (3- to 14-membered non-aromatic heterocyclyl)amino group (e.g., methyl(piperidinyl)amino),
(12) a carbamoyl group,
(13) a nonaromatic heterocyclic group (e.g., pyrrolidinyl, piperidinyl, piperazinyl, tetrahydropyridinyl, morpholinyl, 1,4-diazepanyl) optionally substituted by 1 to 3 substituents selected from a hydroxy group, an oxo group, a C₁₋₆ alkyl group (e.g., methyl, ethyl, isobutyl) optionally substituted by a hydroxy group, a C₆₋₁₄ aryl group (e.g., phenyl), mono- or di-C₁₋₆ alkylamino group (e.g., dimethylamino) and a C₁₋₆ alkoxy-carbonyl group (e.g., tert-butoxycarbonyl), or
(14) a 7- to 10-membered crosslinked heterocyclic group (e.g., 3,8-diazabicyclo[3.2.1]octanyl);
R⁴ is
(1) a hydrogen atom,
(2) a halogen atom (e.g., chlorine atom),
(3) a C₁₋₆ alkyl group (e.g., methyl),
(4) a C₁₋₆ alkoxy group (e.g., methoxy, ethoxy) optionally substituted by 1 to 3 substituents selected from a halogen atom (e.g., fluorine atom) and a C₁₋₆ alkoxy group (e.g., methoxy),
(5) a C₁₋₆ alkyl-carbonylamino group (e.g., methylcarbonylamino),
(6) a carbamoyl group,
(7) a nonaromatic heterocyclic group (e.g., pyrrolidinyl) optionally substituted by an oxo group, or
(8) an aromatic heterocyclic group (e.g., pyrazolyl) optionally substituted by a C₁₋₆ alkyl group (e.g., methyl); or
R⁴ and R³ are joined to form
(1) a non-aromatic heterocycle (e.g., dihydropyrrole ring, dihydrofuran ring, dihydrothiophene ring, tetrahydropyridine ring, 3,4-dihydro-2H-1,4-oxazine ring, 2,3-dihydro-1,4-dioxin ring, 2,3-dihydro-1,4-oxathiin ring, 6,7-dihydro-5H-1,4-dioxepine ring) optionally substituted by 1 to 3 substituents selected from a C₁₋₆ alkyl group (e.g., methyl, ethyl) optionally substituted by a C₆₋₁₄ aryl group (e.g., phenyl), an oxo group, a C₁₋₆ alkyl-carbonyl group (e.g., methylcarbonyl) and a C₁₋₆ alkylsulfonyl group (e.g., methylsulfonyl), or
(2) an aromatic heterocycle (e.g., furan ring, thiophene ring, imidazole ring) optionally substituted by 1 to 3 substituents selected from a C₁₋₆ alkyl group (e.g., methyl, ethyl) optionally substituted by a hydroxy group;
ring A is a benzene ring optionally further substituted by 1 - 3 (preferably 1 or 2) substituents selected from a halogen atom (e.g., fluorine atom, chlorine atom), a C₁₋₆ alkyl group (e.g., methyl) and a C₃₋₁₀ cycloalkyl group (e.g., cyclopropyl), or a pyridine ring, a pyrazine ring or a pyridazine ring; and
ring B is a pyridine ring optionally further substituted by 1 or 2 (preferably 1) substituents selected from a halogen atom (e.g., chlorine atom), a hydroxy group, a C₁₋₆ alkyl group (e.g., methyl) and an amino group, or a furan ring, an imidazole ring, a pyrazole ring, a pyrimidine ring, a pyridazine ring or a pyrazine ring.

### [Compound I-4]

Compound (I) wherein
Z is N or CR¹ (R¹ is a hydrogen atom or a halogen atom (e.g., fluorine atom, bromine atom));
R² is an optionally substituted C₁₋₆ alkyl group (e.g., methyl, ethyl, n-propyl), an optionally substituted C₁₋₆ alkoxy group (e.g., methoxy, ethoxy, n-propoxy, isopropoxy, n-butoxy), an optionally substituted C₃₋₁₀ cycloalkyl group (e.g., cyclopropyl), an optionally substituted C₃₋₁₀ cycloalkyloxy group (e.g., cyclobutyloxy, cyclohexyloxy), an optionally substituted C₆₋₁₄ aryl group (e.g., phenyl), an optionally substituted C₆₋₁₄ aryloxy group (e.g., phenoxy), an optionally substituted nonaromatic heterocyclic group (e.g., tetrahydropyranyl), an optionally substituted non-aromatic heterocyclyl-oxy group (e.g., piperidinyloxy, tetrahydropyranyloxy) or an optionally substituted mono- or di-C₁₋₆ alkylamino group (e.g., ethyl(methyl)amino);
R³ is a hydrogen atom, a halogen atom (e.g., chlorine atom), a cyano group, a hydroxy group, an optionally substituted C₁₋₆ alkyl group (e.g., methyl, ethyl, isopropyl), an optionally substituted C₂₋₆ alkenyl group (e.g., allyl), an optionally substituted C₃₋₁₀ cycloalkyl group (e.g., cyclopropyl), an optionally substituted C₁₋₆ alkoxy group (e.g., methoxy, ethoxy, isopropoxy), an optionally substituted C₃₋₁₀ cycloalkyloxy group (e.g., cyclopropyloxy), an optionally substituted amino group (e.g., amino group, optionally substituted mono- or di-C₁₋₆ alkylamino group (e.g., methylamino, ethylamino, dimethylamino, ethyl(methyl)amino), an optionally substituted (C₁₋₆ alkyl)(3- to 14-membered non-aromatic heterocyclyl)amino group (e.g., methyl(piperidinyl)amino), optionally substituted mono- or di-C₇₋₁₆ aralkylamino group (e.g., benzylamino)), an optionally substituted carbamoyl group, an optionally substituted nonaromatic heterocyclic group (e.g., pyrrolidinyl, piperidinyl, piperazinyl, tetrahydropyridinyl, morpholinyl, 1,4-diazepanyl), or an optionally substituted 7-to 10-membered crosslinked heterocyclic group (e.g., 3,8-diazabicyclo[3.2.1]octanyl);
R⁴ is a hydrogen atom, a halogen atom (e.g., fluorine atom, chlorine atom), an optionally substituted C₁₋₆ alkyl group (e.g., methyl), an optionally substituted C₃₋₁₀ cycloalkyl group (e.g., cyclopropyl), an optionally substituted C₆₋₁₄ aryl group (e.g., phenyl), an optionally substituted C₁₋₆ alkoxy group (e.g., methoxy, ethoxy), an optionally substituted C₁₋₆ alkyl-carbonylamino group (e.g., methylcarbonylamino), an optionally substituted carbamoyl group, an optionally substituted nonaromatic heterocyclic group (e.g., pyrrolidinyl, dihydropyranyl), or an optionally substituted aromatic heterocyclic group (e.g., furanyl, thienyl, pyrazolyl, thiazolyl, pyridyl, indazolyl, pyrazolopyridyl, isoquinolinyl); or
R⁴ and R³ are joined to form a non-aromatic heterocycle (e.g., dihydropyrrole ring, dihydrofuran ring, dihydrothiophene ring, tetrahydropyridine ring, 3,4-dihydro-2H-1,4-oxazine ring, 2,3-dihydro-1,4-dioxin ring, 2,3-dihydro-1,4-oxathiin ring, 6,7-dihydro-5H-1,4-dioxepine ring) or an aromatic heterocycle (e.g., furan ring, thiophene ring, imidazole ring), each of which is optionally substituted by 1 to 3 substituents selected from an optionally substituted C₁₋₆ alkyl group (e.g., methyl, ethyl), an oxo group, an optionally substituted C₁₋₆ alkyl-carbonyl group (e.g., methylcarbonyl) and an optionally substituted C₁₋₆ alkylsulfonyl group (e.g., methylsulfonyl);
ring A is an optionally further substituted benzene ring, or a pyridine ring, a pyrazine ring or a pyridazine ring, each of which is optionally further substituted; and
ring B is a furan ring, an imidazole ring, a pyrazole ring, a pyridine ring, a pyrimidine ring, a pyridazine ring or a pyrazine ring, each of which is optionally further substituted.

### [Compound I-5]

Compound (I) wherein
Z is N or CR¹ (R¹ is a hydrogen atom, a fluorine atom or a bromine atom);
R² is
(1) a C₁₋₆ alkyl group (e.g., methyl, ethyl, n-propyl),
(2) a C₁₋₆ alkoxy group (e.g., methoxy, ethoxy, n-propoxy, isopropoxy, n-butoxy, sec-butoxy, n-pentoxy) optionally substituted by 1 - 5 substituents selected from a halogen atom (e.g., fluorine atom), a hydroxy group, a C₁₋₆ alkoxy group (e.g., methoxy), a C₃₋₁₀ cycloalkyl group (e.g., cyclopropyl), a C₁₋₆ alkyl-carbonyloxy group (e.g., acetyloxy) and a tri-C₁₋₆ alkylsilyl-oxy group (e.g., tert-butyl(dimethyl)silyloxy),
(3) a C₃₋₁₀ cycloalkyl group (e.g., cyclopropyl),
(4) a C₃₋₁₀ cycloalkyloxy group (e.g., cyclobutyloxy, cyclohexyloxy) optionally substituted by 1 - 5 substituents selected from a di-C₁₋₆ alkylamino group (e.g., dimethylamino) and a nonaromatic heterocyclic group (e.g., morpholinyl),
(5) a C₆₋₁₄ aryl group (e.g., phenyl),
(6) a C₆₋₁₄ aryloxy group (e.g., phenoxy),
(7) a nonaromatic heterocyclic group (e.g., tetrahydropyranyl),
(8) a non-aromatic heterocyclyl-oxy group (e.g., piperidinyloxy, tetrahydropyranyloxy) optionally substituted by a C₁₋₆ alkyl group (e.g., methyl), or
(9) a mono- or di-C₁₋₆ alkylamino group (e.g., ethyl (methyl) amino) optionally substituted by a C₆₋₁₄ aryl group (e.g., phenyl);
R³ is
(1) a hydrogen atom,
(2) a halogen atom (e.g., chlorine atom),
(3) a cyano group,
(4) a hydroxy group,
(5) a C₁₋₆ alkyl group (e.g., methyl, ethyl, isopropyl) optionally substituted by 1 to 3 substituents selected from a halogen atom (e.g., fluorine atom), a C₃₋₁₀ cycloalkyl group (e.g., cyclopropyl) and an amino group,
(6) a C₂₋₆ alkenyl group (e.g., allyl),
(7) a C₃₋₁₀ cycloalkyl group (e.g., cyclopropyl),
(8) a C₁₋₆ alkoxy group (e.g., methoxy, ethoxy, isopropoxy) optionally substituted by 1 to 3 substituents selected from a halogen atom (e.g., fluorine atom), a C₁₋₆ alkoxy group (e.g., methoxy) and an amino group,
(9) a C₃₋₁₀ cycloalkyloxy group (e.g., cyclopropyloxy),
(10) an amino group,
(11) a mono- or di-C₁₋₆ alkylamino group (e.g., methylamino, ethylamino, dimethylamino, ethyl(methyl)amino) optionally substituted by a mono- or di-C₁₋₆ alkylamino group (e.g., dimethylamino),
(12) a (C₁₋₆ alkyl)(3- to 14-membered non-aromatic heterocyclyl)amino group (e.g., methyl(piperidinyl)amino),
(13) a mono- or di-C₇₋₁₆ aralkylamino group (e.g., benzylamino),
(14) a carbamoyl group,
(15) a nonaromatic heterocyclic group (e.g., pyrrolidinyl, piperidinyl, piperazinyl, tetrahydropyridinyl, morpholinyl, 1,4-diazepanyl) optionally substituted by 1 to 3 substituents selected from a hydroxy group, an oxo group, a C₁₋₆ alkyl group (e.g., methyl, ethyl, isobutyl) optionally substituted by a hydroxy group, a C₆₋₁₄ aryl group (e.g., phenyl), a mono- or di-C₁₋₆ alkylamino group (e.g., dimethylamino) and a C₁₋₆ alkoxy-carbonyl group (e.g., tert-butoxycarbonyl), or
(16) a 7- to 10-membered crosslinked heterocyclic group (e.g., 3,8-diazabicyclo[3.2.1]octanyl);
R⁴ is
(1) a hydrogen atom,
(2) a halogen atom (e.g., fluorine atom, chlorine atom),
(3) a C₁₋₆ alkyl group (e.g., methyl),
(4) a C₃₋₁₀ cycloalkyl group (e.g., cyclopropyl),
(5) a C₆₋₁₄ aryl group (e.g., phenyl) optionally substituted by 1 to 3 halogen atoms (e.g., fluorine atom),
(6) a C₁₋₆ alkoxy group (e.g., methoxy, ethoxy) optionally substituted by 1 to 3 substituents selected from a halogen atom (e.g., fluorine atom) and a C₁₋₆ alkoxy group (e.g., methoxy),
(7) a C₁₋₆ alkyl-carbonylamino group (e.g., methylcarbonylamino),
(8) a carbamoyl group,
(9) a nonaromatic heterocyclic group (e.g., pyrrolidinyl, dihydropyranyl) optionally substituted by an oxo group, or
(10) an aromatic heterocyclic group (e.g., furanyl, thienyl, pyrazolyl, thiazolyl, pyridyl, indazolyl, pyrazolopyridyl, isoquinolinyl) optionally substituted by 1 to 3 substituents selected from (i) a C₁₋₆ alkyl group (e.g., methyl, ethyl) optionally substituted by 1 to 3 substituents selected from a C₃₋₁₀ cycloalkyl group (e.g., cyclopropyl) and an aromatic heterocyclic group (e.g., pyridyl) and (ii) a nonaromatic heterocyclic group (e.g., tetrahydropyranyl); or
R⁴ and R³ are joined to form
(1) a non-aromatic heterocycle (e.g., dihydropyrrole ring, dihydrofuran ring, dihydrothiophene ring, tetrahydropyridine ring, 3,4-dihydro-2H-1,4-oxazine ring, 2,3-dihydro-1,4-dioxin ring, 2,3-dihydro-1,4-oxathiin ring, 6,7-dihydro-5H-1,4-dioxepine ring) optionally substituted by 1 to 3 substituents selected from a C₁₋₆ alkyl group (e.g., methyl, ethyl) optionally substituted by a C₆₋₁₄ aryl group (e.g., phenyl), an oxo group, C₁₋₆ alkyl-carbonyl group (e.g., methylcarbonyl) and a C₁₋₆ alkylsulfonyl group (e.g., methylsulfonyl), or
(2) an aromatic heterocycle (e.g., furan ring, thiophene ring, imidazole ring) optionally substituted by 1 to 3 substituents selected from a C₁₋₆ alkyl group (e.g., methyl, ethyl) optionally substituted by a hydroxy group;
ring A is a benzene ring optionally further substituted by 1 - 3 (preferably 1 or 2) substituents selected from a halogen atom (e.g., fluorine atom, chlorine atom), a C₁₋₆ alkyl group (e.g., methyl) and a C₃₋₁₀ cycloalkyl group (e.g., cyclopropyl), or a pyridine ring, a pyrazine ring or a pyridazine ring; and
ring B is a pyridine ring optionally further substituted by 1 or 2 (preferably 1) substituents selected from a halogen atom (e.g., chlorine atom), a hydroxy group, a C₁₋₆ alkyl group (e.g., methyl), a C₁₋₆ alkoxy group (e.g., methoxy) and an amino group, or a furan ring, an imidazole ring, a pyrazole ring, a pyrimidine ring, a pyridazine ring or a pyrazine ring.

### [Compound I-6]

Compound (I) wherein
Z is CR¹ (R¹ is a hydrogen atom);
R² is
(1) a C₁₋₆ alkoxy group (e.g., ethoxy), or
(2) a C₃₋₁₀ cycloalkyloxy group (e.g., cyclobutyloxy);
R³ is
(1) a C₁₋₆ alkyl group (e.g., methyl, ethyl),
(2) a C₁₋₆ alkoxy group (e.g., methoxy),
(3) a carbamoyl group, or
(4) a nonaromatic heterocyclic group (e.g., piperazinyl);
R⁴ is
(1) a hydrogen atom,
(2) a halogen atom (e.g., fluorine atom),
(3) a C₁₋₆ alkoxy group (e.g., ethoxy), or
(4) an aromatic heterocyclic group (e.g., pyrazolyl) optionally substituted by 1 to 3 C₁₋₆ alkyl groups (e.g., methyl); or
R⁴ and R³ are joined to form a non-aromatic heterocycle (e.g., 2,3-dihydro-1,4-dioxin ring);
ring A is a benzene ring optionally further substituted by 1 - 3 (preferably 1 or 2) halogen atoms (e.g., fluorine atom, chlorine atom), or a pyridine ring or a pyridazine ring; and
ring B is a pyridine ring, furan ring, an imidazole ring, a pyrazole ring, a pyrimidine ring, a pyridazine ring or a pyrazine ring.

Specific examples of compound (I) include the compounds of Examples 1 - 116 and 119 - 230, of which
7-ethoxy-1-(4-methoxyquinolin-6-yl)-1,3-dihydro-2H-imidazo[4,5-b]pyridin-2-one or a salt thereof (Example 41);
7-ethoxy-1-(8-fluoro-2,3-dihydro[1,4]dioxino[2,3-c]quinolin-9-yl)-1,3-dihydro-2H-imidazo[4,5-b]pyridin-2-one or a salt thereof (Example 94); and
7-ethoxy-1-(4-methoxy-3-(1-methyl-1H-pyrazol-4-yl)quinolin-6-yl)-1,3-dihydro-2H-imidazo[4,5-b]pyridin-2-one or a salt thereof (Example 177)
are preferable.

When compound (I) is a salt, examples of such salt include metal salts, ammonium salts, salts with organic base, salts with inorganic acid, salts with organic acid, salts with basic or acidic amino acids, and the like. Preferable examples of the metal salt include alkaline metal salts such as sodium salt, potassium salt, and the like; alkaline earth metal salts such as calcium salt, magnesium salt, barium salt, and the like; and aluminum salt. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, and the like. Preferable examples of the salt with inorganic acid include salts with hydrochloric acid, hydrobromic acid, nitric acid, sulfuric acid, phosphoric acid, and the like. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, and the like. Preferable examples of the salt with basic amino acid include salts with arginine, lysine, ornithine, and the like. Preferable examples of the salt with acidic amino acid include salt with aspartic acid, glutamic acid, and the like.

Of these, pharmaceutically acceptable salt is preferable. Examples thereof include inorganic salts such as alkali metal salt (e.g., sodium salt, potassium salt etc.), alkaline earth metal salt (e.g., calcium salt, magnesium salt etc.) and the like, ammonium salt, and the like when an acidic functional group is contained in the compound, and salts with inorganic acids such as hydrochloric acid, hydrobromic acid, nitric acid, sulfuric acid, phosphoric acid and the like, and salts with organic acids such as acetic acid, phthalic acid, fumaric acid, oxalic acid, tartaric acid, maleic acid, citric acid, succinic acid, methanesulfonic acid, benzenesulfonic acid, p-toluenesulfonic acid and the like when a basic functional group is contained in the compound.

The production method of the compound of the present invention is explained in the following.

The starting materials and reagents used in each step in the following production method, and the obtained compounds each may form a salt. Examples of the salt include those similar to the aforementioned salts of the compound of the present invention and the like.

When the compound obtained in each step is a free compound, it can be converted to a desired salt by a method known per se. Conversely, when the compound obtained in each step is a salt, it can be converted to a free form or a desired other kind of salt by a method known per se.

The compound obtained in each step can also be used for the next reaction as a reaction mixture thereof or after obtaining a crude product thereof. Alternatively, the compound obtained in each step can be isolated and/or purified from the reaction mixture by a separation means such as concentration, crystallization, recrystallization, distillation, solvent extraction, fractionation, chromatography and the like according to a conventional method.

When the starting materials and reagent compounds of each step are commercially available, the commercially available products can be used as they are.

In the reaction of each step, while the reaction time varies depending on the reagents and solvents to be used, unless otherwise specified, it is generally 1 min - 48 hr, preferably 10 min - 8 hr.

In the reaction of each step, while the reaction temperature varies depending on the reagents and solvents to be used, unless otherwise specified, it is generally -78°C to 300°C, preferably -78°C to 150°C.

In the reaction of each step, while the pressure varies depending on the reagents and solvents to be used, unless otherwise specified, it is generally 1 atm - 20 atm, preferably 1 atm - 3 atm.

In the reaction of each step, for example, microwave synthesizers such as Initiator manufactured by Biotage and the like are sometimes used. While the reaction temperature varies depending on the reagents and solvents to be used, unless otherwise specified, it is generally room temperature - 300°C, preferably 50°C - 250°C. While the reaction time varies depending on the reagents and solvents to be used, unless otherwise specified, it is generally 1 min - 48 hr, preferably 1 min - 8 hr.

In the reaction of each step, unless otherwise specified, a reagent is used in 0.5 equivalent - 20 equivalents, preferably 0.8 equivalent - 5 equivalents, relative to the substrate. When a reagent is used as a catalyst, the reagent is used in 0.001 equivalent - 1 equivalent, preferably 0.01 equivalent - 0.2 equivalent, relative to the substrate. When the reagent is also a reaction solvent, the reagent is used in a solvent amount.

In the reaction of each step, unless otherwise specified, it is performed without solvent or by dissolving or suspending in a suitable solvent. Specific examples of the solvent include those described in Examples and the following.
alcohols: methanol, ethanol, tert-butyl alcohol, 2-methoxyethanol and the like;
ethers: diethyl ether, diphenyl ether, tetrahydrofuran, 1,2-dimethoxyethane and the like;
aromatic hydrocarbons: chlorobenzene, toluene, xylene and the like;
saturated hydrocarbons: cyclohexane, hexane and the like;
amides: N,N-dimethylformamide, N-methylpyrrolidone and the like;
halogenated hydrocarbons: dichloromethane, carbon tetrachloride and the like;
nitriles: acetonitrile and the like;
sulfoxides: dimethyl sulfoxide and the like;
aromatic organic bases: pyridine and the like;
acid anhydrides: acetic anhydride and the like;
organic acids: formic acid, acetic acid, trifluoroacetic acid and the like;
inorganic acids: hydrochloric acid, sulfuric acid and the like; esters: ethyl acetate and the like;
ketones: acetone, methyl ethyl ketone and the like; and water.

Two or more kinds of the above-mentioned solvents may be used by mixing at an appropriate ratio.

When a base is used in the reaction of each step, for example, bases shown below or those described in Examples are used.
inorganic bases: sodium hydroxide, magnesium hydroxide and the like;
basic salts: sodium carbonate, calcium carbonate, sodium hydrogen carbonate and the like;
organic bases: triethylamine, diethylamine, pyridine, 4-dimethylaminopyridine, N,N-dimethylaniline, 1,4-diazabicyclo[2.2.2]octane, 1,8-diazabicyclo[5.4.0]-7-undecene, imidazole, piperidine and the like;
metal alkoxides: sodium ethoxide, potassium tert-butoxide and the like;
alkali metal hydrides: sodium hydride and the like;
metal amides: sodium amide, lithium diisopropyl amide, lithium hexamethyl disilazide and the like; and
organic lithiums: n-butyllithium and the like.

When an acid or acidic catalyst is used in the reaction of each step, for example, acids and acidic catalysts shown below or those described in Examples are used.
inorganic acids: hydrochloric acid, sulfuric acid, nitric acid, hydrobromic acid, phosphoric acid and the like;
organic acids: acetic acid, trifluoroacetic acid, citric acid, p-toluenesulfonic acid, 10-camphorsulfonic acid and the like; and
Lewis acids: boron trifluoride diethyl ether complex, zinc iodide, anhydrous aluminum chloride, anhydrous zinc chloride, anhydrous iron chloride and the like.

Unless otherwise specified, the reaction of each step is performed according to a method known per se, for example, the methods described in Jikken Kagaku Kouza 5th edition, vol. 13 - vol. 19 (The Chemical Society of Japan ed.); Shinjikken Kagaku Kouza (Courses in Experimental Chemistry), vol. 14 - vol. 15 (The Chemical Society of Japan ed.); Fine Organic Chemistry rev. 2nd edition (L. F. Tietze, Th. Eicher, NANKODO); rev. Organic Name Reactions, Their Mechanism and Essence (Hideo Togo, Kodansha); ORGANIC SYNTHESES Collective Volume I - VII (John Wiley & Sons Inc); Modern Organic Synthesis in the Laboratory, A Collection of Standard Experimental Procedures (Jie Jack Li, OXFORD UNIVERSITY); Comprehensive Heterocyclic Chemistry III, Vol. 1 - Vol. 14 (Elsevier Japan KK); Strategic Applications of Named Reactions in Organic Synthesis (translation supervisor Kiyoshi Tomioka, KAGAKUDOJIN); Comprehensive Organic Transformations (VCH Publishers Inc.), 1989 and the like, or the methods described in the Examples.

In each step, protection or deprotection of a functional group is performed by the method known per se, for example, the methods described in "Protective Groups in Organic Synthesis, 4th Ed." (Theodora W. Greene, Peter G. M. Wuts) Wiley-Interscience, 2007; "Protecting Groups 3rd Ed." (P. J. Kocienski) Thieme, 2004 and the like, or the methods described in the Examples.

Examples of the protecting group of the hydroxyl group of alcohol and the like and a phenolic hydroxyl group include ether protecting groups such as methoxymethyl ether, benzyl ether, t-butyldimethylsilyl ether, tetrahydropyranyl ether and the like; carboxylate protecting groups such as acetate and the like; sulfonate ester protecting groups such as methanesulfonate ester and the like; carbonate ester protecting groups such as t-butylcarbonate and the like, and the like.

Examples of the protecting group of the carbonyl group of aldehyde include acetal protecting groups such as dimethyl acetal and the like; cyclic acetal protecting groups such as cyclic 1,3-dioxane and the like, and the like.

Examples of the protecting group of the carbonyl group of ketone include ketal protecting groups such as dimethyl ketal and the like; cyclic ketal protecting groups such as cyclic 1,3-dioxane and the like; oxime protecting groups such as O-methyloxime and the like; hydrazone protecting groups such as N,N-dimethylhydrazone and the like, and the like.

Examples of the carboxyl protecting group include ester protecting groups such as methyl ester and the like; amide protecting groups such as N,N-dimethylamide and the like, and the like.

Examples of the thiol protecting group include ether protecting groups such as benzyl thioether and the like; ester protecting groups such as thioacetate ester, thiocarbonate, thiocarbamate and the like, and the like.

Examples of the protecting group of an amino group and an aromatic hetero ring such as imidazole, pyrrole, indole and the like include carbamate protecting groups such as benzyl carbamate and the like; amide protecting groups such as acetamide and the like; alkylamine protecting groups such as N-triphenylmethylamine and the like, sulfonamide protecting groups such as methanesulfonamide and the like, and the like.

The protecting group can be removed by a method known per se, for example, a method using acid, base, ultraviolet light, hydrazine, phenylhydrazine, sodium N-methyldithiocarbamate, tetrabutylammonium fluoride, palladium acetate, trialkylsilyl halide (e.g., trimethylsilyl iodide, trimethylsilyl bromide), a reduction method and the like.

When a reduction reaction is performed in each step, examples of the reducing agent to be used include metal hydrides such as lithium aluminum hydride, sodium triacetoxyborohydride, sodium cyanoborohydride, diisobutylaluminum hydride (DIBAL-H), sodium borohydride, tetramethylammonium triacetoxyborohydride and the like; boranes such as borane tetrahydrofuran complex and the like; Raney nickel; Raney cobalt; hydrogen; formic acid and the like. When a carbon-carbon double bond or triple bond is reduced, a method using a catalyst such as palladium-carbon, Lindlar catalyst and the like.

When an oxidation reaction is performed in each step, examples of an oxidant to be used include peracids such as m-chloroperbenzoic acid (mCPBA), hydrogen peroxide, t-butyl hydroperoxide and the like; perchlorates such as tetrabutylammonium perchlorate and the like; chlorates such as sodium chlorate and the like; chlorites such as sodium chlorite and the like; periodic acids such as sodium periodate and the like; high valent iodine reagents such as iodosylbenzene and the like; reagents containing manganese such as manganese dioxide, potassium permanganate and the like; leads such as lead tetraacetate and the like; reagents containing chrome such as pyridinium chlorochromate (PCC), pyridinium dichromate (PDC), Jones reagent and the like; halogen compounds such as N-bromosuccinimide (NBS) and the like; oxygen; ozone; sulfur trioxide pyridine complex; osmium tetraoxide; selenium dioxide; 2,3-dichloro-5,6-dicyano-1,4-benzoquinone (DDQ) and the like.

When a radical cyclization reaction is performed in each step, examples of the radical initiator to be used include azo compounds such as azobisisobutyronitrile (AIBN) and the like; water-soluble radical initiators such as 4,4'-azobis-4-cyanopentanoic acid (ACPA) and the like; triethylboron in the presence of air or oxygen; benzoyl peroxide and the like. In addition, examples of the radical reaction agent to be used include tributylstannane, tristrimethylsilylsilane, 1,1,2,2-tetraphenyldisilane, diphenylsilane, samarium iodide and the like.

When the Wittig reaction is performed in each step, examples of the Wittig reagent to be used include alkylidenephosphoranes and the like. Alkylidenephosphoranes can be prepared by a method known per se, for example, by reacting a phosphonium salt with a strong base.

When the Horner-Emmons reaction is performed in each step, examples of the reagent to be used include phosphonoacetic acid esters such as methyl dimethylphosphonoacetate, ethyl diethylphosphonoacetate and the like; and bases such as alkali metal hydrides, organic lithiums and the like.

When the Friedel-Crafts reaction is performed in each step, examples of the reagent to be used include Lewis acid and acid chloride or alkylating agents (e.g., alkyl halides, alcohol, olefins and the like). Alternatively, an organic acid and an inorganic acid can also be used instead of the Lewis acid, and acid anhydride such as acetic anhydride and the like can also be used instead of acid chloride.

When an aromatic nucleophilic substitution reaction is performed in each step, a nucleophilic agent (e.g., amines, imidazole and the like) and a base (e.g., basic salts, organic bases and the like) are used as the reagent.

When a nucleophilic addition reaction with carbanion, a nucleophilic 1,4-addition reaction with carbanion (Michael addition reaction) or a nucleophilic substitution reaction with carbanion is performed in each step, examples of the base to be used for developing carbanion include organic lithiums, metal alkoxides, inorganic bases, organic bases and the like.

When the Grignard reaction is performed in each step, examples of the Grignard reagent include aryl magnesium halides such as phenyl magnesium bromide and the like; and alkyl magnesium halides such as methyl magnesium bromide and the like. The Grignard reagent can be prepared by a method known per se, for example, by reacting alkyl halide or aryl halide with metal magnesium in ether or tetrahydrofuran as a solvent.

When the Knoevenagel condensation reaction is performed in each step, an active methylene compound held between two electron-withdrawing groups (e.g., malonic acid, diethyl malonate, malononitrile and the like) and a base (e.g., organic bases, metal alkoxides, inorganic bases) are used as the reagents.

When the Vilsmeier-Haack reaction is performed in each step, phosphoryl chloride and an amide derivative (e.g., N,N-dimethylformamide and the like) are used as the reagents.

When an azidation reaction of alcohols, alkylhalides or sulfonate esters is performed in each step, examples of the azidation agent to be used include diphenylphosphoryl azide (DPPA), trimethylsilylazide, sodium azide and the like. For example, when alcohols are azidated, a method using diphenylphosphoryl azide and 1,8-diazabicyclo[5,4,0]undec-7-ene (DBU), a method using trimethylsilylazide and the Lewis acid and the like can be employed.

When a reductive amination reaction is performed in each step, examples of the reducing agent to be used include sodium triacetoxyborohydride, sodium cyanoborohydride, hydrogen, formic acid and the like. When the substrate is an amine compound, examples of the carbonyl compound to be used besides para-formaldehyde include aldehydes such as acetaldehyde and the like, ketones such as cyclohexanone and the like. When the substrate is a carbonyl compound, examples of the amines to be used include primary amines such as ammonia, methylamine and the like; secondary amines such as dimethylamine and the like, and the like.

When the Mitsunobu reaction is performed in each step, azodicarboxylate esters (e.g., diethyl azodicarboxylate (DEAD), diisopropyl azodicarboxylate (DIAD) and the like) and triphenylphosphine are used as the reagents.

When an esterification reaction, amidation reaction or ureation reaction is performed in each step, examples of the reagent to be used include halogenated acyl forms such as acid chloride, acid bromide and the like; and activated carboxylic acids such as acid anhydride, active ester form, sulfuric acid ester form and the like. Examples of the carboxylic acid activator include carbodiimide condensing agents such as 1-ethyl-3-(3-dimethylaminopropyl)carbodiimide hydrochloride (WSCD) and the like; triazine condensing agents such as 4-(4,6-dimethoxy-1,3,5-triazin-2-yl)-4-methylmorpholinium chloride-n-hydrate (DMT-MM) and the like; carbonate ester condensing agents such as 1,1-carbonyldiimidazole (CDI) and the like; diphenylphosphoryl azide (DPPA); benzotriazol-1-yloxy-trisdimethylaminophosphonium salt (BOP reagent); 2-chloro-1-methyl-pyridinium iodide (Mukaiyama reagent); thionyl chloride; lower alkyl haloformates such as ethyl chloroformate and the like; O-(7-azabenzotriazol-1-yl)-N,N,N',N'-tetramethyluronium hexafluorophosphate (HATU); sulfuric acid; a combination thereof and the like. When a carbodiimide condensing agent is used, additives such as 1-hydroxybenzotriazole (HOBt), N-hydroxysuccinimide (HOSu), dimethylaminopyridine (DMAP) and the like can be further added to the reaction.

When a coupling reaction is performed in each step, examples of the metal catalyst to be used include palladium compounds such as palladium(II) acetate, tetrakis(triphenylphosphine)palladium(0), dichlorobis(triphenylphosphine)palladium(II), dichlorobis(triethylphosphine)palladium(II), tris(dibenzylideneacetone)dipalladium(O), 1,1'-bis(diphenylphosphino)ferrocene palladium(II) chloride, palladium(II) acetate and the like; nickel compounds such as tetrakis(triphenylphosphine)nickel(0) and the like; rhodium compounds such as tris(triphenylphosphine)rhodium(III) chloride and the like; a cobalt compound; copper compounds such as copper oxide, copper(I) iodide and the like; a platinum compound and the like. A base may be further added to the reaction and examples of such base include inorganic bases, basic salts and the like.

When a thiocarbonylation reaction is performed in each step, diphosphorus pentasulfide is representatively used as a thiocarbonylating agent. Besides diphosphorus pentasulfide, a reagent having a 1,3,2,4-dithiadiphosphetane-2,4-disulfide structure such as 2,4-bis(4-methoxyphenyl-1,3,2,4-dithiadiphosphetane-2,4-disulfide (Lawesson reagent) and the like may also be used.

When the Wohl-Ziegler reaction is performed in each step, examples of the halogenating agent to be used include N-iodosuccinimide, N-bromosuccinimide (NBS), N-chlorosuccinimide (NCS), bromine, sulfuryl chloride and the like. Furthermore, the reaction can be accelerated by adding heat, light, radical initiators such as benzoyl peroxide, azobisisobutyronitrile and the like to the reaction.

When a halogenating reaction of a hydroxy group is performed in each step, examples of the halogenating agent to be used include hydrohalic acid and acid halide of inorganic acid; specifically, hydrochloric acid, thionyl chloride, phosphorus oxychloride and the like for chlorination, and 48% hydrobromic acid and the like for bromination. In addition, a method of obtaining a halogenated alkyl form from alcohol by reacting with triphenylphosphine and carbon tetrachloride or carbon tetrabromide, and the like may be used. Alternatively, a method of synthesizing a halogenated alkyl form via a two-step reaction including conversion of alcohol to sulfonic acid ester, and reacting same with lithium bromide, lithium chloride or sodium iodide may also be used.

When the Arbuzov reaction is performed in each step, examples of the reagent to be used include alkyl halides such as ethyl bromoacetate and the like; and phosphites such as triethyl phosphite, tri(isopropyl)phosphite and the like.

When a sulfonate esterification reaction is performed in each step, examples of the sulfonating agent to be used include methanesulfonyl chloride, p-toluenesulfonyl chloride, methanesulfonic anhydride, p-toluenesulfonic anhydride and the like.

When hydrolysis reaction is performed in each step, an acid or a base is used as the reagent. In addition, when acid hydrolysis reaction of t-butyl ester is performed, formic acid, triethylsilane and the like are sometimes added to reductively trap the by-produced t-butyl cation.

When a dehydrating reaction is performed in each step, examples of the dehydrating agent to be used include sulfuric acid, phosphorus pentaoxide, phosphorus oxychloride, N,N'-dicyclohexylcarbodiimide, alumina, polyphosphoric acid and the like. 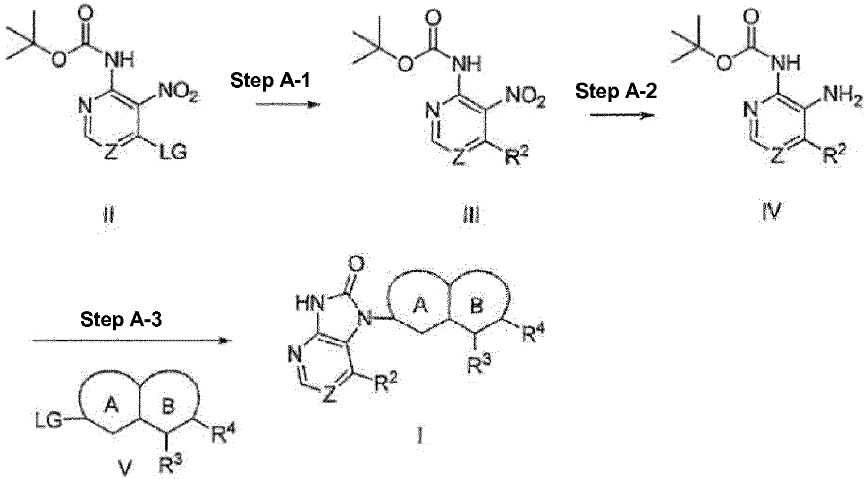 wherein LG is a leaving group, and other symbols are as defined above.

### (Step A-1)

In this step, compound (II) is converted to compound (III) by a nucleophilic substitution reaction or a coupling reaction.

As the leaving group for LG, a halogen atom (e.g., chlorine atom, bromine atom, iodine atom and the like), an optionally substituted sulfonyloxy group (e.g., C₁₋₆ alkylsulfonyloxy group (e.g., methanesulfonyloxy group, ethanesulfonyloxy group, trifluoromethanesulfonyloxy group and the like) optionally substituted by 1 to 3 halogen atoms; a C₆₋₁₄ arylsulfonyloxy group (e.g., benzenesulfonyloxy group, p-toluenesulfonyloxy group and the like) optionally substituted by 1 to 3 C₁₋₆ alkyl groups; a C₇₋₁₄ aralkylsulfonyloxy group (e.g., benzylsulfonyloxy group and the like) and the like), [(oxido)phenyl-λ4-sulfanylidene]dimethylammonium group and the like can be mentioned.

Compound (II) to be used as a starting material in this method may be a commercially available product, or can also be produced by a method known per se or a method analogous thereto.

The "nucleophilic substitution reaction" is generally performed in the presence of a base. This reaction is advantageously performed in a solvent inert to the reaction. While such solvent is not particularly limited as long as the reaction proceeds, for example, solvents such as alcohols, ethers, aromatic hydrocarbons, saturated hydrocarbons, amides, nitriles, sulfoxides, esters, water and the like or a mixed solvent thereof and the like are preferable. To promote the reaction, microwave may be irradiated.

The coupling reaction is performed under the conditions of the coupling reaction mentioned above. When necessary, a ligand (e.g., 4,5-bis(diphenylphosphino)-9,9-dimethylxanthene, 2,2'-bis(diphenylphosphino)-1,1'-binaphthyl, 2,2'-bis(di-p-tolylphosphino)-1,1'-binaphthyl, 2-dicyclohexylphosphino-2',6'-diisopropoxy-1,1'-biphenyl, 5-(di-t-butylphosphino)-1',3',5'-triphenyl-1,4'-bi-1H-pyrazole, 2-dicyclohexylphosphino-2',4',6'-triisopropyl-1,1'-biphenyl, triphenylphosphine, tri-tert-butylphosphine, 1,10-phenanthrolin etc.) may be added. To promote the reaction, moreover, microwave may be irradiated.

### (Step A-2)

In this step, compound (III) is converted to compound (IV) by reduction of a nitro group thereof.

The "reduction of nitro group" can be performed by, for example, a method by a catalytic reduction reaction using a transition metal catalyst such as palladium, platinum, rhodium or Raney-nickel and the like, a method using a metal hydride such as lithium aluminum hydride, sodium tetrahydroborate in the presence of divalent nickel chloride and the like, a method using a metal powder such as zinc, iron, tin and the like under acidic conditions and the like. Of these, a method by a catalytic reduction reaction is preferable. Compound (IV) may be used for the next reaction without isolation.

### (Step A-3)

In this step, compound (IV) and compound (V) are converted to compound (I) by subjecting them to a coupling reaction and performing an intramolecular cyclization reaction.

Compound (V) can be synthesized by a method known per se, or according to the method described in Example.

The reaction is performed under the conditions of the coupling reaction mentioned above, and the ligand indicated in (step A-1) is prepared. 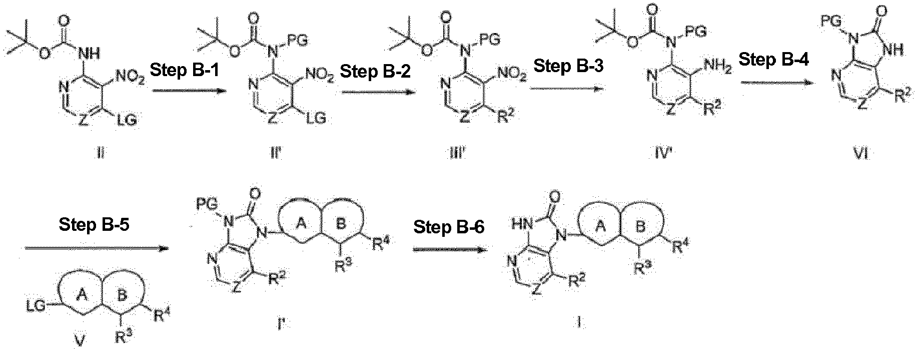 wherein PG is an amino-protecting group, and other symbols are as defined above.

### (Step B-1)

In this step, compound (II) is converted to compound (II') by protecting an amino group. The reaction is performed under the conditions of the protection of functional group mentioned above.

### (Step B-2)

In this step, compound (II') is converted to compound (III') by a nucleophilic substitution reaction or a coupling reaction, which can be performed according to Step A-1.

### (Step B-3)

In this step, compound (III') is converted to compound (IV') by reducing a nitro group thereof, which can be performed according to Step A-2.

### (Step B-4)

In this step, compound (IV') is converted to compound (VI) by an intramolecular cyclization reaction. The "intramolecular cyclization reaction" is performed in the presence of a base, without solvent, or in a solvent that does not adversely influence the reaction.

### (Step B-5)

In this step, compound (V) and compound (VI) are converted to compound (I') by subjecting them to a coupling reaction.

The reaction is performed under the conditions of the coupling reaction mentioned above, and the ligand indicated in (step A-1) is prepared.

### (Step B-6)

In this step, compound (I') is converted to compound (I) by deprotection. The reaction is performed under the conditions of the deprotection of functional group mentioned above.

The thus-obtained compound (I) can be isolated and purified by a known separation and purification means, for example, concentration, concentration under reduced pressure, solvent extraction, crystallization, recrystallization, phase transfer, chromatography and the like.

The compounds of the present invention obtained by the above-mentioned production methods can be isolated and purified by a known means such as concentration, concentration under reduced pressure, solvent extraction, crystallization, recrystallization, phase transfer, chromatography and the like. Each starting compound used for the above-mentioned production methods can be isolated and purified by a known means similar to those mentioned above. On the other hand, these starting compounds may be directly used as the starting materials for the next step as a reaction mixture without isolation.

Compound (I) produced by such method can be isolated and purified by a typical separation means such as recrystallization, distillation, chromatography, and the like.

When compound (I) has an optical isomer, a stereoisomer, a regioisomer or a rotamer, these are also encompassed in compound (I), and can be obtained as a single product according to a synthesis method and separation method known per se (e.g., concentration, solvent extraction, column chromatography, recrystallization etc.). For example, when compound (I) has an optical isomer, the optical isomer resolved from the compound is also encompassed in compound (I).

The optical isomer can be produced according to a method known per se. Specifically, the optical isomer is obtained using an optically active synthetic intermediate or by subjecting the racemic final product to an optical resolution according to a known method.

The method of optical resolution may be a method known per se, such as a fractional recrystallization method, a chiral column method, a diastereomer method etc.

### 1) Fractional recrystallization method

A method wherein a salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine etc.) is formed, which is separated by a fractional recrystallization method, and if desired, a neutralization step to give a free optical isomer.

### 2) Chiral column method

A method wherein a racemate or a salt thereof is applied to a column (a chiral column) for separation of an optical isomer to allow separation. In the case of a liquid chromatography, for example, a mixture of the optical isomers is applied to a chiral column such as ENANTIO-OVM (manufactured by Tosoh Corporation), CHIRAL series (manufactured by Daicel Corporation) and the like, and developed with water, various buffers (e.g., phosphate buffer, etc.) and organic solvents (e.g., ethanol, methanol, isopropanol, acetonitrile, trifluoroacetic acid, diethylamine, etc.) as an eluent, solely or in admixture to separate the optical isomer. In the case of a gas chromatography, for example, a chiral column such as CP-Chirasil-DeX CB (manufactured by GL Sciences Inc.) and the like is used to allow separation.

### 3) Diastereomer method

A method wherein a racemic mixture is prepared into a diastereomeric mixture by chemical reaction with an optically active reagent, which is made into a single substance by a typical separation means (e.g., a fractional recrystallization method, a chromatography method etc.) and the like, and is subjected to a chemical treatment such as hydrolysis reaction and the like to separate an optically active reagent moiety, whereby an optical isomer is obtained. For example, when compound (I) contains hydroxy or primary or secondary amino in a molecule, the compound and an optically active organic acid (e.g., MTPA [α-methoxy-α-(trifluoromethyl)phenylacetic acid], (-)-menthoxyacetic acid etc.) and the like are subjected to condensation reaction to give diastereomers of the ester compound or the amide compound, respectively. When compound (I) has a carboxylic acid group, the compound and an optically active amine or an optically active alcohol reagent are subjected to condensation reaction to give diastereomers of the amide compound or the ester compound, respectively. The separated diastereomer is converted to an optical isomer of the original compound by acid hydrolysis or base hydrolysis reaction.

Compound (I) may be a crystal.

The crystal of compound (I) can be produced according to a crystallization method known per se.

Examples of the crystallization method include crystallization method from a solution, crystallization method from vapor, crystallization method from a melt, and the like.

The "crystallization method from a solution" is typically a method of shifting a non-saturated state to supersaturated state by varying factors involved in solubility of compounds (solvent composition, pH, temperature, ionic strength, redox state, etc.) or the amount of solvent. Specific examples thereof include a concentration method, a slow cooling method, a reaction method (a diffusion method, an electrolysis method), a hydrothermal growth method, a flux method and the like. Examples of the solvent to be used include aromatic hydrocarbons (e.g., benzene, toluene, xylene, etc.), halogenated hydrocarbons (e.g., dichloromethane, chloroform, etc.), saturated hydrocarbons (e.g., hexane, heptane, cyclohexane, etc.), ethers (e.g., diethyl ether, diisopropyl ether, tetrahydrofuran, 1,4-dioxane, etc.), nitriles (e.g., acetonitrile, etc.), ketones (e.g., acetone, etc.), sulfoxides (e.g., dimethyl sulfoxide, etc.), acid amides (e.g., N,N-dimethylformamide, etc.), esters (e.g., ethyl acetate, etc.), alcohols (e.g., methanol, ethanol, isopropyl alcohol, etc.), water and the like. These solvents are used alone or in a combination of two or more at a suitable ratio (e.g., 1:1 to 1:100 (a volume ratio)). Where necessary, a seed crystal can be used.

The "crystallization method from vapor" is, for example, a vaporization method (a sealed tube method, a gas stream method), a gas phase reaction method, a chemical transportation method and the like.

The "crystallization method from a melt" is, for example, a normal freezing method (a pulling method, a temperature gradient method, a Bridgman method), a zone melting method (a zone leveling method, a floating zone method), a special growth method (a VLS method, a liquid phase epitaxy method) and the like.

Preferable examples of the crystallization method include a method comprising dissolving compound (I) in a suitable solvent (e.g., alcohols such as methanol, ethanol etc.) at 20°C to 120°C, and cooling the obtained solution to a temperature (e.g., 0 - 50°C, preferably 0 - 20°C) not higher than the dissolution temperature, and the like.

The thus-obtained crystals of the present invention can be isolated, for example, by filtration and the like.

An analysis method of the obtained crystal is generally a method of crystal analysis by powder X-ray diffraction. As a method of determining crystal orientation, for example, a mechanical method or an optical method and the like can also be used.

The crystal of compound (I) obtained by the above-mentioned production method (hereinafter to be abbreviated as "the crystal of the present invention") has high purity, high quality, and low hygroscopicity, is not denatured even after a long-term preservation under general conditions, and is extremely superior in the stability. In addition, it is also superior in the biological properties (e.g., pharmacokinetics (absorption, distribution, metabolism, excretion), efficacy expression etc.) and is extremely useful as a medicament.

In the present specification, the specific rotation ([α]_{D}) means, for example, a specific rotation measured using a polarimeter (JASCO, measured by P-1030 polarimeter (No. AP-2)) and the like.

In the present specification, the melting point is measured by, for example, a micro melting point apparatus (Yanaco, MP-500D) or DSC (differential scanning calorimetry) apparatus (SEIKO, EXSTAR 6000) and the like.

Compound (I) may be used as a prodrug. The prodrug of compound (I) means a compound which is converted to compound (I) with a reaction due to an enzyme, gastric acid and the like under the physiological condition in the living body, that is, a compound which is converted to compound (I) by enzymatic oxidation, reduction, hydrolysis and the like; a compound which is converted to compound (I) by hydrolysis and the like due to gastric acid, and the like.

Examples of the prodrug for compound (I) include
(1) a compound obtained by subjecting an amino in compound (I) to acylation, alkylation or phosphorylation [e.g., a compound obtained by subjecting an amino group in compound (I) to eicosanoylation, alanylation, pentylaminocarbonylation, (5-methyl-2-oxo-1,3-dioxolen-4-yl)methoxycarbonylation, tetrahydrofurylation, pyrrolidylmethylation, pivaloyloxymethylation, tert-butylation, ethoxycarbonylation, tert-butoxycarbonylation, acetylation, cyclopropylcarbonylation and the like];
(2) a compound obtained by subjecting a hydroxy in compound (I) to acylation, alkylation, phosphorylation or boration (e.g., a compound obtained by subjecting a hydroxy in compound (I) to acetylation, palmitoylation, propanoylation, pivaloylation, succinylation, fumarylation, alanylation or dimethylaminomethylcarbonylation, and the like);
(3) a compound obtained by subjecting a carboxy in compound (I) to esterification or amidation (e.g., a compound obtained by subjecting a carboxy in compound (I) to ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation, and the like) and the like. These compounds can be produced from compound (I) according to a method known *per se.*

The prodrug of compound (I) may also be one which is converted to compound (I) under physiological conditions as described in "IYAKUHIN no KAIHATSU (Development of Pharmaceuticals)", Vol. 7, Design of Molecules, p. 163-198, Published by HIROKAWA SHOTEN (1990).

In the present specification, compound (I) and a prodrug thereof are sometimes collectively abbreviated as "the compound of the present invention".

Compound (I) may be a hydrate, a non-hydrate, a solvate or a non-solvate.

Compound (I) also encompasses a compound labeled with an isotope (e.g., ³H, ¹⁴C, ³⁵S, ¹²⁵I etc.) and the like.

Compound (I) also encompasses a deuterium conversion form wherein ¹H is converted to ²H(D).

Compound (I) also encompasses a tautomer thereof.

Compound (I) may be a pharmaceutically acceptable co-crystal or co-crystal salt. Here, the co-crystal or co-crystal salt means a crystalline substance consisting of two or more particular substances which are solids at room temperature, each having different physical properties (e.g., structure, melting point, heat of melting, hygroscopicity, solubility, stability etc.). The cocrystal and cocrystal salt can be produced by cocrystallization method known per se.

Compound (I) may also be used as a PET tracer.

Since the compound of the present invention has superior PKC (particularly, PKC-θ) inhibitory action, it is also useful as safe medicaments based on such action. For example, the medicament of the present invention containing the compound of the present invention can be used for a mammal (e.g., mouse, rat, hamster, rabbit, cat, dog, bovine, sheep, monkey, human etc.) as a prophylactic or therapeutic agent for PKC (particularly, PKC-θ)-associated diseases, and T cell-associated diseases, more specifically, the diseases described in (1) - (5) below.
(1) inflammatory diseases (e.g., acute pancreatitis, chronic pancreatitis, asthma, adult respiratory distress syndrome, chronic obstructive pulmonary disease (COPD), inflammatory bone disease, pulmonary sarcoidosis, inflammatory bowel disease, celiac disease, hepatitis, systemic inflammatory response syndrome (SIRS), postoperative or posttraumatic inflammation, pneumonia, nephritis, meningitis, cystitis, pharyngolaryngitis, gastric mucosal injury, meningitis, spondylitis, arthritis, dermatitis, chronic pneumonia, bronchitis, pulmonary infarction, silicosis, pulmonary sarcoidosis, diabetic nephropathy, uveitis, hidradenitis suppurativa, gout etc.)
(2) immune diseases (e.g., rheumatoid arthritis, psoriasis, inflammatory bowel disease (e.g., Crohn's disease, ulcerative colitis etc.), Sjogren's syndrome, Behcet's syndrome, multiple sclerosis, systemic lupus erythematosus, lupus nephritis, discoid lupus erythematosus, Castleman's disease, ankylopoietic spondylarthritis, polymyositis, dermatomyositis (DM), polyarteritis nodosa (PN), mixed connective tissue disease (MCTD), scleroderma, profundus lupus erythematosus, chronic thyroiditis, Graves' disease, autoimmune gastritis, type I and type II diabetes, autoimmune hemolytic anemia, autoimmune neutropenia, thrombocytopenia, atopic dermatitis, chronic active hepatitis, myasthenia gravis, graft versus host disease, Addison's disease, abnormal immunoresponse, arthritis, dermatitis, radiodermatitis, primary biliary cirrhosis etc.),
(3) osteoarticular degenerative disease (e.g., rheumatoid arthritis, osteoporosis, osteoarthritis etc.),
(4) neoplastic diseases [e.g., malignant tumor, angiogenesis glaucoma, infantile hemangioma, multiple myeloma, chronic sarcoma, metastatic melanoma, Kaposi's sacroma, vascular proliferation, cachexia, metastasis of the breast cancer, cancer (e.g., colon cancer (e.g., familial colorectal cancer, hereditary nonpolyposis colorectal cancer, gastrointestinal stromal tumor etc.), lung cancer (e.g., non-small cell lung cancer, small cell lung cancer, malignant mesothelioma etc.), mesothelioma, pancreatic cancer (e.g., pancreatic duct cancer etc.), gastric cancer (e.g., papillary adenocarcinoma, mucinous adenocarcinoma, adenosquamous carcinoma, etc.), breast cancer (e.g., invasive ductal carcinoma, ductal carcinoma in situ, inflammatory breast cancer etc.), ovarian cancer (e.g., ovarian epithelial carcinoma, extragonadal germ cell tumor, ovarian germ cell tumor, ovarian low malignant potential tumor etc.), prostate cancer (e.g., hormone-dependent prostate cancer, non-hormone dependent prostate cancer etc.), liver cancer (e.g., primary liver cancer, extrahepatic bile duct cancer etc.), thyroid cancer (e.g., medullary thyroid carcinoma etc.), kidney cancer (e.g., renal cell carcinoma, transitional cell carcinoma in kidney and urinary duct etc.), uterine cancer, brain tumor (e.g., pineal astrocytoma, pilocytic astrocytoma, diffuse astrocytoma, anaplastic astrocytoma etc.), melanoma, sarcoma, urinary bladder cancer, hematologic cancer and the like including multiple myeloma, hypophyseal adenoma, glioma, acoustic schwannoma, retinoblastoma, pharyngeal cancer, laryngeal cancer, cancer of the tongue, thymoma, esophagus cancer, duodenal cancer, colon cancer, rectal cancer, hepatoma, pancreatic endocrine tumor, bile duct cancer, gall bladder cancer, penile cancer, urinary duct cancer, testis tumor, vulvar cancer, cervix cancer, uterine body cancer, uterus sarcoma, cholionic disease, vaginal cancer, skin cancer, fungoid mycosis, basal cell tumor, soft tissue sarcoma, malignant lymphoma, Hodgkin's disease, myelodysplastic syndrome, adult T cell leukemia, chronic bone marrow proliferative disease, pancreatic endocrine tumor, fibrous histiocytoma, leiomyosarcoma, rhabdomyosarcoma, cancer of unknown primary), leukemia (e.g., acute leukemia (e.g., acute lymphocytic leukemia, acute myeloid leukemia etc.), chronic leukemia (e.g., chronic lymphocytic leukemia, chronic myeloid leukemia etc.), myelodysplastic syndrome etc.), uterus sarcoma (e.g., mixed mesodermal tumor, uterine leiomyosarcoma, endometrial stromal tumor etc.), myelofibrosis and the like],
(5) central nervous system disease (e.g., schizophrenia, Alzheimer's disease (e.g., dementia of Alzheimer type)).

The medicament of the present invention can be preferably used as a prophylactic or therapeutic agent for autoimmune diseases, inflammatory diseases, degenerative joint and bone disease, central nervous system disease or neoplastic disease, more preferably, graft versus host disease, aplastic anemia, systemic lupus erythematosus, lupus nephritis, pemphigus, inflammatory bowel disease (preferably, Crohn's disease or ulcerative colitis), pure red cell aplasia, myasthenia gravis, asthma, vasculitis, spondylarthritis ankylopoietica, rheumatoid arthritis, psoriasis, Sjogren's syndrome, atopic dermatitis, Behcet's syndrome, multiple sclerosis, Alzheimer's disease (preferably, dementia of Alzheimer type), adult respiratory distress syndrome, celiac disease, Castleman's disease, leukemia, uterine leiomyosarcoma, prostate cancer, multiple myeloma, cachexia, myelofibrosis, nephrotic syndrome, or rejection in transplantation such as kidney transplant, liver transplant, cardiac transplant, lung transplant, pancreas transplant, small bowel transplant, hematopoietic stem cell transplant and the like.

Here, the above-mentioned "prophylaxis" of a disease means, for example, administration of a medicament containing the compound of the present invention to patients who are expected to have a high risk of the onset due to some factor relating to the disease but have not developed the disease or patients who have developed the disease but do not have a subjective symptom, or administration of a medicament containing the compound of the present invention to patients who are feared to show recurrence of the disease after treatment of the disease.

The medicament of the present invention shows superior pharmacokinetics (e.g., a half-life of the drug in plasma), low toxicity (e.g., HERG inhibition, CYP inhibition, CYP induction), and decreased drug interaction. The compound of the present invention can be directly used as a medicament, or as the medicament of the present invention by producing a pharmaceutical composition by mixing with a pharmaceutically acceptable carrier by a means known per se and generally used in a production method of pharmaceutical preparations. The medicament of the present invention can be orally or parenterally administered safely to mammals (e.g., humans, monkeys, cows, horses, pigs, mice, rats, hamsters, rabbits, cats, dogs, sheep and goats). The medicament containing the compound of the present invention can be used in the compound of the present invention as the sole regimen or as a pharmaceutical composition mixed with the compound of the present invention and a pharmacologically acceptable carrier according to method known per se as a production method for pharmaceutical preparation (e.g., the method described in the Japanese Pharmacopoeia etc.). The medicament containing the compound of the present invention can be safely administered orally or parenterally (e.g., intravenous, intramuscular, subcutaneous, intraorgan, intranasal, intradermal, instillation, intracerebral, intrarectal, vaginal, intraperitoneal, interior of a tumor, proximal of a tumor, lesion and the like) in the form of a tablet (inclusive of a sugar-coated tablet, a film-coated tablet, a sublingual tablet, an orally disintegrating tablet, a buccal tablet and the like), a pill, powders, granules, a capsule (inclusive of a soft capsule, a microcapsule), a troche, syrups, a liquid drug, emulsions, a suspension drug, controlled-release preparation (e.g., immediate-release preparation, sustained-release preparation, sustained-release microcapsule), aerosols, films (e.g., orally disintegrable films, oral mucosal adhesive film), an injection drug (e.g., subcutaneous injection, intravenous injection, intramuscular injection, intraperitoneal injection), drip infusion, transdermal absorption type preparation, cream, ointment, lotion, adhesive preparation, suppository (e.g., rectal suppository, vaginal suppository), pellet, nasal preparations, pulmonary preparation (e.g., inhalant), eye drop and the like.

The content of the compound of the present invention in the medicament of the present invention is about 0.01 to about 100% by weight of the entire medicament.

While the dose of the compound of the present invention varies depending on the subject of administration, administration route, disease and the like, for example, for oral administration to a patient with graft-versus-host disease (body weight about 60 kg), it is about 0.01 mg/kg body weight - about 500 mg/kg body weight, preferably about 0.1 mg/kg body weight - about 50 mg/kg body weight, further preferably about 1 mg/kg body weight - 30 mg/kg body weight as an active ingredient (free form of compound (I)) for one day, which is administered once to several times per day.

The pharmaceutically acceptable carrier, which may be used for the production of the medicament of the present invention, may be exemplified by various organic or inorganic carrier materials that are conventionally used as preparation materials, for example, excipient, lubricant, binding agent and disintegrant for solid preparations; or solvent, solubilizing agent, suspending agent, isotonic agent, buffering agent, soothing agent and the like for liquid preparations. Furthermore, when necessary, ordinary additives such as preservative, antioxidant, colorant, sweetening agent, adsorbing agent, wetting agent and the like can be also used as appropriate in an appropriate amount.

Examples of the excipient include lactose, white sugar, D-mannitol, starch, corn starch, crystalline cellulose, light anhydrous silicic acid and the like.

Examples of the lubricant include magnesium stearate, calcium stearate, talc, colloidal silica and the like. Examples of the binding agent include crystalline cellulose, white sugar, D-mannitol, dextrin, hydroxypropylcellulose, hydroxypropylmethylcellulose, polyvinylpyrrolidone, starch, sucrose, gelatin, methylcellulose, carboxymethylcellulose sodium and the like.

Examples of the disintegrant include starch, carboxymethylcellulose, carboxymethylcellulose calcium, carboxymethylstarch sodium, L-hydroxypropylcellulose and the like.

Examples of the solvent include water for injection, alcohol, propylene glycol, Macrogol, sesame oil, corn oil, olive oil and the like.

Examples of the solubilizing agent include polyethylene glycol, propylene glycol, D-mannitol, benzyl benzoate, ethanol, trisaminomethane, cholesterol, triethanolamine, sodium carbonate, sodium citrate and the like.

Examples of the suspending agent include surfactants such as stearyl triethanolamine, sodium lauryl sulfate, laurylaminopropionic acid, lecithin, benzalkonium chloride, benzetonium chloride, glycerin monostearate and the like; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose and the like; and the like.

Examples of the isotonic agent include glucose, D-sorbitol, sodium chloride, glycerin, D-mannitol and the like.

Examples of the buffering agent include buffer solutions such as phosphates, acetates, carbonates, citrates and the like.

Examples of the soothing agent include benzyl alcohol and the like.

Examples of the preservative include paraoxybenzoate esters, chlorobutanol, benzyl alcohol, phenylethyl alcohol, dehydroacetic acid, sorbic acid and the like.

Examples of the antioxidant include sulfite salts, ascorbic acid, α-tocopherol and the like.

For the prophylaxis or treatment of various diseases, the compound of the present invention can also be used together with other medicaments. In the following, a medicament to be used when the compound of the present invention is used together with other drug is referred to as "the combination agent of the present invention".

For example, the compound of the present invention can also be used together with the following drugs.
(1) non-steroidal anti-inflammatory drug (NSAIDs)
   (i) Classical NSAIDs
      alcofenac, aceclofenac, sulindac, tolmetin, etodolac, fenoprofen, thiaprofenic acid, meclofenamic acid, meloxicam, tenoxicam, lornoxicam, nabumeton, acetaminophen, phenacetin, ethenzamide, sulpyrine, antipyrine, migrenin, aspirin, mefenamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, piroxicam, epirizole, tiaramide hydrochloride, zaltoprofen, gabexate mesylate, camostat mesylate, ulinastatin, colchicine, probenecid, sulfinpyrazone, benzbromarone, allopurinol, sodium aurothiomalate, hyaluronate sodium, sodium salicylate, morphine hydrochloride, salicylic acid, atropine, scopolamine, morphine, pethidine, levorphanol, oxymorphone or a salt thereof and the like.
   (ii) cyclooxygenase inhibitor (COX-1 selective inhibitor, COX-2 selective inhibitor and the like)
      salicylic acid derivatives (e.g., celecoxib, aspirin), etoricoxib, valdecoxib, diclofenac, indomethacin, loxoprofen and the like.
   (iii) nitric oxide-releasing NSAIDs
   (iv) JAK inhibitor
      tofacitinib, ruxolitinib and the like.
(2) disease-modifying anti-rheumatic drugs (DMARDs)
   (i) Gold preparation
      auranofin and the like.
   (ii) penicillamine
      D-penicillamine.
   (iii) aminosalicylic acid preparation
      sulfasalazine, mesalamine, olsalazine, balsalazide.
   (iv) antimalarial drug
      chloroquine and the like.
   (v) pyrimidine synthesis inhibitor
      leflunomide and the like.
   (vi) prograf
(3) anti-cytokine drug
   (I) protein drug
      (i) TNF inhibitor
         etanercept, infliximab, adalimumab, certolizumab pegol, golimumab, PASSTNF-α, soluble TNF-α receptor, TNF-α binding protein, anti-TNF-α antibody and the like.
      (ii) interleukin-1 inhibitor
         anakinra (interleukin-1 receptor antagonist), soluble interleukin-1 receptor and the like.
      (iii) interleukin-6 inhibitor
         tocilizumab (anti-interleukin-6 receptor antibody), anti-interleukin-6 antibody and the like.
      (iv) interleukin-10 drug
         interleukin-10 and the like.
      (v) interleukin-12/23 inhibitor
         ustekinumab, briakinumab (anti-interleukin-12/23 antibody) and the like.
   (II) non-protein drug
      (i) MAPK inhibitor
         BMS-582949 and the like.
      (ii) gene modulator
         inhibitor of molecule involved in signal transduction, such as NF-κ, NF-κB, IKK-1, IKK-2, AP-1 and the like, and the like.
      (iii) cytokine production inhibitor
         iguratimod, tetomilast and the like.
      (iv) TNF-α converting enzyme inhibitor
      (v) interleukin-1β converting enzyme inhibitor
         VX-765 and the like.
      (vi) interleukin-6 antagonist
         HMPL-004 and the like.
      (vii) interleukin-8 inhibitor
         IL-8 antagonist, CXCR1 & CXCR2 antagonist, reparixin and the like.
      (viii) chemokine antagonist
         CCR9 antagonist (CCX-282, CCX-025), MCP-1 antagonist and the like.
      (ix) interleukin-2 receptor antagonist
         denileukin, diftitox and the like.
      (x) therapeutic vaccines
         TNF-α vaccine and the like.
      (xi) gene therapy drug
         gene therapy drugs aiming at promoting the expression of gene having an anti-inflammatory action such as interleukin-4, interleukin-10, soluble interleukin-1 receptor, soluble TNF-α receptor and the like.
      (xii) antisense compound
         ISIS 104838 and the like.
(4) integrin inhibitor
   natalizumab, vedolizumab, AJM300, TRK-170, E-6007 and the like.
(5) immunomodulator (immunosuppressant)
   methotrexate, cyclophosphamide, MX-68, atiprimod dihydrochloride, BMS-188667, CKD-461, rimexolone, cyclosporine, tacrolimus, gusperimus, sirolimus, everolimus, azathiopurine, antilymphocyte serum, freeze-dried sulfonated normal immunoglobulin, erythropoietin, colony stimulating factor, interleukin, interferon and the like.
(6) steroid
   dexamethasone, hexestrol, methimazole, betamethasone, triamcinolone, triamcinolone acetonide, fluocinonide, fluocinolone acetonide, predonisolone, methylpredonisolone, cortisone acetate, hydrocortisone, fluorometholone, beclomethasone dipropionate, estriol, budesonide and the like.
(7) angiotensin converting enzyme inhibitor
   enalapril, captopril, ramipril, lisinopril, cilazapril, perindopril and the like.
(8) angiotensin II receptor antagonist
   candesartan, cilexetil (TCV-116), valsartan, irbesartan, olmesartan, eprosartan and the like.
(9) diuretic drug
   hydrochlorothiazide, spironolactone, furosemide, indapamide, bendrofluazide, cyclopenthiazide and the like.
(10) cardiotonic drug
   digoxin, dobutamine and the like.
(11) β receptor antagonist
   carvedilol, metoprolol, atenolol and the like.
(12) Ca sensitizer
   MCC-135 and the like.
(13) Ca channel antagonist
   nifedipine, diltiazem, verapamil and the like.
(14) anti-platelet drug, anticoagulator
   heparin, aspirin, warfarin and the like.
(15) HMG-CoA reductase inhibitor
   atorvastatin, simvastatin and the like.
(16) contraceptive
   (i) sex hormone or derivatives thereof
      gestagen or a derivative thereof (progesterone, 17α-hydroxy progesterone, medroxyprogesterone, medroxyprogesterone acetate, norethisterone, norethisterone enanthate, norethindrone, norethindrone acetate, norethynodrel, levonorgestrel, norgestrel, ethynodiol diacetate, desogestrel, norgestimate, gestodene, progestin, etonogestrel, drospirenone, dienogest, trimegestone, nestorone, chlormadinone acetate, mifepristone, nomegestrol acetate, Org-30659, TX-525, EMM-310525) or a combination agent of a gestagen or a derivative thereof and an estrogen or a derivative thereof (estradiol, estradiol benzoate, estradiol cypionate, estradiol dipropionate, estradiol enanthate, estradiol hexahydrobenzoate, estradiol phenylpropionate, estradiol undecanoate, estradiol valerate, estrone, ethinylestradiol, mestranol) and the like.
   (ii) antiestrogen
      ormeloxifene, mifepristone, Org-33628 and the like.
   (iii) spermatocide
      ushercell and the like.
(17) others
   (i) T cell inhibitors
   (ii) inosine monophosphate dehydrogenase (IMPDH) inhibitor mycophenolate mofetil and the like.
   (iii) adhesion molecule inhibitor
      ISIS-2302, selectin inhibitor, ELAM-1, VCAM-1, ICAM-1 and the like.
   (iv) thalidomide
   (v) cathepsin inhibitor
   (vi) matrix metalloprotease (MMPs) inhibitor V-85546 and the like.
   (vii) glucose-6-phosphate dehydrogenase inhibitor
   (viii) Dihydroorotate dehydrogenase (DHODH) inhibitor
   (ix) phosphodiesterase IV (PDE IV) inhibitor
      roflumilast, CG-1088 and the like.
   (x) phospholipase A₂ inhibitor
   (xi) iNOS inhibitor
      VAS-203 and the like.
   (xii) microtubule stimulating drug
      paclitaxel and the like.
   (xiii) microtuble inhibitor
      reumacon and the like.
   (xiv) MHC class II antagonist
   (xv) prostacyclin agonist
      iloprost and the like.
   (xvi) CD4 antagonist
      zanolimumab and the like.
   (xvii) CD23 antagonist
   (xviii) LTB4 receptor antagonist
      DW-1305 and the like.
   (xix) 5-lipoxygenase inhibitor
      zileuton and the like.
   (xx) cholinesterase inhibitor
      galanthamine and the like.
   (xxi) tyrosine kinase inhibitor
      Tyk2 inhibitor (WO2010142752) and the like.
   (xxii) carepsin B inhibitor
   (xxiii) adenosine deaminase inhibitor
      pentostatin and the like.
   (xxiv) osteogenesis stimulator
   (xxv) dipeptidylpeptidase inhibitor
   (xxvi) collagen agonist
   (xxvii) capsaicin cream
   (xxviii) hyaluronic acid derivative
      synvisc (hylan G-F 20), orthovisc and the like.
   (xxix) glucosamine sulfate
   (xxx) amiprilose
   (xxxi) CD-20 inhibitor
      rituximab, ibritumomab, tositumomab, ofatumumab and the like.
   (xxxii) BAFF inhibitor
      belimumab, tabalumab, atacicept, A-623 and the like.
   (xxxiii) CD52 inhibitor
      alemtuzumab and the like.
   (xxxiv) IL-17 inhibitor
      secukinumab (AIN-457), LY-2439821, AMG827 and the like.
   (xxxv) PDE4 inhibitor
      Roflumilast, Apremilast.
   (xxxvi) human lymphocyte ·thymocyte antibody
      anti-human thymocyte rabbit immunoglobulin, anti-human thymocyte horse immunoglobulin etc.
   (xxxvii) anti CD3 antibody
      visilizumab etc.
   (xxxviii) anti CD25 antibody
      basiliximab, daclizumab etc.
   (xxxix) CTLA-4-Ig
      abatacept, belatacept etc.

Other concomitant drugs besides the above-mentioned include, for example, antibacterial agent, antifungal agent, antiprotozoal agent, antibiotic, antitussive and expectorant drug, sedative, anesthetic, antiulcer drug, antiarrhythmic agent, hypotensive diuretic drug, anticoagulant, tranquilizer, antipsychotic, antitumor drug, hypolipidemic drug, muscle relaxant, antiepileptic drug, antidepressant, antiallergic drug, cardiac stimulants, therapeutic drug for arrhythmia, vasodilator, vasoconstrictor, hypotensive diuretic, therapeutic drug for diabetes, antinarcotic, vitamin, vitamin derivative, antiasthmatic, therapeutic agent for pollakisuria/anischuria, antipruritic drug, therapeutic agent for atopic dermatitis, therapeutic agent for allergic rhinitis, hypertensor, endotoxin-antagonist or -antibody, signal transduction inhibitor, inhibitor of inflammatory mediator activity, antibody to inhibit inflammatory mediator activity, inhibitor of anti-inflammatory mediator activity, antibody to inhibit anti-inflammatory mediator activity and the like. Specific examples thereof include the following.

(1) Antibacterial agent
   (i) sulfa drug
      sulfamethizole, sulfisoxazole, sulfamonomethoxine, sulfamethizole, salazosulfapyridine, silver sulfadiazine and the like.
   (ii) quinolone antibacterial agent
      nalidixic acid, pipemidic acid trihydrate, enoxacin, norfloxacin, ofloxacin, tosufloxacin tosylate, ciprofloxacin hydrochloride, lomefloxacin hydrochloride, sparfloxacin, fleroxacin and the like.
   (iii) antiphthisic
      isoniazid, ethambutol (ethambutol hydrochloride), p-aminosalicylic acid (calcium p-aminosalicylate), pyrazinamide, ethionamide, protionamide, rifampicin, streptomycin sulfate, kanamycin sulfate, cycloserine and the like.
   (iv) antiacidfast bacterium drug
      diaphenylsulfone, rifampicin and the like.
   (v) antiviral drug
      idoxuridine, acyclovir, vidarabine, gancyclovir, foscarnet and the like.
   (vi) anti-HIV agent
      zidovudine, didanosine, zalcitabine, indinavir sulfate ethanolate, ritonavir and the like.
   (vii) antispirochetele
   (viii) antibiotic
      tetracycline hydrochloride, ampicillin, piperacillin, gentamicin, dibekacin, kanendomycin, lividomycin, tobramycin, amikacin, fradiomycin, sisomicin, tetracycline, oxytetracycline, rolitetracycline, doxycycline, ampicillin, piperacillin, ticarcillin, cephalothin, cephapirin, cephaloridine, cefaclor, cephalexin, cefroxadine, cefadroxil, cefamandole, cefotoam, cefuroxime, cefotiam, cefotiam hexetil, cefuroxime axetil, cefdinir, cefditoren pivoxil, ceftazidime, cefpiramide, cefsulodin, cefmenoxime, cefpodoxime proxetil, cefpirome, cefozopran, cefepime, cefsulodin, cefmenoxime, cefmetazole, cefminox, cefoxitin, cefbuperazone, latamoxef, flomoxef, cefazolin, cefotaxime, cefoperazone, ceftizoxime, moxalactam, thienamycin, sulfazecin, aztreonam or a salt a salt thereof, griseofulvin, lankacidin-group [Journal of Antibiotics (J. Antibiotics), 38, 877-885(1985)], azole compound [2-[(1R,2R)-2-(2,4-difluorophenyl)-2-hydroxy-1-methyl-3-(1H-1,2,4-triazol-1-yl)propyl]-4-[4-(2,2,3,3-tetrafluoropropoxy)phenyl]-3(2H,4H)-1,2,4-triazolone, fluconazole, itraconazole, imipenem, meropenem, trimethoprim, sulfamethoxazole and the like] and the like.
(2) antifungal agent
   (i) polyethylene antibiotic (e.g., amphotericin B, nystatin, trichomycin)
   (ii) griseofulvin, pyrrolnitrin and the like
   (iii) cytosine metabolism antagonist (e.g., flucytosine)
   (iv) imidazole derivative (e.g., econazole, clotrimazole, miconazole nitrate, bifonazole, croconazole)
   (v) triazole derivative (e.g., fluconazole, itraconazole)
   (vi) thiocarbamic acid derivative (e.g., trinaphthol) and the like.
(3) antiprotozoal agent
   metronidazole, tinidazole, diethylcarbamazine citrate, quinine hydrochloride, quinine sulfate and the like.
(4) antitussive and expectorant drug
   ephedrine hydrochloride, noscapine hydrochloride, codeine phosphate, dihydrocodeine phosphate, isoproterenol hydrochloride, ephedrine hydrochloride, methylephedrine hydrochloride, noscapine hydrochloride, alloclamide, chlophedianol, picoperidamine, cloperastine, protokylol, isoproterenol, salbutamol, terputaline, oxypetebanol, morphine hydrochloride, dextropethorfan hydrobromide, oxycodone hydrochloride, dimorphan phosphate, tipepidine hibenzate, pentoxyverine citrate, clofedanol hydrochloride, benzonatate, guaifenesin, bromhexine hydrochloride, ambroxol hydrochloride, acetylcysteine, ethyl cysteine hydrochloride, carbocysteine and the like.
(5) sedative
   chlorpromazine hydrochloride, atropine sulfate, phenobarbital, barbital, amobarbital, pentobarbital, thiopental sodium, thiamylal sodium, nitrazepam, estazolam, flurazepam, haloxazolam, triazolam, flunitrazepam, bromovalerylurea, chloral hydrate, triclofos sodium and the like.
(6) anesthetic
   (6-1) local anesthetic
      cocaine hydrochloride, procaine hydrochloride, lidocaine, dibucaine hydrochloride, tetracaine hydrochloride, mepivacaine hydrochloride, bupivacaine hydrochloride, oxybuprocaine hydrochloride, ethyl aminobenzoate, oxethazaine and the like.
   (6-2) general anesthetic
      (i) inhalation anesthetic (e.g., ether, halothane, nitrous oxide, isoflurane, enflurane),
      (ii) intravenous anesthetic (e.g., ketamine hydrochloride, droperidol, thiopental sodium, thiamylal sodium, pentobarbital) and the like.
(7) antiulcer drug
   histidine hydrochloride, lansoprazole, metoclopramide, pirenzepine, cimetidine, ranitidine, famotidine, urogastrine, oxethazaine, proglumide, omeprazole, sucralfate, sulpiride, cetraxate, gefarnate, aldioxa, teprenone, prostaglandin and the like.
(8) antiarrhythmic agent
   (i) sodium channel blocker (e.g., quinidine, procainamide, disopyramide, ajmaline, lidocaine, mexiletine, phenytoin),
   (ii) β-blocker (e.g., propranolol, alprenolol, bufetolol hydrochloride, oxprenolol, atenolol, acebutolol, metoprolol, bisoprolol, pindolol, carteolol, arotinolol hydrochloride),
   (iii) potassium channel blocker (e.g., amiodarone),
   (iv) calcium channel blocker (e.g., verapamil, diltiazem) and the like.
(9) hypotensive diuretic drug
   hexamethonium bromide, clonidine hydrochloride, hydrochlorothiazide, trichlormethiazide, furosemide, ethacrynic acid, bumetanide, mefruside, azosemide, spironolactone, potassium canrenoate, triamterene, amiloride, acetazolamide, D-mannitol, isosorbide, aminophylline and the like.
(10) anticoagulant
   heparin sodium, sodium citrate, activated protein C, tissue factor pathway inhibitor, antithrombin III, dalteparin sodium, warfarin potassium, argatroban, gabexate, sodium citrate, ozagrel sodium, ethyl icosapentate, beraprost sodium, alprostadil, ticlopidine hydrochloride, pentoxifylline, dipyridamole, tisokinase, urokinase, streptokinase and the like.
(11) tranquilizer
   diazepam, lorazepam, oxazepam, chlordiazepoxide, medazepam, oxazolam, cloxazolam, clotiazepam, bromazepam, etizolam, fludiazepam, hydroxyzine and the like.
(12) antipsychotic
   chlorpromazine hydrochloride, prochlorperazine, trifluoperazine, thioridazine hydrochloride, perphenazine maleate, fluphenazine enanthate, prochlorperazine maleate, levomepromazine maleate, promethazine hydrochloride, haloperidol, bromperidol, spiperone, reserpine, clocapramine hydrochloride, sulpiride, zotepine and the like.
(13) antitumor drug
   6-O-(N-chloroacetylcarbamoyl)fumagillol, bleomycin, methotrexate, actinomycin D, mitomycin C, daunorubicin, adriamycin, neocarzinostatin, cytosine arabinoside, fluorouracil, tetrahydrofuryl-5-fluorouracil, picibanil, lentinan, levamisole, bestatin, azimexon, glycyrrhizin, doxorubicin hydrochloride, aclarubicin hydrochloride, bleomycin hydrochloride, peplomycin sulfate, vincristine sulfate, vinblastine sulfate, irinotecan hydrochloride, cyclophosphamide, melphalan, zusulfan, thiotepa, procarbazine hydrochloride, cisplatin, azathioprine, mercaptopurine, tegafur, carmofur, cytarabine, methyltestosterone, testosterone propionate, testosterone enanthate, mepitiostane, fosfestrol, chlormadinone acetate, leuprorelin acetate, buserelin acetate, bortezomib, lenalidomide, clofarabine, azacitidine, imatinib and the like.
(14) hypolipidemic drug
   clofibrate, ethyl 2-chloro-3-[4-(2-methyl-2-phenylpropoxy)phenyl]propionate [Chem. Pharm. Bull, 38, 2792-2796 (1990)], pravastatin, simvastatin, probucol, bezafibrate, clinofibrate, nicomol, cholestyramine, dextran sulfate sodium and the like.
(15) muscle relaxant
   pridinol, tubocurarine, pancuronium, tolperisone hydrochloride, chlorphenesin carbamate, baclofen, chlormezanone, mephenesin, chlorzoxazone, eperisone, tizanidine and the like.
(16) antiepileptic drug
   phenytoin, ethosuximide, acetazolamide, chlordiazepoxide, tripethadione, carbamazepine, phenobarbital, primidone, sulthiame, sodium valproate, clonazepam, diazepam, nitrazepam and the like.
(17) antidepressant
   imipramine, clomipramine, noxiptiline, phenelzine, amitriptyline hydrochloride, nortriptyline hydrochloride, amoxapine, mianserin hydrochloride, maprotiline hydrochloride, sulpiride, fluvoxamine maleate, trazodone hydrochloride and the like.
(18) antiallergic drug
   diphenhydramine, chlorpheniramine, tripelennamine, metodilamine, clemizole, diphenylpyraline, methoxyphenamine, sodium cromoglicate, tranilast, repirinast, amlexanox, ibudilast, ketotifen, terfenadine, mequitazine, azelastine hydrochloride, epinastine, ozagrel hydrochloride, pranlukast hydrate, seratrodast and the like.
(19) cardiac stimulants
   trans-π-oxocamphor, terephyllol, aminophylline, etilefrine, dopamine, dobutamine, denopamine, vesinarine, amrinone, pimobendan, ubidecarenone, digitoxin, digoxin, methyldigoxin, lanatoside C, G-strophanthin and the like.
(20) vasodilator
   oxyfedrine, diltiazem, tolazoline, hexobendine, bamethan, clonidine, methyldopa, guanabenz and the like.
(21) vasoconstrictor
   dopamine, dobutamine denopamine and the like.
(22) hypotensive diuretic
   hexamethonium bromide, pentolinium, mecamylamine, ecarazine, clonidine, diltiazem, nifedipine and the like.
(23) therapeutic drug for diabetes
   tolbutamide, chlorpropamide, acetohexamide, glibenclamide, tolazamide, acarbose, epalrestat, troglitazone, glucagon, glymidine, glipuzide, phenformin, puformin, metformin and the like.
(24) antinarcotic
   levallorphan, nalorphine, naloxone or a salt thereof and the like.
(25) liposoluble vitamins
   (i) vitamin A: vitamin A₁, vitamin A₂ and retinol palmitate
   (ii) vitamin D: vitamin D₁, D₂, D₃, D₄ and D₅
   (iii) vitamin E: α-tocopherol, β-tocopherol, γ-tocopherol, δ-tocopherol, dl-α-tocopherol nicotinate
   (iv) vitamin K: vitamin K₁, K₂, K₃ and K₄
   (v) folic acid (vitamin M), folinate calcium and the like.
(26) vitamin derivative
   various derivatives of vitamins, for example, vitamin D₃ derivatives such as 5,6-trans-cholecalciferol, 2,5-hydroxycholecalciferol, 1-α-hydroxycholecalciferol and the like, vitamin D₂ derivatives such as 5,6-trans-ergocalciferol and the like, and the like.
(27) antiasthmatic
   isoprenaline hydrochloride, salbutamol sulfate, procaterol hydrochloride, terbutaline sulfate, trimetoquinol hydrochloride, tulobuterol hydrochloride, orciprenaline sulfate, fenoterol hydrobromide, ephedrine hydrochloride, ipratropium bromide, oxitropium bromide, flutropium bromide, theophylline, aminophylline, sodium cromoglicate, tranilast, repirinast, amlexanox, ibudilast, ketotifen, terfenadine, mequitazine, azelastine, epinastine, ozagrel hydrochloride, pranlkast hydrate, seratrodast, dexamethasone, prednisolone, hydrocortisone, hydrocortisone sodium succinate, beclometasone dipropionate and the like.
(28) therapeutic agent for pollakisuria/anischuria
   flavoxate hydrochloride and the like.
(29) therapeutic agent for atopic dermatitis
   sodium cromoglicate and the like.
(30) therapeutic agent for allergic rhinitis
   sodium cromoglicate, chlorpheniramine maleate, alimemazine tartrate, clemastine fumarate, homochlorcyclizine hydrochloride, fexofenadine, mequitazine and the like.
(31) hypertensor
   dopamine, dobutamine, denopamine, digitoxin, digoxin, methyldigoxin, lanatoside C, G-strophanthin and the like.
(32) therapeutic drug before hematopoietic stem cell transplantation
   busulfan, fludarabine, cladribine, etoposide.
(33) others
   hydroxycam, diacerein, megestrol acetate, nicergoline, Clofazimine, etretinate, bisphosphonate, defibrotide, prostaglandins and the like.

For combined use, the administration time of the compound of the present invention and the concomitant drug is not restricted, and the compound of the present invention and the concomitant drug can be administered to an administration subject simultaneously, or may be administered at different times. The dosage of the concomitant drug may be determined according to the dose clinically used, and can be appropriately selected depending on an administration subject, administration route, disease, combination and the like.

The administration form of the combined use is not particularly limited, and the compound of the present invention and a concomitant drug only need to be combined on administration. Examples of such administration mode include the following:
(1) administration of a single preparation obtained by simultaneously processing the compound of the present invention and the concomitant drug, (2) simultaneous administration of two kinds of preparations of the compound of the present invention and the concomitant drug, which have been separately produced, by the same administration route, (3) administration of two kinds of preparations of the compound of the present invention and the concomitant drug, which have been separately produced, by the same administration route in a staggered manner, (4) simultaneous administration of two kinds of preparations of the compound of the present invention and the concomitant drug, which have been separately produced, by different administration routes, (5) administration of two kinds of preparations of the compound of the present invention and the concomitant drug, which have been separately produced, by different administration routes in a staggered manner (e.g., administration in the order of the compound of the present invention and the concomitant drug, or in the reverse order) and the like.

The mixing ratio of the compound of the present invention and a concomitant drug in the combination agent of the present invention can be appropriately selected based on the subject of administration, administration route, disease and the like.

For example, while the content of the compound of the present invention in the combination agent of the present invention varies depending on the preparation form, it is generally about 0.01 - 100 wt%, preferably about 0.1 - 50 wt%, more preferably about 0.5 - 20 wt%, of the whole preparation.

The content of the concomitant drug in the combination agent of the present invention varies depending on the preparation form, and generally about 0.01 to 100% by weight, preferably about 0.1 to 50% by weight, further preferably about 0.5 to 20% by weight, of the entire preparation.

While the content of the additive such as a carrier and the like in the combination agent of the present invention varies depending on the form of a preparation, it is generally about 1 to 99.99% by weight, preferably about 10 to 90% by weight, based on the preparation.

When the compound of the present invention and the concomitant drug are separately prepared, the same content may be adopted.

The dose varies depending on the kind of the compound of the present invention, administration route, symptom, age of patients and the like. For example, for oral administration to patients with graft-versus-host disease (body weight about 60 kg), about 0.1 mg/kg body weight - about 50 mg/kg body weight, preferably about 1 mg/kg body weight - 30 mg/kg body weight, of a free form of compound (I) can be administered once to several portions per day.

The dose of the pharmaceutical composition of the present invention as a sustained-release preparation varies depending on the kind and content of compound (I), dosage form, period of sustained drug release, subject animal of administration (e.g., mammals such as mouse, rat, hamster, guinea pig, rabbit, cat, dog, bovine, horse, swine, sheep, monkey, human and the like), and administration object. For example, for application by parenteral administration, about 0.1 to about 100 mg of compound (I) needs to be released from the administered preparation per 1 week.

Any amount of the concomitant drug can be adopted as long as the side effects do not cause a problem. The daily dosage in terms of the concomitant drug varies depending on the severity of symptom, age, sex, body weight, sensitivity difference of the subject, administration period, interval, and nature, pharmacology, kind of the pharmaceutical preparation, kind of effective ingredient, and the like, and not particularly restricted, and the amount of a drug is, in the case of oral administration for example, generally about 0.001 to 2000 mg, preferably about 0.01 to 500 mg, further preferably about 0.1 to 100 mg, per 1 kg body weight of a mammal and this is generally administered once to 4-times divided in a day.

When the combination agent of the present invention is administered, the compound of the present invention and the concomitant drug can be administered simultaneously, or may be administered in a staggered manner. When administered at a time interval, the interval varies depending on the effective ingredient to be administered, dosage form and administration method, and, for example, when the concomitant drug is administered first, a method in which the compound of the present invention is administered within time range of from 1 minute to 3 days, preferably from 10 minutes to 1 day, more preferably from 15 minutes to 1 hour, after administration of the concomitant drug is an example. When the compound of the present invention is administered first, a method in which the concomitant drug is administered within time range of from 1 minute to 1 day, preferably from 10 minutes to 6 hours, more preferably from 15 minutes to 1 hour after administration of the compound of the present invention is an example.

### [Examples]

The present invention is explained in detail in the following by referring to Examples, Experimental Examples and Formulation Examples, which are not to be construed as limitative, and the invention may be changed within the scope of the present invention.

In the following Examples, the "room temperature" generally means about 10°C to about 35°C. The ratios indicated for mixed solvents are volume mixing ratios, unless otherwise specified. % means wt%, unless otherwise specified.

In silica gel column chromatography, NH means use of aminopropylsilane-bonded silica gel, Diol means use of 3-(2,3-dihydroxypropoxy)propylsilane-bonded silica gel and DiNH means use of N-(2-aminoethyl)-3-aminopropylsilane-bonded silica gel. In HPLC (high performance liquid chromatography), C18 means use of octadecyl-bonded silica gel. The ratios of elution solvents are volume mixing ratios, unless otherwise specified.

In Examples, the following abbreviations are used.
mp: melting point
MS: mass spectrum
[M+H]⁺, [M-H]⁻. molecular ion peak
M: mol concentration
N: normal
CDCl₃: deuterochloroform
DMSO-d₆: deuterodimethyl sulfoxide
¹H NMR: proton nuclear magnetic resonance
LC/MS: liquid chromatograph mass spectrometer
ESI: Electron Spray Ionization
APCI: atmospheric pressure chemical ionization
THF: tetrahydrofuran
DME: 1,2-dimethoxyethane
DMF: N,N-dimethylformamide
DMA: N,N-dimethylacetamide
HATU: 2-(7-azabenzotriazol-1-yl)-1,1,3,3-tetramethyluronium hexafluorophosphate
HOBt: 1-hydroxybenzotriazole

¹H NMR was measured by Fourier-transform type NMR. For the analysis, ACD/SpecManager (trade name) and the like were used. Peaks with very mild protons such as a hydroxy group, an amino group and the like are not described.

MS was measured by LC/MS. As ionization method, ESI method or APCI method was used. The data indicates those actual measured value (found). Generally, molecular ion peaks are observed. For example, in the case of a compound having a tert-butoxycarbonyl group, a peak after elimination of a tert-butoxycarbonyl group or tert-butyl group may be observed as a fragment ion. In the case of a compound having a hydroxy group, a peak after elimination of H₂O may be observed as a fragment ion. In the case of a salt, a molecular ion peak or fragment ion peak of free form is generally observed.

### Example 19

### 7-methoxy-1-(quinolin-6-yl)-1,3-dihydro-2H-imidazo[4,5-b]pyridin-2-one

### A) 2-chloro-4-methoxypyridin-3-amine

To a mixture of 4-methoxypyridin-3-amine (2.0 g) and 12N hydrochloric acid (10 mL) was added 30% hydrogen peroxide water (2.375 g) at 0°C, and the mixture was stirred at room temperature overnight. The reaction mixture was diluted with ethyl acetate, neutralized with potassium carbonate, and extracted with ethyl acetate. The extract was washed with saturated aqueous sodium sulfite solution and saturated brine, and dried over anhydrous sodium sulfate. The solvent was evaporated under reduced pressure and the residue was purified by silica gel chromatography (hexane/ethyl acetate) to give the title compound (1.77 g).
¹H NMR (300 MHz, CDCl₃) δ 3.92 (3H, s), 4.06 (2H, brs), 6.68 (1H, d, J = 5.3 Hz), 7.76 (1H, d, J = 5.3 Hz).

### B) N-(2-chloro-4-methoxypyridin-3-yl)quinolin-6-amine

A mixture of 2-chloro-4-methoxypyridin-3-amine (100 mg), 6-bromoquinoline (0.128 mL),
tris(dibenzylideneacetone)dipalladium(0) (57.7 mg), 2-dicyclohexylphosphino-2',6'-diisopropoxy-1,1'-biphenyl (58.8 mg) and sodium tert-butoxide (121 mg) in toluene (3 mL) was stirred at 100°C for 1 hr in a microwave reactor. The reaction mixture was purified by basic silica gel chromatography (methanol/ethyl acetate) to give the title compound (122 mg).
¹H NMR (300 MHz, CDCl₃) δ 3.89 (3H, s), 5.81 (1H, s), 6.78 (1H, d, J = 2.6 Hz), 6.91 (1H, d, J = 5.7 Hz), 7.22-7.35 (2H, m), 7.91 (1H, d, J = 7.6 Hz), 7.98 (1H, d, J = 9.1 Hz), 8.20 (1H, d, J = 5.7 Hz), 8.72 (1H, dd, J = 4.3, 1.7 Hz).

### C) 1-(2-chloro-4-methoxypyridin-3-yl)-1-(quinolin-6-yl)urea

A solution of N-(2-chloro-4-methoxypyridin-3-yl)quinolin-6-amine (120 mg) in THF (1 mL) was added to a solution of chlorosulfonyl isocyanate (0.044 mL) in THF (0.5 mL) at 0°C, and the mixture was stirred at room temperature for 2 hr. Chlorosulfonyl isocyanate (0.044 mL) was further added at 0°C, and the mixture was stirred at room temperature for 2 hr. Water (3 mL) was added at room temperature, and the mixture was stirred at room temperature overnight. Saturated aqueous sodium hydrogen carbonate solution was added, and the mixture was extracted with ethyl acetate. The organic layer was washed with water and saturated brine, and dried over anhydrous sodium sulfate. The solvent was evaporated under reduced pressure and the residue was purified by silica gel chromatography (methanol/ethyl acetate) to give the title compound (107 mg).
¹H NMR (300 MHz, DMSO-d₆) δ 3.91 (3H, s), 6.36 (2H, s), 7.30 (1H, d, J = 5.7 Hz), 7.46 (1H, dd, J = 8.3, 4.2 Hz), 7.56-7.70 (2H, m), 7.93 (1H, d, J = 9.1 Hz), 8.24-8.35 (2H, m), 8.78-8.85 (1H, m).

### D) 7-methoxy-1-(quinolin-6-yl)-1,3-dihydro-2H-imidazo[4,5-b]pyridin-2-one

A mixture of 1-(2-chloro-4-methoxypyridin-3-yl)-1-(quinolin-6-yl)urea (100 mg), palladium acetate (6.83 mg), 2-dicyclohexylphosphino-2',4',6'-triisopropyl-1,1'-biphenyl (29 mg) and sodium hydrogen carbonate (77 mg) in 2-propanol (2 mL) was stirred at 90°C for 1 hr in a microwave reactor. Palladium acetate (6.83 mg) and 2-dicyclohexylphosphino-2',4',6'-triisopropyl-1,1'-biphenyl (29 mg) were further added, and the mixture was stirred at 90°C for 5 hr in a microwave reactor. The mixture was diluted with ethyl acetate, insoluble material was removed by celite filtration, and the filtrate was washed with saturated brine, and dried over sodium sulfate. The solvent was evaporated under reduced pressure, and the residue was purified by basic silica gel chromatography (methanol/ethyl acetate) to give the title compound (24.3 mg).
¹H NMR (300 MHz, DMSO-d₆) δ 3.69 (3H, s), 6.88 (1H, d, J = 6.0 Hz), 7.59 (1H, dd, J = 8.7, 4.2 Hz), 7.78 (1H, dd, J = 8.7, 2.3 Hz), 7.97 (1H, d, J = 6.0 Hz), 8.03-8.11 (2H, m), 8.43 (1H, d, J = 7.9 Hz), 8.95 (1H, dd, J = 4.2, 2.3 Hz), 11.81 (1H, brs).

### Example 33

### 1-(4-ethoxyquinolin-6-yl)-7-(tetrahydro-2H-pyran-4-yloxy)-1,3-dihydro-2H-imidazo[4,5-b]pyridin-2-one

### A) di-tert-butyl (4-chloro-3-nitropyridin-2-yl)imidodicarbonate

To a solution of 4-chloro-3-nitropyridin-2-amine (3.0 g) and di-tert-butyl dicarbonate (7.94 mL) in THF (57.6 mL) was added 4-dimethylaminopyridine (0.021 g) at room temperature, and the mixture was stirred at 50°C for 1 hr. Ethyl acetate was added, and the organic layer was washed with water and saturated brine, and dried over anhydrous sodium sulfate. The solvent was evaporated under reduced pressure to give the title compound (6.3 g).
¹H NMR (300 MHz, CDCl₃) δ 1.42 (18H, s), 7.52 (1H, d, J = 5.3 Hz), 8.56 (1H, d, J = 5.3 Hz).

### B) tert-butyl (3-nitro-4-((tetrahydro-2H-pyran-4-yl)oxy)pyridin-2-yl)carbamate

To a solution of tetrahydro-2H-pyran-4-ol (5.20 mL) in THF (91 mL) was added 60% sodium hydride (2.183 g) under a nitrogen atmosphere at 0°C, and the mixture was stirred at room temperature for 30 min. To this reaction mixture was added di-tert-butyl (4-chloro-3-nitropyridin-2-yl)imidodicarbonate (6.80 g) at 0°C, and the mixture was stirred at 50°C for 1 hr. To the reaction mixture was added ethyl acetate, and the mixture was washed with water and saturated brine, and dried over anhydrous sodium sulfate. The solvent was evaporated under reduced pressure, and the residue was purified by silica gel chromatography (hexane/ethyl acetate) to give the title compound (4.15 g).
¹H NMR (300 MHz, CDCl₃) δ 1.51 (9H, s), 1.79-1.93 (2H, m), 1.97-2.13 (2H, m), 3.58-3.73 (2H, m), 3.88-4.02 (2H, m), 4.71-4.82 (1H, m), 6.71 (1H, d, J = 5.7 Hz), 7.82 (1H, s), 8.36 (1H, d, J = 5.7 Hz).

### C) tert-butyl (3-nitro-4-((tetrahydro-2H-pyran-4-yl)oxy)pyridin-2-yl)((2-(trimethylsilyl)ethoxy)methyl)carbamate

To a solution of tert-butyl (3-nitro-4-((tetrahydro-2H-pyran-4-yl)oxy)pyridin-2-yl)carbamate (4.15 g) in DMF (82 mL) was added 60% sodium hydride (0.137 g) under a nitrogen atmosphere at 0°C, and the mixture was stirred at 0°C for 1 hr. To the mixture was added 2-(trimethylsilyl)ethoxymethyl chloride (2.60 mL), and the mixture was stirred at room temperature for 2 hr. To the reaction mixture was added ethyl acetate, and the mixture was washed with water and saturated brine, and dried over anhydrous sodium sulfate. The solvent was evaporated under reduced pressure, and the residue was purified by silica gel chromatography (hexane/ethyl acetate) to give the title compound (5.65 g).
¹H NMR (300 MHz, CDCl₃) δ 0.02 (9H, s), 0.93-1.02 (2H, m), 1.44 (9H, s), 1.78-1.93 (2H, m), 1.96-2.13 (2H, m), 3.57-3.79 (4H, m), 3.93 (2H, ddd, J = 11.6, 8.0, 3.4 Hz), 4.72-4.81 (1H, m), 5.27 (2H, s), 6.88 (1H, d, J = 5.7 Hz), 8.40 (1H, d, J = 5.7 Hz).

### D) tert-butyl (3-amino-4-((tetrahydro-2H-pyran-4-yl)oxy)pyridin-2-yl)((2-(trimethylsilyl)ethoxy)methyl)carbamate

To a solution of tert-butyl (3-nitro-4-((tetrahydro-2H-pyran-4-yl)oxy)pyridin-2-yl)((2-(trimethylsilyl)ethoxy)methyl)carbamate (5.65 g) in ethanol (120 mL) was added 10% palladium carbon (500 mg), and the mixture was stirred under a hydrogen atmosphere at room temperature for 4 hr. Insoluble material was removed by filtration, and the filtrate was concentrated under reduced pressure to give the title compound (5.30 g).
¹H NMR (300 MHz, CDCl₃) δ 0.01 (9H, s), 0.87-0.98 (2H, m), 1.46 (9H, brs), 1.76-1.91 (2H, m), 2.01-2.14 (2H, m), 3.55-3.68 (4H, m), 3.91-4.03 (2H, m), 4.06-4.11 (2H, m), 4.54-4.66 (1H, m), 5.18 (2H, brs), 6.65 (1H, d, J = 5.7 Hz), 7.81 (1H, d, J = 5.7 Hz).

### E) 7-((tetrahydro-2H-pyran-4-yl)oxy)-3-((2-(trimethylsilyl)ethoxy)methyl)-1H-imidazo[4,5-b]pyridin-2(3H)-one

To a solution of tert-butyl (3-amino-4-((tetrahydro-2H-pyran-4-yl)oxy)pyridin-2-yl)((2-(trimethylsilyl)ethoxy)methyl)carbamate (5.30 g) in DMF (60.3 mL) was added sodium methoxide (3.26 g), and the mixture was stirred at 50°C for 2 hr. To the reaction mixture were added ethyl acetate and saturated aqueous ammonium chloride solution, and the mixture was extracted with ethyl acetate. The extract was washed with saturated brine, and dried over anhydrous sodium sulfate. The solvent was evaporated under reduced pressure, and the residue was purified by basic silica gel chromatography (hexane/ethyl acetate) to give the title compound (3.25 g).
¹H NMR (300 MHz, CDCl₃) δ 0.02 (9H, s), 0.91-1.04 (2H, m), 1.77-1.92 (2H, m), 2.06-2.14 (2H, m), 3.56-3.77 (4H, m), 3.95-4.06 (2H, m), 4.64-4.74 (1H, m), 5.39 (2H, s), 6.63 (1H, d, J = 5.7 Hz), 7.99 (1H, d, J = 5.7 Hz), 8.13 (1H, brs).

### F) 1-(4-chloroquinolin-6-yl)-7-((tetrahydro-2H-pyran-4-yl)oxy)-3-((2-(trimethylsilyl)ethoxy)methyl)-1H-imidazo[4,5-b]pyridin-2(3H)-one

A mixture of 7-((tetrahydro-2H-pyran-4-yl)oxy)-3-((2-(trimethylsilyl)ethoxy)methyl)-1H-imidazo[4,5-b]pyridin-2(3H)-one (400 mg), 6-bromo-4-chloroquinoline (398 mg), copper(I) iodide (417 mg), 1,10-phenanthrolin (592 mg) and potassium carbonate (378 mg) in NMP (7.296 mL) was stirred at 200°C for 2 hr. To the reaction mixture was added ethyl acetate, and the mixture was filtered through celite, and the filtrate was washed with water and saturated brine, and dried over sodium sulfate. The solvent was evaporated under reduced pressure, and the residue was purified by silica gel chromatography (hexane/ethyl acetate) to give the title compound (295 mg).
¹H NMR (300 MHz, CDCl₃) δ 0.00 (9H, s), 0.98-1.07 (2H, m), 1.36-1.49 (2H, m), 1.76-1.89 (2H, m), 3.18-3.37 (4H, m), 3.75-3.85 (2H, m), 4.54-4.65 (1H, m), 5.51 (2H, s), 6.66 (1H, d, J = 6.0 Hz), 7.56 (1H, d, J = 4.5 Hz), 7.86 (1H, dd, J = 9.1, 2.3 Hz), 8.07 (1H, d, J = 6.0 Hz), 8.21 (1H, d, J = 9.1 Hz), 8.32 (1H, d, J = 2.3 Hz), 8.84 (1H, d, J = 4.5 Hz).

### G) 1-(4-chloroquinolin-6-yl)-7-((tetrahydro-2H-pyran-4-yl)oxy)-1H-imidazo[4,5-b]pyridin-2(3H)-one

To 1-(4-chloroquinolin-6-yl)-7-((tetrahydro-2H-pyran-4-yl)oxy)-3-((2-(trimethylsilyl)ethoxy)methyl)-1H-imidazo[4,5-b]pyridin-2(3H)-one (290 mg) was added trifluoroacetic acid (6 mL), and the mixture was stirred at room temperature for 30 min. The solvent was evaporated under reduced pressure, 8N ammonia-methanol solution (6 mL) was added to the residue and the mixture was stirred overnight. The solvent was evaporated under reduced pressure, and the residue was purified by silica gel chromatography (methanol/ethyl acetate) to give the title compound (220 mg).
¹H NMR (300 MHz, DMSO-d₆) δ 1.14-1.31 (2H, m), 1.68-1.81 (2H, m), 3.05-3.28 (4H, m), 4.66-4.79 (1H, m), 6.93 (1H, d, J = 6.0 Hz), 7.85 (1H, d, J = 4.5 Hz), 7.91-8.03 (2H, m), 8.16-8.27 (2H, m), 8.90 (1H, d, J = 4.5 Hz), 11.87 (1H, brs).

### H) 1-(4-ethoxyquinolin-6-yl)-7-(tetrahydro-2H-pyran-4-yloxy)-1,3-dihydro-2H-imidazo[4,5-b]pyridin-2-one

To a suspension of 1-(4-chloroquinolin-6-yl)-7-((tetrahydro-2H-pyran-4-yl)oxy)-1H-imidazo[4,5-b]pyridin-2(3H)-one (50 mg) in THF (2 mL) was added 20% sodium ethoxide ethanol solution (214 mg) under a nitrogen atmosphere, and the mixture was stirred at 50°C overnight. To the reaction mixture were added ethyl acetate and saturated aqueous ammonium chloride solution, and the mixture was extracted with ethyl acetate. The organic layer was separated, washed with saturated brine, and dried over anhydrous sodium sulfate. The solvent was evaporated under reduced pressure, and the residue was purified by silica gel chromatography (methanol/ethyl acetate) to give the title compound (18.9 mg).
¹H NMR (300 MHz, DMSO-d₆) δ 1.17-1.31 (2H, m), 1.47 (3H, t, J = 6.8 Hz), 1.67-1.81 (2H, m), 3.17-3.25 (4H, m), 4.33 (2H, q, J = 6.8 Hz), 4.66-4.75 (1H, m), 6.90 (1H, d, J = 5.7 Hz), 7.07 (1H, d, J = 5.7 Hz), 7.79 (1H, dd, J = 8.7, 2.3 Hz), 7.91 (1H, d, J = 5.7 Hz), 8.00 (1H, d, J = 8.7 Hz), 8.12 (1H, d, J = 2.3 Hz), 8.76 (1H, d, J = 5.7 Hz), 11.79 (1H, brs).

### Example 94

### 7-ethoxy-1-(8-fluoro-2,3-dihydro[1,4]dioxino[2,3-c]quinolin-9-yl)-1,3-dihydro-2H-imidazo[4,5-b]pyridin-2-one

### A) tert-butyl (4-chloro-3-nitropyridin-2-yl)carbamate

To a solution of 4-chloro-3-nitropyridin-2-amine (25.05 g) in THF (361 mL) was added 60% sodium hydride (13.85 g) under ice-cooling, and the mixture was stirred for 1 hr. Di-tert-butyl dicarbonate (33.5 mL) was added dropwise, and the mixture was stirred for 1.5 hr. A half amount of solvent was removed by concentration under reduced pressure, ethyl acetate was added, and the mixture was washed with water containing acetic acid (19.83 mL), water and saturated brine, and dried over anhydrous sodium sulfate. The solvent was evaporated under reduced pressure, and the residue was washed with IPE to give the title compound (36.4 g).
¹H NMR (300 MHz, CDCl₃) δ 1.49-1.54 (9H, m), 7.21-7.28 (1H, m), 7.72 (1H, brs), 8.41 (1H, dd, J = 5.3, 2.3 Hz).

### B) tert-butyl (4-ethoxy-3-nitropyridin-2-yl)carbamate

To a solution of tert-butyl (4-chloro-3-nitropyridin-2-yl)carbamate (15 g) and ethanol (50 mL) in THF (200 mL) was added 60% sodium hydride (6.58 g) under ice-cooling, and the mixture was stirred at 50°C for 4 hr. DMF (40 mL) was added and the mixture was stirred at the same temperature for 2.5 hr. The solvent was removed by concentration under reduced pressure, and water (250 mL) was added. The precipitate was collected by filtration, and washed with water and THF to give the title compound (14.38 g).
¹H NMR (300 MHz, DMSO-d₆) δ 1.32 (3H, t, J = 7.0 Hz), 1.40 (9H, s), 4.28 (2H, q, J = 6.8 Hz), 7.18 (1H, d, J = 6.0 Hz), 8.36 (1H, d, J = 6.0 Hz), 10.00 (1H, s).

### C) tert-butyl (3-amino-4-ethoxypyridin-2-yl)carbamate

To a solution of tert-butyl (4-ethoxy-3-nitropyridin-2-yl)carbamate (17.4 g) in ethanol (400 mL) and THF (250 mL) was added 5% palladium carbon (2.0 g), and the mixture was stirred under a hydrogen atmosphere at room temperature for 5 days. The insoluble material was removed by filtration. The solvent was evaporated under reduced pressure, and the residue was washed with IPE to give the title compound (11.6 g).
¹H NMR (300 MHz, CDCl₃) δ 1.46 (3H, t, J = 7.0 Hz), 1.51 (9H, s), 4.12 (2H, q, J = 7.2 Hz), 4.35 (2H, brs), 6.60 (1H, d, J = 5.3 Hz), 7.12 (1H, brs), 7.74 (1H, d, J = 5.3 Hz).

### D) 5-(((4-bromo-3-fluorophenyl)amino)methylene)-2,2-dimethyl-1,3-dioxane-4,6-dione

A mixture of meldrum's acid (16.46 g) and trimethoxymethane (160 mL) was stirred under an argon atmosphere at 100°C for 2 hr. After cooling to room temperature, 3-fluoro-4-bromoaniline (15.5 g) was added, and the mixture was stirred under an argon atmosphere at 100°C overnight and cooled to room temperature. The precipitate was collected by filtration while washing with IPE to give the title compound (24.1 g).
¹H NMR (300 MHz, CDCl₃) δ 1.76 (6H, s), 6.92-6.99 (1H, m), 7.06 (1H, dd, J = 9.1, 2.6 Hz), 7.62 (1H, dd, J = 8.5, 7.4 Hz), 8.57 (1H, d, J = 14.0 Hz), 11.21 (1H, d, J = 13.6 Hz).

### E) mixture of 6-bromo-5-fluoroquinolin-4-ol and 6-bromo-7-fluoroquinolin-4-ol

A mixture of 5-(((4-bromo-3-fluorophenyl)amino)methylene)-2,2-dimethyl-1,3-dioxane-4,6-dione (6.5 g) and Dowtherm A (60 mL) was stirred under an argon atmosphere at 200°C for 2 hr. After cooling to room temperature, the mixture was diluted with toluene. The precipitate was collected by filtration, and washed with toluene and IPE. This operation was repeated 4 times to give the title compound (15.4 g).

### F) mixture of 6-bromo-5-fluoro-3-iodoquinolin-4-ol and 6-bromo-7-fluoro-3-iodoquinolin-4-ol

To a solution of a mixture (8.00 g) of 6-bromo-5-fluoroquinolin-4-ol and 6-bromo-7-fluoroquinolin-4-ol in acetic acid (331 mL) was added N-iodosuccinimide (7.44 g) under a nitrogen atmosphere, and the mixture was stirred at 60°C for 2 hr. The solvent was evaporated under reduced pressure, and the residue was washed with saturated sodium hydrogen carbonate solution, distilled water and acetone to give the title compound (12 g).

### G) 6-bromo-4-chloro-7-fluoro-3-iodoquinoline

A mixture of a mixture (10.9 g) of 6-bromo-5-fluoro-3-iodoquinolin-4-ol and 6-bromo-7-fluoro-3-iodoquinolin-4-ol and phosphoryl chloride (92 mL) was stirred at 90°C overnight. The solvent was evaporated under reduced pressure, and saturated sodium hydrogen carbonate solution was added to the residue. The precipitate was collected by filtration, and purified by silica gel chromatography (hexane/ethyl acetate) to give the title compound (5.07 g).
¹H NMR (300 MHz, CDCl₃) δ 7.79 (1H, d, J = 8.7 Hz), 8.53 (1H, d, J = 7.2 Hz), 9.10 (1H, s).

### H) 9-bromo-8-fluoro-2,3-dihydro-[1,4]dioxino[2,3-c]quinoline

A mixture of 6-bromo-4-chloro-7-fluoro-3-iodoquinoline (2.30 g), ethylene glycol (11 mL), cesium carbonate (5.82 g) and copper(I) iodide (1.134 g) in DMA (23 mL) was stirred under an argon atmosphere at 80°C for 1 hr. To the reaction mixture was added saturated aqueous ammonium chloride solution, and the mixture was extracted with ethyl acetate. The organic layer was washed with saturated brine, and dried over anhydrous sodium sulfate. The solvent was evaporated under reduced pressure, and the residue was purified by silica gel chromatography (hexane/ethyl acetate) to give the title compound (628 mg).
¹H NMR (300 MHz, CDCl₃) δ 4.37-4.42 (2H, m), 4.50-4.55 (2H, m), 7.68 (1H, d, J = 9.8 Hz), 8.25 (1H, d, J = 7.6 Hz), 8.55 (1H, s).

### I) 7-ethoxy-1-(8-fluoro-2,3-dihydro[1,4]dioxino[2,3-c]quinolin-9-yl)-1,3-dihydro-2H-imidazo[4,5-b]pyridin-2-one

A mixture of tert-butyl (3-amino-4-ethoxypyridin-2-yl)carbamate (401 mg), 9-bromo-8-fluoro-2,3-dihydro-[1,4]dioxino[2,3-c]quinoline (300 mg),
tris(dibenzylideneacetone)dipalladium(0) (290 mg), 5-(di-tert-butylphosphino)-1',3',5'-triphenyl-1'H-1,4'-bipyrazole (321 mg) and sodium tert-butoxide (203 mg) in tert-amylalcohol (10.6 mL) was stirred under an argon atmosphere at 100°C for 3 hr. To the reaction mixture was added saturated aqueous ammonium chloride solution, and the mixture was extracted with ethyl acetate/2-propanol. The organic layer was washed with saturated brine, and dried over anhydrous sodium sulfate. The solvent was evaporated under reduced pressure, and the residue was purified by silica gel chromatography (methanol/ethyl acetate) and basic silica gel chromatography (methanol/ethyl acetate) to give the title compound (111 mg).
¹H NMR (300 MHz, DMSO-d₆) δ 0.90 (3H, t, J = 7.0 Hz), 3.99 (2H, q, J = 7.1 Hz), 4.41-4.48 (2H, m), 4.54-4.62 (2H, m), 6.81 (1H, d, J = 6.0 Hz), 7.83 (1H, d, J = 11.3 Hz), 7.92 (1H, d, J = 6.0 Hz), 8.11 (1H, d, J = 8.3 Hz), 8.62 (1H, s), 11.87 (1H, brs).

The Example compounds produced according to the above-mentioned method or a method analogous thereto are shown in the following Tables. In the Tables, MS shows measured values.

**Table 1**

| **Ex. No.** | **IUPAC NAME** | **Structure** | **Salt** | **MS** |
|---|---|---|---|---|
| 1 | 1-(1-methyl-1H-benzimidazol-6-yl)-7-(tetrahydro-2H-pyran-4-yloxy)-1,3-dihydro-2H-imidazo[4,5-b]pyridin-2-one | 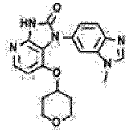 | | 366.1 |
| 2 | 7-methoxy-1-(1-methyl-1H-benzimidazol-6-yl)-1,3-dihydro-2H-imidazo[4,5-b]pyridin-2-one | 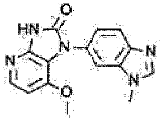 | | 296.1 |
| 3 | 1-(1-(cyclopropylmethyl)-1H-benzimidazol-6-yl)-7-methoxy-1,3-dihydro-2H-imidazo[4,5-b]pyridin-2-one | 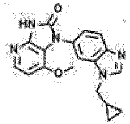 | | 336.1 |
| 4 | 7-ethoxy-1-(1-ethyl-2-methyl-1H-benzimidazol-6-yl)-1,3-dihydro-2H-imidazo[4,5-b]pyridin-2-one | 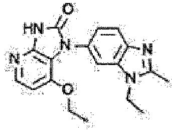 | | 338.1 |
| 5 | 1-(1-ethyl-1H-benzimidazol-6-yl)-7-methoxy-1,3-dihydro-2H-imidazo[4,5-b]pyridin-2-one | 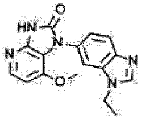 | | 310.1 |
| 6 | 7-ethoxy-1-(1-ethyl-1H-benzimidazol-6-yl)-1,3-dihydro-2H-imidazo[4,5-b]pyridin-2-one | 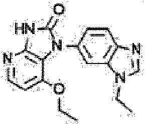 | | 324.2 |
| 7 | 7-ethoxy-1-(1-methyl-1H-benzimidazol-6-yl)-1,3-dihydro-2H-imidazo[4,5-b]pyridin-2-one | 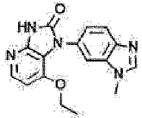 | | 310.1 |
| 8 | 7-ethoxy-1-(1-isopropyl-1H-benzimidazol-6-yl)-1,3-dihydro-2H-imidazo[4,5-b]pyridin-2-one | 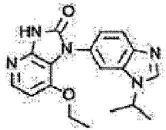 | | 337.8 |
| 9 | 7-(2,2-difluoroethoxy)-1-(1-ethyl-1H-benzimidazol-6-yl)-1,3-dihydro-2H-imidazo[4,5-b]pyridin-2-one | 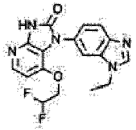 | | 360.1 |
| 10 | 7-ethoxy-1-(1-(2,2,2-trifluoroethyl)-1H-benzimidazol-6-yl)-1,3-dihydro-2H-imidazo[4,5-b]pyridin-2-one | 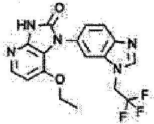 | | 378.1 |

**Table 2**

| **Ex. No.** | **IUPAC NAME** | **Structure** | **Salt** | **MS** |
|---|---|---|---|---|
| 11 | 7-ethoxy-1-(3-ethylimidazo[1,2-a]pyridin-6-yl)-1,3-dihydro-2H-imidazo[4,5-b]pyridin-2-one | 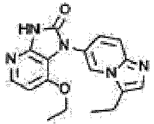 | | 324.1 |
| 12 | 7-ethoxy-1-(3-ethylimidazo[1,2-b]pyridazin-6-yl)-1,3-dihydro-2H-imidazo[4,5-b]pyridin-2-one | 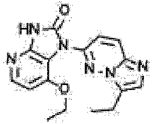 | | 325.2 |
| 13 | 7-ethoxy-3'-methyl-3'H-1,5'-biimidazo[4,5-b]pyridin-2(3H)-one | 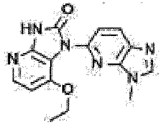 | | 311.1 |
| 14 | 6-ethoxy-7-(3-methyl-3H-imidazo[4,5-b]pyridin-5-yl)-7,9-dihydro-8H-purin-8-one | 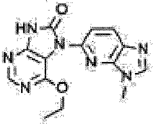 | | 312.1 |
| 15 | 7-ethoxy-1-(3-methyl-1-benzofuran-5-yl)-1,3-dihydro-2H-imidazo[4,5-b]pyridin-2-one | 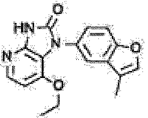 | | 310.1 |
| 16 | 6-ethoxy-7-(1-ethyl-1H-benzimidazol-6-yl)-7,9-dihydro-8H-purin-8-one | 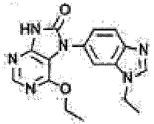 | | 325.1 |
| 17 | 7-ethoxy-1-(3-ethylpyrazolo[1,5-a]pyridin-5-yl)-1,3-dihydro-2H-imidazo[4,5-b]pyridin-2-one | 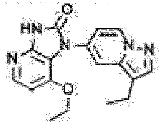 | | 324.2 |
| 18 | 7-ethoxy-1-(3-methyl-1H-indazol-5-yl)-1,3-dihydro-2H-imidazo[4,5-b]pyridin-2-one | 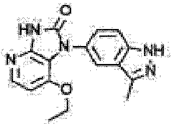 | | 310.1 |
| 19 | 7-methoxy-1-(quinolin-6-yl)-1,3-dihydro-2H-imidazo[4,5-b]pyridin-2-one | 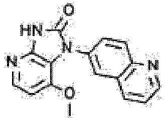 | | 293.1 |
| 20 | 7-methoxy-1-(3-methylquinolin-6-yl)-1,3-dihydro-2H-imidazo[4,5-b]pyridin-2-one | 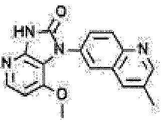 | | 307.1 |

**Table 3**

| **Ex. No.** | **IUPAC NAME** | **Structure** | **Salt** | **MS** |
|---|---|---|---|---|
| 21 | 7-methoxy-1-(2-methylquinolin-6-yl)-1,3-dihydro-2H-imidazo[4,5-b]pyridin-2-one | 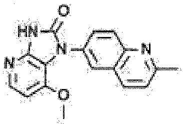 | | 307.1 |
| 22 | 7-methoxy-1-(4-methoxyquinolin-6-yl)-1,3-dihydro-2H-imidazo[4,5-b]pyridin-2-one | 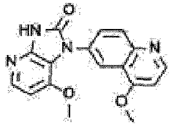 | | 323.1 |
| 23 | 1-(4-methoxyquinolin-6-yl)-7-(tetrahydro-2H-pyran-4-yloxy)-1,3-dihydro-2H-imidazo[4,5-b]pyridin-2-one | 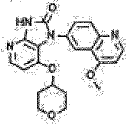 | | 393.2 |
| 24 | 1-(4-methoxyquinolin-6-yl)-7-((1-methylpiperidin-4-yl)oxy)-1,3-dihydro-2H-imidazo[4,5-b]pyridin-2-one | 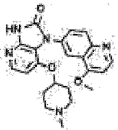 | | 406.2 |
| 25 | 1-(4-chloroquinolin-6-yl)-7-ethoxy-1,3-dihydro-2H-imidazo[4,5-b]pyridin-2-one | 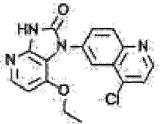 | | 341.0 |
| 26 | 7-(2-methoxyethoxy)-1-(4-methoxyquinolin-6-yl)-1,3-dihydro-2H-imidazo[4,5-b]pyridin-2-one | 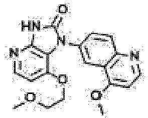 | | 367.0 |
| 27 | 6-(7-ethoxy-2-oxo-2,3-dihydro-1H-imidazo[4,5-b]pyridin-1-yl)quinoline-4-carboxamide | 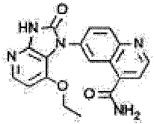 | | 350.1 |
| 28 | 6-(7-ethoxy-2-oxo-2,3-dihydro-1H-imidazo[4,5-b]pyridin-1-yl)quinoline-4-carbonitrile | 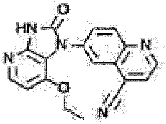 | | 332.1 |
| 29 | 1-(4-methoxyquinolin-6-yl)-7-methyl-1,3-dihydro-2H-imidazo[4,5-b]pyridin-2-one | 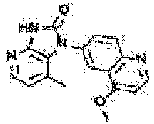 | | 307.1 |
| 30 | 7-cyclopropyl-1-(4-methoxyquinolin-6-yl)-1,3-dihydro-2H-imidazo[4,5-b]pyridin-2-one | 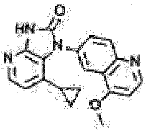 | | 333.1 |

**[Table 4]**

| **Ex. No.** | **IUPAC NAME** | **Structure** | **Salt** | **MS** |
|---|---|---|---|---|
| 31 | 1-(4-methoxyquinolin-6-yl)-7-propoxy-1,3-dihydro-2H-imidazo[4,5-b]pyridin-2-one | 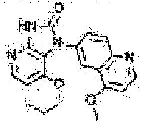 | | 351.1 |
| 32 | 1-(4-methoxyquinolin-6-yl)-7-(2,2,2-trifluoroethoxy)-1,3-dihydro-2H-imidazo[4,5-b]pyridin-2-one | 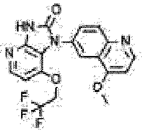 | | 391.0 |
| 33 | 1-(4-ethoxyquinolin-6-yl)-7-(tetrahydro-2H-pyran-4-yloxy)-1,3-dihydro-2H-imidazo[4,5-b]pyridin-2-one | 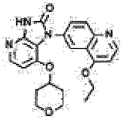 | | 407.1 |
| 34 | 1-(4-chloroquinolin-6-yl)-7-(2,2,2-trifluoroethoxy)-1,3-dihydro-2H-imidazo[4,5-b]pyridin-2-one | 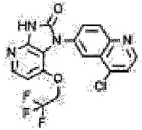 | | 395.1 |
| 35 | 1-(4-isopropoxyquinolin-6-yl)-7-(tetrahydro-2H-pyran-4-yloxy)-1,3-dihydro-2H-imidazo[4,5-b]pyridin-2-one | 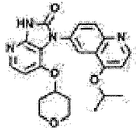 | | 421.2 |
| 36 | 1-(4-(dimethylamino)quinolin-6-yl)-7-(tetrahydro-2H-pyran-4-yloxy)-1,3-dihydro-2H-imidazo[4,5-b]pyridin-2-one | 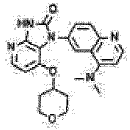 | | 406.2 |
| 37 | 1-(4-(methylamino)quinolin-6-yl)-7-(tetrahydro-2H-pyran-4-yloxy)-1,3-dihydro-2H-imidazo[4,5-b]pyridin-2-one | 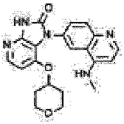 | | 392.1 |
| 38 | 1-(4-chloroquinolin-6-yl)-7-(tetrahydro-2H-pyran-4-yloxy)-1,3-dihydro-2H-imidazo[4,5-b]pyridin-2-one | 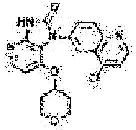 | | 397.1 |
| 39 | 7-ethoxy-1-(4-methoxyquinazolin-6-yl)-1,3-dihydro-2H-imidazo[4,5-b]pyridin-2-one | 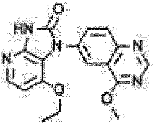 | | 338.0 |
| 40 | 7-ethoxy-1-(3-methoxyquinolin-6-yl)-1,3-dihydro-2H-imidazo[4,5-b]pyridin-2-one | 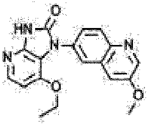 | | 337.0 |

**[Table 5]**

| **Ex. No.** | **IUPAC NAME** | **Structure** | **Salt** | **MS** |
|---|---|---|---|---|
| 41 | 7-ethoxy-1-(4-methoxyquinolin-6-yl)-1,3-dihydro-2H-imidazo[4,5-b]pyridin-2-one | 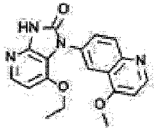 | | 337.1 |
| 42 | 1-(quinolin-6-yl)-7-(tetrahydro-2H-pyran-4-yloxy)-1,3-dihydro-2H-imidazo[4,5-b]pyridin-2-one | 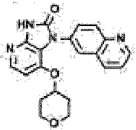 | | 363.2 |
| 43 | 1-(4-methylquinolin-6-yl)-7-(tetrahydro-2H-pyran-4-yloxy)-1,3-dihydro-2H-imidazo[4,5-b]pyridin-2-one | 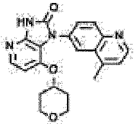 | | 377.1 |
| 44 | 7-butoxy-1-(4-methoxyquinolin-6-yl)-1,3-dihydro-2H-imidazo[4,5-b]pyridin-2-one | 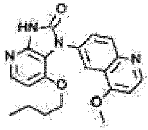 | | 365.1 |
| 45 | 7-isopropoxy-1-(4-methoxyquinolin-6-yl)-1,3-dihydro-2H-imidazo[4,5-b]pyridin-2-one | 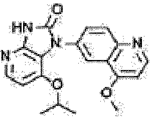 | | 351.1 |
| 46 | 7-(2,2-difluoroethoxy)-1-(4-methoxyquinolin-6-yl)-1,3-dihydro-2H-imidazo[4,5-b]pyridin-2-one | 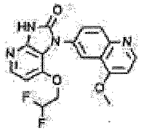 | | 371.0 |
| 47 | 7-ethoxy-1-(4-(2-methoxyethoxy)quinolin-6-yl)-1,3-dihydro-2H-imidazo[4,5-b]pyridin-2-one | 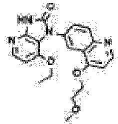 | | 381.1 |
| 48 | 7-ethoxy-1-(4-(2,2,2-trifluoroethoxy)quinolin-6-yl)-1,3-dihydro-2H-imidazo[4,5-b]pyridin-2-one | 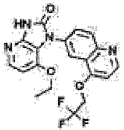 | | 405.1 |
| 49 | 7-ethyl-1-(4-methoxyquinolin-6-yl)-1,3-dihydro-2H-imidazo[4,5-b]pyridin-2-one | 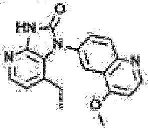 | | 321.1 |
| 50 | 6-bromo-7-ethoxy-1-(4-methoxyquinolin-6-yl)-1,3-dihydro-2H-imidazo[4,5-b]pyridin-2-one | 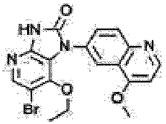 | | 415.0 |

**[Table 6]**

| **Ex. No.** | **IUPAC NAME** | **Structure** | **Salt** | **MS** |
|---|---|---|---|---|
| 51 | 6-ethoxy-7-(4-(2,2,2-trifluoroethoxy)quinolin-6-yl)-7,9-dihydro-8H-purin-8-one | 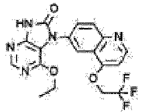 | | 406.0 |
| 52 | 7-(2,2-difluoroethoxy)-1-(3-methoxyquinolin-6-yl)-1,3-dihydro-2H-imidazo[4,5-b]pyridin-2-one | 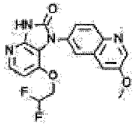 | | 373.1 |
| 53 | 1-(4-methoxyquinolin-6-yl)-7-phenoxy-1,3-dihydro-2H-imidazo[4,5-b]pyridin-2-one | 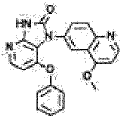 | | 385.0 |
| 54 | 1-(4-methoxyquinolin-6-yl)-7-propyl-1,3-dihydro-2H-imidazo[4,5-b]pyridin-2-one | 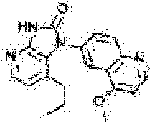 | | 335.1 |
| 55 | 7-ethoxy-1-(8-methoxy-1,5-naphthyridin-2-yl)-1,3-dihydro-2H-imidazo[4,5-b]pyridin-2-one | 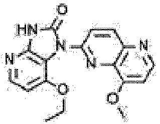 | | 338.1 |
| 56 | 7-(2,3-dihydro[1,4]dioxino[2,3-c]quinolin-9-yl)-6-ethoxy-7,9-dihydro-8H-purin-8-one | 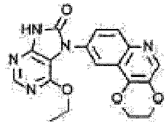 | | 366.1 |
| 57 | 7-((²H₅)ethyloxy)-1-(4-methoxyquinolin-6-yl)-1,3-dihydro-2H-imidazo[4,5-b]pyridin-2-one | 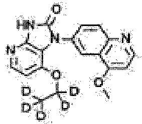 | | 342.2 |
| 58 | 1-(2,3-dihydro[1,4]dioxino[2,3-c]quinolin-9-yl)-7-((²H₅)ethyloxy)-1,3-dihydro-2H-imidazo[4,5-b]pyridin-2-one | 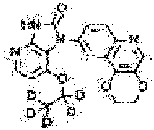 | | 370.0 |
| 59 | 7-((²H₅)ethyloxy) -1- (4-((²H₃)methyloxy)quinolin-6-yl)-1,3-dihydro-2H-imidazo[4,5-b]pyridin-2-one | 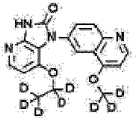 | | 345.1 |
| 60 | 7-ethoxy-1-(4-methoxycinnolin-6-yl)-1,3-dihydro-2H-imidazo[4,5-b]pyridin-2-one | 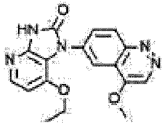 | | 338.1 |

**[Table 7]**

| **Ex. No.** | **IUPAC NAME** | **Structure** | **Salt** | **MS** |
|---|---|---|---|---|
| 61 | 1-(3-chloro-4-methoxyquinolin-6-yl)-7-ethoxy-1,3-dihydro-2H-imidazo[4,5-b]pyridin-2-one | 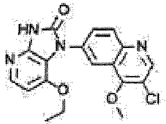 | | 371.1 |
| 62 | 7-((trans-4-(dimethylamino)-cyclohexyl)oxy)-1-(4-methoxyquinolin-6-yl)-1,3-dihydro-2H-imidazo[4,5-b]pyridin-2-one | 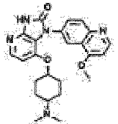 | | 434.2 |
| 63 | 7-ethoxy-1-(isoquinolin-6-yl)-1,3-dihydro-2H-imidazo[4,5-b]pyridin-2-one | 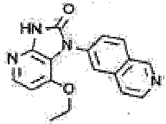 | | 307.1 |
| 64 | 1-(4-methoxyquinolin-6-yl)-7-(tetrahydro-2H-pyran-4-yl)-1,3-dihydro-2H-imidazo[4,5-b]pyridin-2-one | 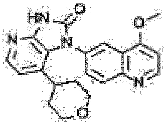 | | 377.1 |
| 65 | 1-(4-ethoxyquinolin-6-yl)-7-methoxy-1,3-dihydro-2H-imidazo[4,5-b]pyridin-2-one | 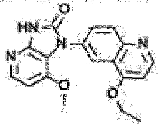 | | 337.1 |
| 66 | 7-methoxy-1-(4-(2,2,2-trifluoroethoxy)quinolin-6-yl)-1,3-dihydro-2H-imidazo[4,5-b]pyridin-2-one | 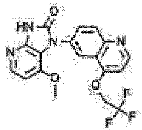 | | 391.0 |
| 67 | 7-methoxy-1-(4-(2-methoxyethoxy)quinolin-6-yl)-1,3-dihydro-2H-imidazo[4,5-b]pyridin-2-one | 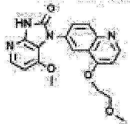 | | 367.2 |
| 68 | 7-ethoxy-1-(3-(2-methoxyethoxy)-quinolin-6-yl)-1,3-dihydro-2H-imidazo[4,5-b]pyridin-2-one | 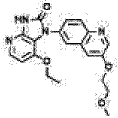 | | 381.1 |
| 69 | 1-(4-(difluoromethoxy)quinolin-6-yl)-7-ethoxy-1,3-dihydro-2H-imidazo[4,5-b]pyridin-2-one | 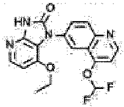 | | 373.0 |
| 70 | 7-ethoxy-1-(4-vinylquinolin-6-yl)-1,3-dihydro-2H-imidazo[4,5-b]pyridin-2-one | 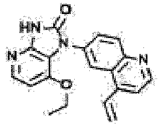 | | 333.1 |

**[Table 8]**

| **Ex. No.** | **IUPAC NAME** | **Structure** | **Salt** | **MS** |
|---|---|---|---|---|
| 71 | 1-(2-chloroquinolin-6-yl)-7-ethoxy-1,3-dihydro-2H-imidazo[4,5-b]pyridin-2-one | 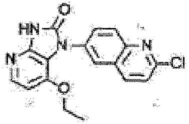 | | 341.0 |
| 72 | 7-ethoxy-1-(2-hydroxyquinolin-6-yl)-1,3-dihydro-2H-imidazo[4,5-b]pyridin-2-one | 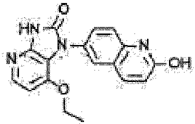 | | 323.1 |
| 73 | 1-(4-(aminomethyl)quinolin-6-yl)-7-ethoxy-1,3-dihydro-2H-imidazo[4,5-b]pyridin-2-one | 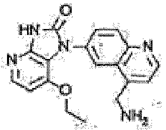 | CF₃COOH | 336.1 |
| 74 | 7-ethoxy-1-(4-((²H₃)methyloxy)quinolin-6-yl)-1,3-dihydro-2H-imidazo[4,5-b]pyridin-2-one | 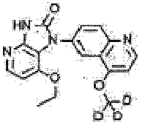 | | 340.1 |
| 75 | 1-(2,3-dihydro[1,4]-dioxino[2,3-c]quinolin-9-yl)-7-ethoxy-1,3-dihydro-2H-imidazo[4,5-b]pyridin-2-one | 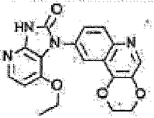 | | 365.0 |
| 76 | 7-(cyclopropylmethoxy)-1-(4-methoxyquinolin-6-yl)-1,3-dihydro-2H-imidazo[4,5-b]pyridin-2-one | 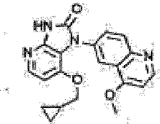 | | 363.1 |
| 77 | 7-ethoxy-1-(3-(2,2,2-trifluoroethoxy)quinolin-6-yl)-1,3-dihydro-2H-imidazo[4,5-b]pyridin-2-one | 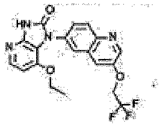 | | 405.0 |
| 78 | 7-(cyclopropylmethoxy)-1-(3-methoxyquinolin-6-yl)-1,3-dihydro-2H-imidazo[4,5-b]pyridin-2-one | 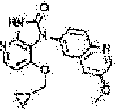 | | 363.1 |
| 79 | 7-ethoxy-1-(4-hydroxyquinolin-6-yl)-1,3-dihydro-2H-imidazo[4,5-b]pyridin-2-one | 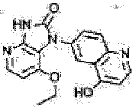 | | 323.1 |
| 80 | 7-ethoxy-1-(quinolin-6-yl)-1,3-dihydro-2H-imidazo[4,5-b]pyridin-2-one | 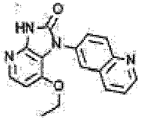 | | 307.1 |

**[Table 9]**

| **Ex. No.** | **IUPAC NAME** | **Structure** | **Salt** | **MS** |
|---|---|---|---|---|
| 81 | 7-ethoxy-1-(2-methylquinolin-6-yl)-1,3-dihydro-2H-imidazo[4,5-b]pyridin-2-one | 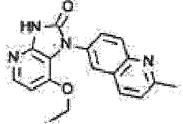 | | 321.1 |
| 82 | 7-ethoxy-1-(3-methylquinolin-6-yl)-1,3-dihydro-2H-imidazo[4,5-b]pyridin-2-one | 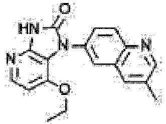 | | 321.1 |
| 83 | 7-ethoxy-1-(4-methylquinolin-6-yl)-1,3-dihydro-2H-imidazo[4,5-b]pyridin-2-one | 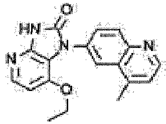 | | 321.1 |
| 84 | 1-(4-methoxyquinolin-6-yl)-7-phenyl-1,3-dihydro-2H-imidazo[4,5-b]pyridin-2-one | 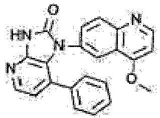 | | 369.0 |
| 85 | 1-(4-chloroquinolin-6-yl)-7-methoxy-1,3-dihydro-2H-imidazo[4,5-b]pyridin-2-one | 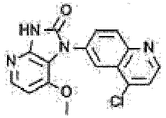 | | 326.9 |
| 86 | 1-(4-(cyclopropyloxy)quinolin-6-yl)-7-ethoxy-1,3-dihydro-2H-imidazo[4,5-b]pyridin-2-one | 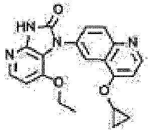 | | 363.1 |
| 87 | 6-ethoxy-7-(4-methoxyquinolin-6-yl)-7,9-dihydro-8H-purin-8-one | 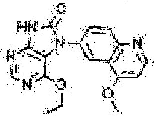 | | 338.0 |
| 88 | 7-ethoxy-1-(8-fluoro-4-methoxyquinolin-6-yl)-1,3-dihydro-2H-imidazo[4,5-b]pyridin-2-one | 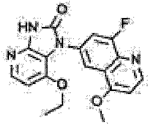 | | 355.1 |
| 89 | 6-ethoxy-7-(8-fluoro-4-methoxyquinolin-6-yl)-7,9-dihydro-8H-purin-8-one | 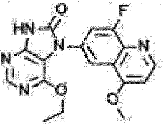 | | 356.1 |
| 90 | 6-ethoxy-7-(3-methoxyquinolin-6-yl)-7,9-dihydro-8H-purin-8-one | 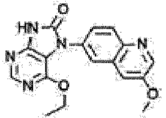 | | 338.1 |

**[Table 10]**

| **Ex. No.** | **IUPAC NAME** | **Structure** | **Salt** | **MS** |
|---|---|---|---|---|
| 91 | tert-butyl 4-(6-(7-ethoxy-2-oxo-2,3-dihydro-1H-imidazo[4,5-b]pyridin-1-yl)quinolin-4-yl)piperazine-1-carboxylate | 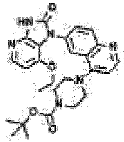 | | 491.2 |
| 92 | 7-ethoxy-1-(4-(piperazin-1-yl)quinolin-6-yl)-1,3-dihydro-2H-imidazo[4,5-b]pyridin-2-one | 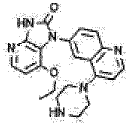 | HCl | 391.2 |
| 93 | 7-ethoxy-1-(3-ethoxyquinoxalin-6-yl)-1,3-dihydro-2H-imidazo[4,5-b]pyridin-2-one | 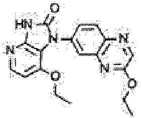 | | 352.1 |
| 94 | 7-ethoxy-1-(8-fluoro-2,3-dihydro[1,4]dioxino[2,3-c]quinolin-9-yl)-1,3-dihydro-2H-imidazo[4,5-b]pyridin-2-one | 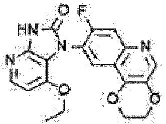 | | 383.0 |
| 95 | 1-(4-(dimethylamino)quinolin-6-yl)-7-ethoxy-1,3-dihydro-2H-imidazo[4,5-b]pyridin-2-one | 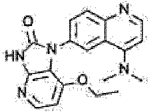 | | 350.1 |
| 96 | 7-ethoxy-1-(6-fluorofuro[3,2-c]quinolin-8-yl)-1,3-dihydro-2H-imidazo[4,5-b]pyridin-2-one | 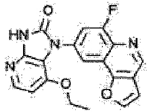 | | 365.0 |
| 97 | 7-ethoxy-1-(4-methyl-3,4-dihydro-2H-[1,4]oxazino[3,2-c]quinolin-9-yl)-1,3-dihydro-2H-imidazo[4,5-b]pyridin-2-one | 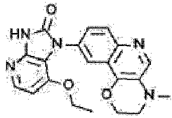 | | 378.1 |
| 98 | 6-ethoxy-7-(7-fluoro-4-methoxyquinolin-6-yl)-7,9-dihydro-8H-purin-8-one | 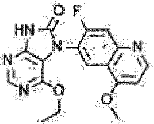 | | 356.1 |
| 99 | 7-ethoxy-1-(7-fluoro-4-methoxyquinolin-6-yl)-1,3-dihydro-2H-imidazo[4,5-b]pyridin-2-one | 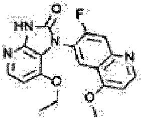 | | 355.1 |
| 100 | 7-ethoxy-1-(5-fluoro-4-methoxyquinolin-6-yl)-1,3-dihydro-2H-imidazo[4,5-b]pyridin-2-one | 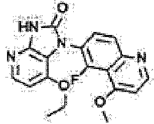 | | 355.1 |

**[Table 11]**

| **Ex. No.** | **IUPAC NAME** | **Structure** | **Salt** | **MS** |
|---|---|---|---|---|
| 101 | 6-ethoxy-7-(5-fluoro-4-methoxyquinolin-6-yl)-7,9-dihydro-8H-purin-8-one | 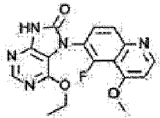 | | 356.1 |
| 102 | 7-ethoxy-1-(furo[2,3-c]quinolin-8-yl)-1,3-dihydro-2H-imidazo[4,5-b]pyridin-2-one | 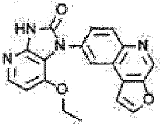 | | 347.1 |
| 103 | 1-(4-(1,4-diazepan-1-yl)quinolin-6-yl)-7-ethoxy-1,3-dihydro-2H-imidazo[4,5-b]pyridin-2-one | 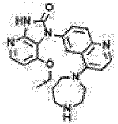 | HCl | 405.2 |
| 104 | 7-ethoxy-1-(9-fluorofuro[3,2-c]quinolin-8-yl)-1,3-dihydro-2H-imidazo[4,5-b]pyridin-2-one | 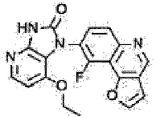 | | 365.0 |
| 105 | 7-ethoxy-1-(1,2,3,4-tetrahydrobenzo[c][2,7]-naphthyridin-9-yl)-1,3-dihydro-2H-imidazo[4,5-b]pyridin-2-one | 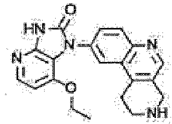 | | 362.1 |
| 106 | 1-(4-acetyl-3,4-dihydro-2H-[1,4]oxazino[3,2-c]quinolin-9-yl)-7-ethoxy-1,3-dihydro-2H-imidazo[4,5-b]pyridin-2-one | 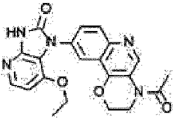 | | 406.1 |
| 107 | 1-(2,3-dihydro-1H-[1,4]-oxazino[2,3-c]quinolin-9-yl)-7-ethoxy-1,3-dihydro-2H-imidazo[4,5-b]pyridin-2-one | 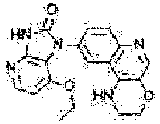 | | 364.1 |
| 108 | 7-ethoxy-1-(2-methylfuro[3,2-c]quinolin-8-yl)-1,3-dihydro-2H-imidazo[4,5-b]pyridin-2-one | 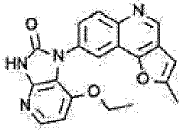 | | 361.0 |
| 109 | 1-(7-chloro-4-methoxyquinolin-6-yl)-7-ethoxy-1,3-dihydro-2H-imidazo[4,5-b]pyridin-2-one | 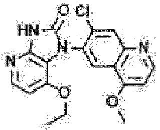 | | 371.0 |
| 110 | 1-(2,3-dihydro[1,4]-oxathiino[3,2-c]quinolin-9-yl)-7-ethoxy-1,3-dihydro-2H-imidazo[4,5-b]pyridin-2-one | 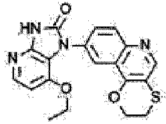 | | 381.0 |

**[Table 12]**

| **Ex. No.** | **IUPAC NAME** | **Structure** | **Salt** | **MS** |
|---|---|---|---|---|
| 111 | 1-(4-chloroquinolin-6-yl)-7-(4-hydroxybutoxy)-1,3-dihydro-2H-imidazo[4,5-b]pyridin-2-one | 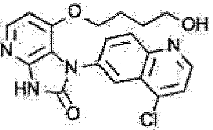 | | 385.0 |
| 112 | 7-ethoxy-1-(3-ethoxyquinolin-6-yl)-1,3-dihydro-2H-imidazo[4,5-b]pyridin-2-one | 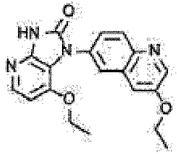 | | 351.1 |
| 113 | 1-(4-chloroquinolin-6-yl)-7-(2-hydroxyethoxy)-1,3-dihydro-2H-imidazo[4,5-b]pyridin-2-one | 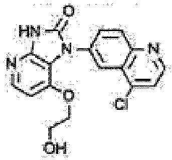 | | 357.0 |
| 114 | 7-ethoxy-1-(4-ethoxyquinolin-6-yl)-1,3-dihydro-2H-imidazo[4,5-b]pyridin-2-one | 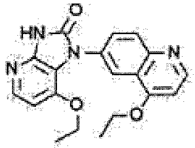 | | 349.0 |
| 115 | 1-(3-acetyl-1,2,3,4-tetrahydrobenzo[c][2,7]-naphthyridin-9-yl)-7-ethoxy-1,3-dihydro-2H-imidazo[4,5-b]pyridin-2-one | 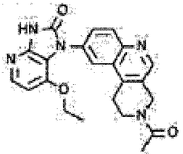 | | 402.0 |
| 116 | 1-(2,3-dihydro[1,4]oxathiino[2,3-c]quinolin-9-yl)-7-ethoxy-1,3-dihydro-2H-imidazo[4,5-b]pyridin-2-one | 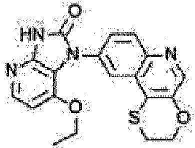 | | 381.1 |
| 119 | 7-(2-hydroxyethoxy)-1-(4-methoxyquinolin-6-yl)-1,3-dihydro-2H-imidazo[4,5-b]pyridin-2-one | 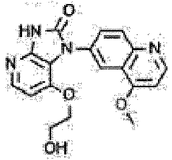 | | 350.9 |
| 120 | 1-(1,1-dioxido-2,3-dihydro[1,4]oxathiino[2,3-c]quinolin-9-yl)-7-ethoxy-1,3-dihydro-2H-imidazo[4,5-b]pyridin-2-one | 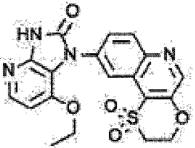 | | 413.1 |

**[Table 13]**

| **Ex. No.** | **IUPAC NAME** | **Structure** | **Salt** | **MS** |
|---|---|---|---|---|
| 121 | 1-(7,8-difluoro-2,3-dihydro[1,4]dioxino[2,3-c]quinolin-9-yl)-7-ethoxy-1,3-dihydro-2H-imidazo[4,5-b]pyridin-2-one | 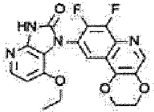 | | 399.0 |
| 122 | 1-(1,2-dihydrofuro[2,3-c]quinolin-8-yl)-7-ethoxy-1,3-dihydro-2H-imidazo[4,5-b]pyridin-2-one | 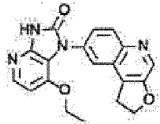 | | 349.1 |
| 123 | 7-ethoxy-1-(4-(methylsulfonyl)-3,4-dihydro-2H-[1,4]oxazino[3,2-c]quinolin-9-yl)-1,3-dihydro-2H-imidazo[4,5-b]pyridin-2-one | 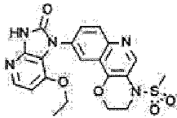 | | 442.0 |
| 124 | 7-ethoxy-1-(1-ethyl-2,3-dihydro-1H-[1,4]oxazino[2,3-c]quinolin-9-yl)-1,3-dihydro-2H-imidazo[4,5-b]pyridin-2-one | 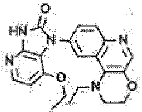 | | 392.1 |
| 125 | 7-ethoxy-1-(1-methyl-2,3-dihydro-1H-[1,4]oxazino[2,3-c]quinolin-9-yl)-1,3-dihydro-2H-imidazo[4,5-b]pyridin-2-one | 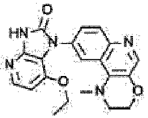 | | 376.0 |
| 126 | 7-ethoxy-1-(thieno[2,3-c]quinolin-8-yl)-1,3-dihydro-2H-imidazo[4,5-b]pyridin-2-one | 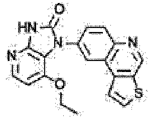 | | 363.1 |
| 127 | 7-ethoxy-1-(4-(methylamino)-quinolin-6-yl)-1,3-dihydro-2H-imidazo[4,5-b]pyridin-2-one | 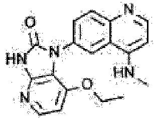 | | 336.2 |
| 128 | 7-ethoxy-1-(4-(piperazin-1-yl)cinnolin-6-yl)-1,3-dihydro-2H-imidazo[4,5-b]pyridin-2-one | 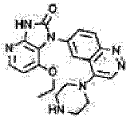 | HCl | 392.2 |
| 129 | 7-ethoxy-1-(7-fluoro-2,3-dihydro[1,4]dioxino[2,3-c]quinolin-9-yl)-1,3-dihydro-2H-imidazo[4,5-b]pyridin-2-one | 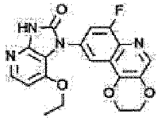 | | 383.1 |
| 130 | 1-(2,3-dihydrofuro[3,2-c]quinolin-8-yl)-7-ethoxy-1,3-dihydro-2H-imidazo[4,5-b]pyridin-2-one | 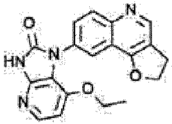 | | 349.1 |

**[Table 14]**

| **Ex. No.** | **IUPAC NAME** | **Structure** | **Salt** | **MS** |
|---|---|---|---|---|
| 131 | 7-ethoxy-1-(4-(morpholin-4-yl)quinolin-6-yl)-1,3-dihydro-2H-imidazo[4,5-b]pyridin-2-one | 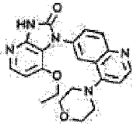 | | 392.1 |
| 132 | 7-ethoxy-1-(2-(hydroxymethyl)furo[3,2-c]quinolin-8-yl)-1,3-dihydro-2H-imidazo[4,5-b]pyridin-2-one | 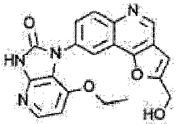 | | 377.1 |
| 133 | 1-(3,4-dihydro-2H-[1,4]-oxazino[3,2-c]quinolin-9-yl)-7-ethoxy-1,3-dihydro-2H-imidazo[4,5-b]pyridin-2-one | 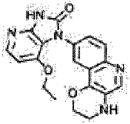 | | 364.1 |
| 134 | 6-ethoxy-7-(4-(piperazin-1-yl)quinolin-6-yl)-7,9-dihydro-8H-purin-8-one | 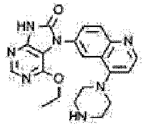 | HCl | 392.1 |
| 135 | 1-(4-cyclopropylquinolin-6-yl)-7-ethoxy-1,3-dihydro-2H-imidazo[4,5-b]pyridin-2-one | 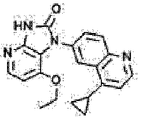 | | 347.2 |
| 136 | 1- (8-chloro-4-methoxyquinolin-6-yl)-7-ethoxy-1,3-dihydro-2H-imidazo[4,5-b]pyridin-2-one | 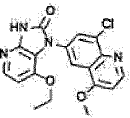 | | 371.0 |
| 137 | 7-ethoxy-1-(4-methoxy-8-methylquinolin-6-yl)-1,3-dihydro-2H-imidazo[4,5-b]pyridin-2-one | 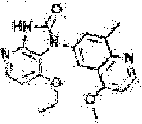 | | 351.1 |
| 138 | 7-ethoxy-1-(4-methoxy-3-methylquinolin-6-yl)-1,3-dihydro-2H-imidazo[4,5-b]pyridin-2-one | 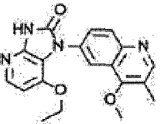 | | 351.0 |
| 139 | 7-(8-chloro-4-methoxyquinolin-6-yl)-6-ethoxy-7,9-dihydro-8H-purin-8-one | 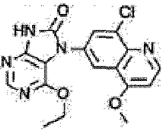 | | 372.1 |
| 140 | 6-ethoxy-7-(4-methoxy-8-methylquinolin-6-yl)-7,9-dihydro-8H-purin-8-one | 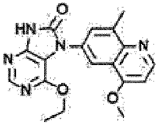 | | 352.1 |

**[Table 15]**

| **Ex. No.** | **IUPAC NAME** | **Structure** | **Salt** | **MS** |
|---|---|---|---|---|
| 141 | 7-ethoxy-1-(7-fluorofuro[3,2-c]quinolin-8-yl)-1,3-dihydro-2H-imidazo[4,5-b]pyridin-2-one | 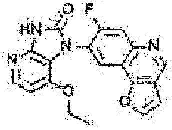 | | 363.0 |
| 142 | 1-(3,4-dihydro-2H-[1,4]dioxepino[2,3-c]quinolin-10-yl)-7-ethoxy-1,3-dihydro-2H-imidazo[4,5-b]pyridin-2-one | 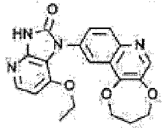 | | 377.0 |
| 143 | 7-ethoxy-1-(6-fluoro-2,3-dihydrofuro[3,2-c]quinolin-8-yl)-1,3-dihydro-2H-imidazo[4,5-b]pyridin-2-one | 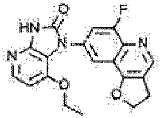 | | 364.9 |
| 144 | 7-ethoxy-1-(thieno[3,2-c]quinolin-8-yl)-1,3-dihydro-2H-imidazo[4,5-b]pyridin-2-one | 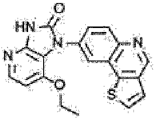 | | 363.1 |
| 145 | 7-((²H₅)ethyloxy)-1-(7-fluoro-2,3-dihydro[1,4]dioxino[2,3-c]quinolin-9-yl)-1,3-dihydro-2H-imidazo[4,5-b]pyridin-2-one | 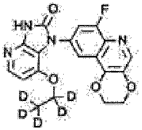 | | 388.1 |
| 146 | 7-((²H₅)ethyloxy)-1-(8-fluoro-2,3-dihydro[1,4]dioxino[2,3-c]quinolin-9-yl)-1,3-dihydro-2H-imidazo[4,5-b]pyridin-2-one | 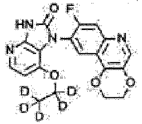 | | 385.9 |
| 147 | 9-(7-ethoxy-2-oxo-2,3-dihydro-1H-imidazo[4,5-b]pyridin-1-yl)-2H-[1,4]oxazino[3,2-c]quinolin-3(4H)-one | 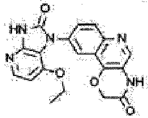 | | 378.1 |
| 148 | 1-(3-benzyl-1,2,3,4-tetrahydrobenzo[c][2,7]-naphthyridin-9-yl)-7-ethoxy-1,3-dihydro-2H-imidazo[4,5-b]pyridin-2-one | 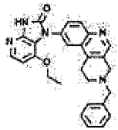 | | 452.2 |
| 149 | 7-ethoxy-1-(furo[3,2-c]quinolin-8-yl)-1,3-dihydro-2H-imidazo[4,5-b]pyridin-2-one | 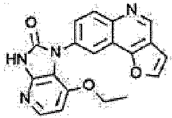 | | 347.1 |
| 150 | 1-(1,1-dioxido-2,3-dihydro-thieno[3,2-c]quinolin-8-yl)-7-ethoxy-1,3-dihydro-2H-imidazo[4,5-b]pyridin-2-one | 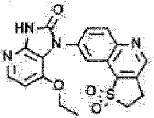 | | 397.0 |

**[Table 16]**

| **Ex. No.** | **IUPAC NAME** | **Structure** | **Salt** | **MS** |
|---|---|---|---|---|
| 151 | N-(6-(7-ethoxy-2-oxo-2,3-dihydro-1H-imidazo[4,5-b]pyridin-1-yl)quinolin-3-yl)acetamide | 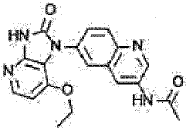 | | 361.9 |
| 152 | 7-ethoxy-1-(1-methyl-2,3-dihydro-1H-pyrrolo[3,2-c]quinolin-8-yl)-1,3-dihydro-2H-imidazo[4,5-b]pyridin-2-one | 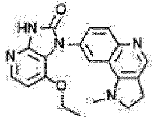 | | 362.1 |
| 153 | 9-(7-ethoxy-2-oxo-2,3-dihydro-1H-imidazo[4,5-b]pyridin-1-yl)-4-methyl-2H-[1,4]oxazino[3,2-c]quinolin-3(4H)-one | 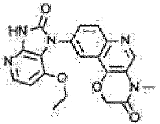 | | 392.0 |
| 154 | 7-ethoxy-1-(3-(methylsulfonyl)-1,2,3,4-tetrahydrobenzo-[c][2,7]naphthyridin-9-yl)-1,3-dihydro-2H-imidazo[4,5-b]pyridin-2-one | 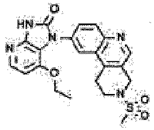 | | 440.1 |
| 155 | 7-ethoxy-1-(1-ethyl-2-methyl-1H-imidazo[4,5-c]quinolin-8-yl)-1,3-dihydro-2H-imidazo[4,5-b]pyridin-2-one | 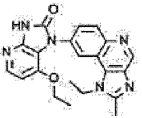 | | 389.1 |
| 156 | 7-ethoxy-1-(4-methoxy-3-(2-oxopyrrolidin-1-yl)quinolin-6-yl)-1,3-dihydro-2H-imidazo[4,5-b]pyridin-2-one | 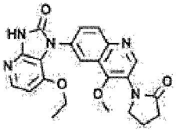 | | 420.1 |
| 157 | 6-(7-ethoxy-2-oxo-2,3-dihydro-1H-imidazo[4,5-b]pyridin-1-yl)-4-methoxyquinoline-3-carboxamide | 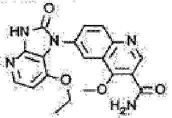 | | 380.2 |
| 158 | 7-(4-hydroxybutoxy)-1-(4-methoxyquinolin-6-yl)-1,3-dihydro-2H-imidazo[4,5-b]pyridin-2-one | 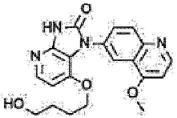 | | 381.1 |
| 159 | 4-((1-(4-methoxyquinolin-6-yl)-2-oxo-2,3-dihydro-1H-imidazo[4,5-b]pyridin-7-yl)oxy)butyl acetate | 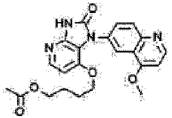 | | 423.1 |
| 160 | 7-ethoxy-1-(8-methoxypyrido[2,3-b]pyrazin-2-yl)-1,3-dihydro-2H-imidazo[4,5-b]pyridin-2-one | 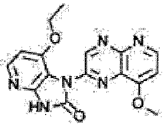 | | 339.1 |

**[Table 17]**

| **Ex. No.** | **IUPAC NAME** | **Structure** | **Salt** | **MS** |
|---|---|---|---|---|
| 161 | 1-(2,3-dihydro[1,4]dioxino[2,3-c]quinolin-9-yl)-7-ethoxy-6-fluoro-1,3-dihydro-2H-imidazo[4,5-b]pyridin-2-one | 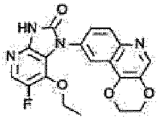 | | 383.0 |
| 162 | 7-ethoxy-1-(4-(ethylamino)quinolin-6-yl)-1,3-dihydro-2H-imidazo[4,5-b]pyridin-2-one | 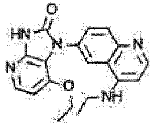 | | 350.0 |
| 163 | 1-(1-ethyl-2,3-dihydro-1H-[1,4]oxazino[2,3-c]quinolin-9-yl)-7-methoxy-1,3-dihydro-2H-imidazo[4,5-b]pyridin-2-one | 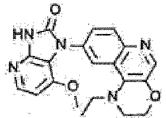 | | 378.2 |
| 164 | 7-ethoxy-1-(4-(4-methylpiperazin-1-yl)quinolin-6-yl)-1,3-dihydro-2H-imidazo[4,5-b]pyridin-2-one | 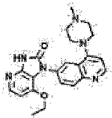 | | 405.1 |
| 165 | 7-ethoxy-1-(4-(3-methyl-piperazin-1-yl)quinolin-6-yl)-1,3-dihydro-2H-imidazo[4,5-b]pyridin-2-one | 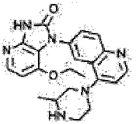 | | 405.1 |
| 166 | 1-(4-(4-(dimethylamino)-piperidin-1-yl)quinolin-6-yl)-7-ethoxy-1,3-dihydro-2H-imidazo[4,5-b]pyridin-2-one | 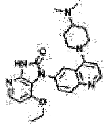 | | 433.1 |
| 167 | 7-ethoxy-1-(4-(3-oxopiperazin-1-yl)quinolin-6-yl)-1,3-dihydro-2H-imidazo[4,5-b]pyridin-2-one | 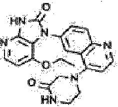 | | 405.0 |
| 168 | 7-ethoxy-1-(4-(3-isobutylpiperazin-1-yl)quinolin-6-yl)-1,3-dihydro-2H-imidazo[4,5-b]pyridin-2-one | 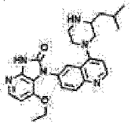 | | 445.0 |
| 169 | 7-ethoxy-1-(4-(4-(2-hydroxyethyl)piperazin-1-yl)quinolin-6-yl)-1,3-dihydro-2H-imidazo[4,5-b]pyridin-2-one | 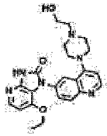 | | 435.1 |
| 170 | 1-(4-(3,8-diazabicyclo-[3.2.1]oct-8-yl)quinolin-6-yl)-7-ethoxy-1,3-dihydro-2H-imidazo[4,5-b]pyridin-2-one | 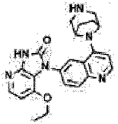 | | 415.1 |

**[Table 18]**

| **Ex. No.** | **IUPAC NAME** | **Structure** | **Salt** | **MS** |
|---|---|---|---|---|
| 171 | 7-ethoxy-1-(4-(4-hydroxypiperidin-1-yl)quinolin-6-yl)-1,3-dihydro-2H-imidazo[4,5-b]pyridin-2-one | 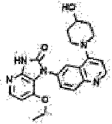 | | 406.1 |
| 172 | 1-(4-((2-(dimethylamino)-ethyl)(methyl)amino)quinolin-6-yl)-7-ethoxy-1,3-dihydro-2H-imidazo[4,5-b]pyridin-2-one | 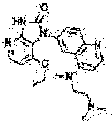 | | 407.1 |
| 173 | 7-ethoxy-1-(4-(methyl(piperidin-4-yl)amino)quinolin-6-yl)-1,3-dihydro-2H-imidazo[4,5-b]pyridin-2-one | 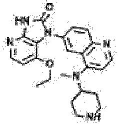 | | 419.1 |
| 174 | 7-((5-((tert-butyl(dimethyl)-silyl)oxy)pentyl)oxy)-1-(4-chloroquinolin-6-yl)-1,3-dihydro-2H-imidazo[4,5-b]pyridin-2-one | 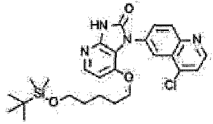 | | 511.0 |
| 175 | 6-(4-((tert-butyl(dimethyl)-silyl)oxy)butoxy)-7-(4-chloroquinolin-6-yl)-7,9-dihydro-8H-purin-8-one | 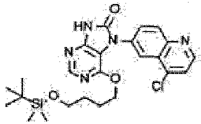 | | 500.0 |
| 176 | 7-(benzyl(ethyl)amino)-1-(4-methoxyquinolin-6-yl)-1,3-dihydro-2H-imidazo[4,5-b]pyridin-2-one | 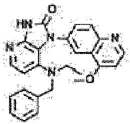 | | 426.1 |
| 177 | 7-ethoxy-1-(4-methoxy-3-(1-methyl-1H-pyrazol-4-yl)quinolin-6-yl)-1,3-dihydro-2H-imidazo[4,5-b]pyridin-2-one | 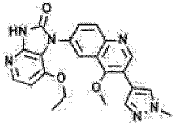 | | 417.0 |
| 178 | 7-ethoxy-1-(4-(pyrrolidin-1-yl)quinolin-6-yl)-1,3-dihydro-2H-imidazo[4,5-b]pyridin-2-one | 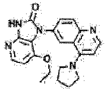 | | 376.1 |
| 179 | 7-ethoxy-1-(4-(1,2,3,6-tetrahydropyridin-4-yl)quinolin-6-yl)-1,3-dihydro-2H-imidazo[4,5-b]pyridin-2-one | 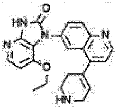 | HCl | 388.1 |
| 180 | 1-(2-aminoquinolin-6-yl)-7-ethoxy-1,3-dihydro-2H-imidazo[4,5-b]pyridin-2-one | 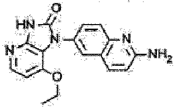 | | 322.0 |

**[Table 19]**

| **Ex. No.** | **IUPAC NAME** | **Structure** | **Salt** | **MS** |
|---|---|---|---|---|
| 181 | 1-(4-chlorocinnolin-6-yl)-7-(4-hydroxybutoxy)-1,3-dihydro-2H-imidazo[4,5-b]pyridin-2-one | 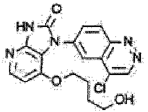 | | 386.0 |
| 182 | tert-butyl 4-(6-(7-ethoxy-2-oxo-2,3-dihydro-1H-imidazo[4,5-b]pyridin-1-yl)quinolin-4-yl)piperidine-1-carboxylate | 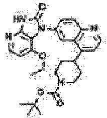 | | 490.1 |
| 183 | 1-(8-fluoro-2,3-dihydro[1,4]-dioxino[2,3-c]quinolin-9-yl)-7-(4-hydroxybutoxy)-1,3-dihydro-2H-imidazo[4,5-b]pyridin-2-one | 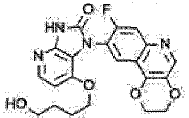 | | 427.0 |
| 184 | 7-ethoxy-1-(3-methyl-2,3-dihydro[1,4]dioxino[2,3-c]quinolin-9-yl)-1,3-dihydro-2H-imidazo[4,5-b]pyridin-2-one | 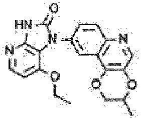 | | 379.1 |
| 185 | 7-ethoxy-1-(4-methoxy-3-(1H-pyrazol-4-yl)quinolin-6-yl)-1,3-dihydro-2H-imidazo[4,5-b]pyridin-2-one | 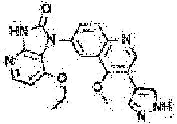 | | 400.9 |
| 186 | 1-(3,4-dimethoxyquinolin-6-yl)-7-ethoxy-1,3-dihydro-2H-imidazo[4,5-b]pyridin-2-one | 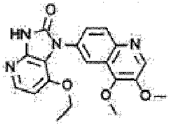 | | 367.0 |
| 187 | 7-ethoxy-1-(4-(piperidin-4-yl)quinolin-6-yl)-1,3-dihydro-2H-imidazo[4,5-b]pyridin-2-one | 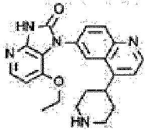 | HCl | 390.1 |
| 188 | 7-ethoxy-1-(8-fluoro-2,3-dihydro[1,4]dioxino[2,3-c]quinolin-9-yl)-2-oxo-1,2-dihydroimidazo[4,5-b]pyridin-3-ide | 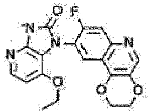 | Na-salt | 383.0 |
| 189 | 1-(8-fluoro-2,3-dihydro[1,4]dioxino[2,3-c]quinolin-9-yl)-7-(3-hydroxypropoxy)-1,3-dihydro-2H-imidazo[4,5-b]pyridin-2-one | 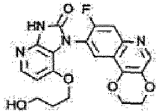 | | 413.0 |
| 190 | 1-(8-fluoro-2,3-dihydro-[1,4]dioxino[2,3-c]quinolin-9-yl)-7-((4-hydroxybutan-2-yl)oxy)-1,3-dihydro-2H-imidazo[4,5-b]pyridin-2-one | 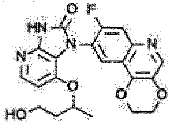 | | 427.1 |

**[Table 20]**

| **Ex. No.** | **IUPAC NAME** | **Structure** | **Salt** | **MS** |
|---|---|---|---|---|
| 191 | 1-(8-fluoro-2,3-dihydro[1,4]dioxino[2,3-c]quinolin-9-yl)-7-(3-hydroxybutoxy)-1,3-dihydro-2H-imidazo[4,5-b]pyridin-2-one | 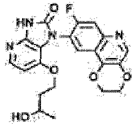 | | 427.1 |
| 192 | 7-ethoxy-1-(4-(3-phenylpiperazin-1-yl)quinolin-6-yl)-1,3-dihydro-2H-imidazo[4,5-b]pyridin-2-one | 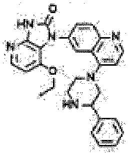 | | 467.1 |
| 193 | 1-(8-cyclopropyl-4-methoxy-3-(1-methyl-1H-pyrazol-4-yl)quinolin-6-yl)-7-ethoxy-1,3-dihydro-2H-imidazo[4,5-b]pyridin-2-one | 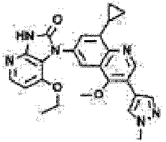 | | 457.1 |
| 194 | 7-(4-hydroxybutoxy)-1-(4-methoxy-3-(1-methyl-1H-pyrazol-4-yl)quinolin-6-yl)-1,3-dihydro-2H-imidazo[4,5-b]pyridin-2-one | 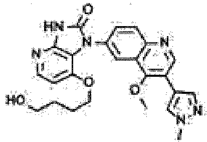 | | 461.1 |
| 195 | 7-ethoxy-1-(3-(1-methyl-1H-pyrazol-4-yl)quinolin-6-yl)-1,3-dihydro-2H-imidazo[4,5-b]pyridin-2-one | 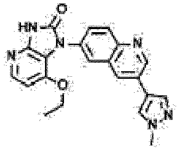 | | 387.0 |
| 196 | 7-(cyclopropylmethoxy)-1-(4-methoxycinnolin-6-yl)-1,3-dihydro-2H-imidazo[4,5-b]pyridin-2-one | 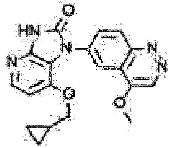 | | 364.2 |
| 197 | 7-(cyclopropylmethoxy)-1-(4-methoxy-3-(1-methyl-1H-pyrazol-4-yl)quinolin-6-yl)-1,3-dihydro-2H-imidazo[4,5-b]pyridin-2-one | 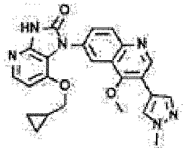 | | 443.1 |
| 198 | 7-ethoxy-1-(7-fluoro-4-methoxycinnolin-6-yl)-1,3-dihydro-2H-imidazo[4,5-b]pyridin-2-one | 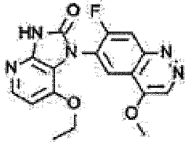 | | 356.1 |

**[Table 21]**

| **Ex. No.** | **IUPAC NAME** | **Structure** | **Salt** | **MS** |
|---|---|---|---|---|
| 199 | 7-ethoxy-1-(4-methoxy-3-(pyridin-3-yl)quinolin-6-yl)-1,3-dihydro-2H-imidazo[4,5-b]pyridin-2-one | 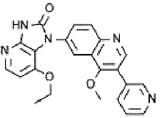 | | 414.1 |
| 200 | 7-ethoxy-1-(4-methoxy-3-(2-methyl-1,3-thiazol-5-yl)quinolin-6-yl)-1,3-dihydro-2H-imidazo[4,5-b]pyridin-2-one | 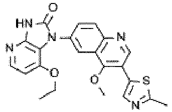 | | 434.1 |
| 201 | 7-(cyclobutyloxy)-1-(3-methoxyquinolin-6-yl)-1,3-dihydro-2H-imidazo[4,5-b]pyridin-2-one | 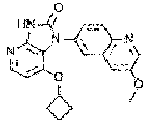 | | 363.2 |
| 202 | 1-(4-(benzylamino)-2-methoxyquinolin-6-yl)-7-ethoxy-1,3-dihydro-2H-imidazo[4,5-b]pyridin-2-one | 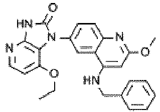 | | 442.2 |
| 203 | 1-(4-amino-2-methoxyquinolin-6-yl)-7-ethoxy-1,3-dihydro-2H-imidazo[4,5-b]pyridin-2-one | 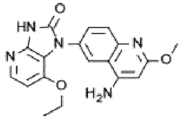 | | 352.2 |
| 204 | 7-(cyclobutyloxy)-1-(4-methoxycinnolin-6-yl)-1,3-dihydro-2H-imidazo[4,5-b]pyridin-2-one | 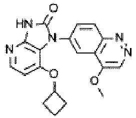 | | 364.2 |
| 205 | 7-(4-hydroxybutoxy)-1-(4-methoxycinnolin-6-yl)-1,3-dihydro-2H-imidazo[4,5-b]pyridin-2-one | 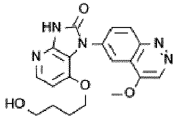 | | 382.1 |
| 206 | 7-ethoxy-1-(4-methoxy-3-(2-thienyl)quinolin-6-yl)-1,3-dihydro-2H-imidazo[4,5-b]pyridin-2-one | 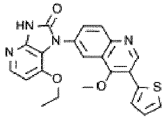 | | 419.1 |
| 207 | 7-ethoxy-1-(3-(2-furyl)-4-methoxyquinolin-6-yl)-1,3-dihydro-2H-imidazo[4,5-b]pyridin-2-one | 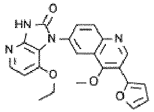 | | 403.1 |
| 208 | 7-ethoxy-1-(3-(3-furyl)-4 methoxyquinolin-6-yl)-1,3-dihydro-2H-imidazo[4,5-b]pyridin-2-one | 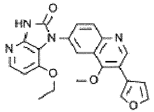 | | 403.1 |

**[Table 22]**

| **Ex. No.** | **IUPAC NAME** | **Structure** | **Salt** | **MS** |
|---|---|---|---|---|
| 209 | 7-ethoxy-1-(4-methoxy-3-(3-thienyl)quinolin-6-yl)-1,3-dihydro-2H-imidazo[4,5-b]pyridin-2-one | 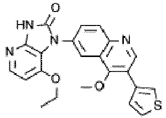 | | 419.1 |
| 210 | 7-ethoxy-1-(3-(1-ethyl-1H-pyrazol-4-yl)-4-methoxyquinolin-6-yl)-1,3-dihydro-2H-imidazo[4,5-b]pyridin-2-one | 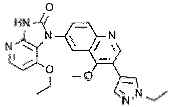 | | 431.1 |
| 211 | 1-(3-(1-(cyclopropylmethyl)-1H-pyrazol-4-yl)-4-methoxyquinolin-6-yl)-7-ethoxy-1,3-dihydro-2H-imidazo[4,5-b]pyridin-2-one | 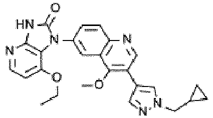 | | 457.2 |
| 212 | 7-ethoxy-1-(4-methoxy-3-(1-(tetrahydro-2H-pyran-4-yl)-1H-pyrazol-4-yl)quinolin-6-yl)-1,3-dihydro-2H-imidazo[4,5-b]pyridin-2-one | 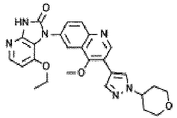 | | 487.2 |
| 213 | 7-ethoxy-1-(4-methoxy-3-(1-(pyridin-3-ylmethyl)-1H-pyrazol-4-yl)quinolin-6-yl)-1,3-dihydro-2H-imidazo[4,5-b]pyridin-2-one | 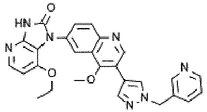 | | 494.2 |
| 214 | 7-ethoxy-1-(4-methoxy-3-(1-(pyridin-2-ylmethyl)-1H-pyrazol-4-yl)quinolin-6-yl)-1,3-dihydro-2H-imidazo[4,5-b]pyridin-2-one | 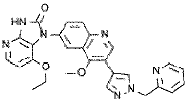 | | 494.1 |
| 215 | 1-(3-(1,3-dimethyl-1H-pyrazol-4-yl)-4-methoxyquinolin-6-yl)-7-ethoxy-1,3-dihydro-2H-imidazo[4,5-b]pyridin-2-one | 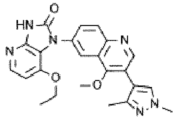 | | 431.1 |
| 216 | 1-(3-(1,5-dimethyl-1H-pyrazol-4-yl)-4-methoxyquinolin-6-yl)-7-ethoxy-1,3-dihydro-2H-imidazo[4,5-b]pyridin-2-one | 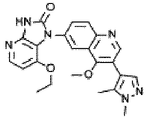 | | 431.1 |
| 217 | 7-ethoxy-1-(4-methoxy-3-(1-methyl-1H-pyrazol-5-yl)quinolin-6-yl)-1,3-dihydro-2H-imidazo[4,5-b]pyridin-2-one | 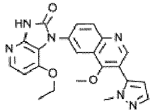 | | 417.1 |
| 218 | 1-(3-(2,4-dimethyl-1,3-thiazol-5-yl)-4-methoxyquinolin-6-yl)-7-ethoxy-1,3-dihydro-2H-imidazo[4,5-b]pyridin-2-one | 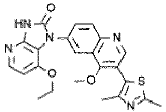 | | 448.1 |

**[Table 23]**

| **Ex. No.** | **IUPAC NAME** | **Structure** | **Salt** | **MS** |
|---|---|---|---|---|
| 219 | 7-ethoxy-1-(4-methoxy-3-phenylquinolin-6-yl)-1,3-dihydro-2H-imidazo[4,5-b]pyridin-2-one | 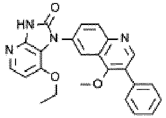 | | 413.1 |
| 220 | 7-ethoxy-1-(4-methoxy-3-(pyridin-4-yl)quinolin-6-yl)-1,3-dihydro-2H-imidazo[4,5-b]pyridin-2-one | 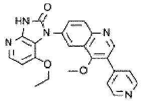 | | 414.1 |
| 221 | 7-ethoxy-1-(4-methoxy-3-(pyrazolo[1,5-a]pyridin-3-yl)quinolin-6-yl)-1,3-dihydro-2H-imidazo[4,5-b]pyridin-2-one | 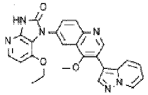 | | 453.1 |
| 222 | 7-ethoxy-1-(3-(isoquinolin-6-yl)-4-methoxyquinolin-6-yl)-1,3-dihydro-2H-imidazo[4,5-b]pyridin-2-one | 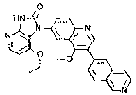 | | 464.1 |
| 223 | 1-(3-(3,6-dihydro-2H-pyran-4-yl)-4-methoxyquinolin-6-yl)-7-ethoxy-1,3-dihydro-2H-imidazo[4,5-b]pyridin-2-one | 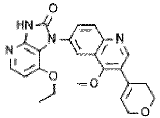 | | 419.1 |
| 224 | 1-(3-cyclopropyl-4-methoxyquinolin-6-yl)-7-ethoxy-1,3-dihydro-2H-imidazo[4,5-b]pyridin-2-one | 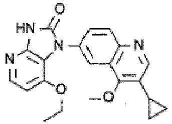 | | 377.1 |
| 225 | 7-ethoxy-1-(3-(1H-indazol-7-yl)-4-methoxyquinolin-6-yl)-1,3-dihydro-2H-imidazo[4,5-b]pyridin-2-one | 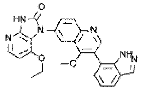 | | 453.1 |
| 226 | 7-ethoxy-1-(3-(2-fluorophenyl)-4-methoxyquinolin-6-yl)-1,3-dihydro-2H-imidazo[4,5-b]pyridin-2-one | 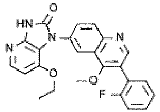 | | 431.1 |
| 227 | 7-ethoxy-1-(3-(3-fluorophenyl)-4-methoxyquinolin-6-yl)-1,3-dihydro-2H-imidazo[4,5-b]pyridin-2-one | 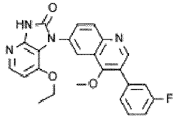 | | 431.1 |
| 228 | 7-ethoxy-1-(3-(4-fluorophenyl)-4-methoxyquinolin-6-yl)-1,3-dihydro-2H-imidazo[4,5-b]pyridin-2-one | 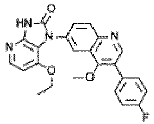 | | 431.1 |

**[Table 24]**

| **Ex. No.** | **IUPAC NAME** | **Structure** | **Salt** | **MS** |
|---|---|---|---|---|
| 229 | 1-(4-methoxycinnolin-6-yl)-7-((trans-4-(morpholin-4-yl)cyclohexyl)oxy)-1,3-dihydro-2H-imidazo[4,5-b]pyridin-2-one | 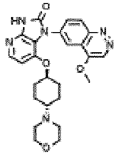 | | 477.2 |
| 230 | 7-ethoxy-1-(3-fluoro-4-methoxyquinolin-6-yl)-1,3-dihydro-2H-imidazo[4,5-b]pyridin-2-one | 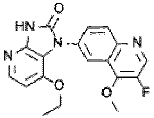 | | 355.0 |

### Experimental Example 1

### PKC θ enzyme inhibition test

PKC θ enzyme inhibitory activities of test compounds were measured by TR-FRET method. First, a test compound diluted with assay buffer (20 mM Tris-HCl (pH 7.5), 5 mM MgAcetate, 0.1 mM CaCl₂, 1 mM DTT, 0.01% Tween20, 0.05% BSA, 10% PKC Activator (Millipore)) was added to 384-well plate at 2 µL each. Then, a solution diluted with assay buffer to 3 pM of PKC θ and 1.2 µM of a fluorescence-labeled peptide substrate (Fluorescein-PKC Substrate, Life Technologies) was added by 2 µL. Then, enzyme reaction was started by adding 2 µL each of ATP solution prepared with assay buffer to 60 µM. After the reaction at room temperature for 1 hr, TR-FRET Dilution Buffer (Life Technologies) prepared to 20 mM EDTA, 0.6 nM terbium-labeled anti-phosphoserine antibody (Life Technologies) was added by 6 µL. After standing at room temperature for 1 hr, fluorescence intensity (excitation wavelength 340 nm, fluorescence wavelength 520 nm, delay time 100 microseconds) was measured by a plate reader, Envision (PerkinElmer). The inhibitory activity of each compound was calculated as relative activity value where fluorescence intensity of a well without enzyme is considered as 100% inhibition. The results thereof are shown in Table 25.

**[Table 25]**

| Example No. | PKC θ inhibitory activity (%, 1 µM) |
|---|---|
| 7 | 100 |
| 11 | 96 |
| 12 | 91 |
| 13 | 90 |
| 15 | 99 |
| 17 | 100 |
| 18 | 97 |
| 27 | 90 |
| 39 | 102 |
| 41 | 97 |
| 55 | 124 |
| 60 | 101 |
| 75 | 101 |
| 92 | 102 |
| 93 | 94 |
| 94 | 101 |
| 177 | 100 |
| 185 | 101 |
| 188 | 101 |
| 204 | 101 |
| 230 | 102 |

### Formulation Example 1 (production of capsule)

| | |
|---|---|
| 1) compound of Example 1 | : 30 mg |
| 2) fine powder cellulose | : 10 mg |
| 3) lactose | : 19 mg |
| 4) magnesium stearate | : 1 mg |
| Total | 60 mg |

1), 2), 3) and 4) are mixed and filled in a gelatin capsule.

### Formulation Example 2 (production of tablet)

| | |
|---|---|
| 1) compound of Example 1 | : 30 g |
| 2) lactose | : 50 g |
| 3) cornstarch | : 15 g |
| 4) calcium carboxymethylcellulose | : 44 g |
| 5) magnesium stearate | : 1 g |
| 1000 tablets total | 140 g |

The total amount of 1), 2) and 3) and 4) (30 g) is kneaded with water, vacuum dried, and sieved. The sieved powder is mixed with 4) (14 g) and 5) (1 g), and the mixture is punched by a tableting machine, whereby 1000 tablets containing 30 mg of the compound of Example 1 per tablet are obtained.

### [Industrial Applicability]

The compound of the present invention has a superior PKC inhibitory action, and is useful as a prophylactic or therapeutic agent for immune diseases, inflammatory diseases and the like.

This application is based on patent application No. 2014-060927 filed in Japan, the contents of which are encompassed in full herein.

## Claims

1. A compound represented by the formula (I): 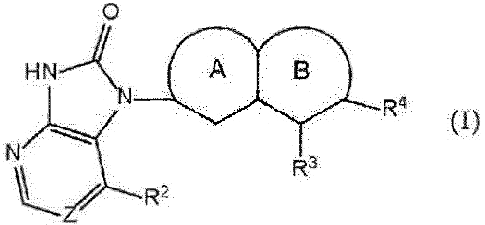 wherein
Z is N or CR¹;
R¹ is a hydrogen atom or a substituent;
R² is a substituent;
R³ is a hydrogen atom or a substituent;
R⁴ is a hydrogen atom or a substituent, or optionally forms an optionally substituted ring together with R³;
ring A is an optionally further substituted benzene ring, or an optionally further substituted 5-membered or 6-membered aromatic heterocycle; and
ring B is an optionally further substituted 5-membered or 6-membered aromatic heterocycle, or a salt thereof.

2. The compound according to claim 1, wherein Z is N or CR¹;
R¹ is a hydrogen atom or a halogen atom;
R² is an optionally substituted C₁₋₆ alkyl group, an optionally substituted C₁₋₆ alkoxy group, an optionally substituted C₃₋₁₀ cycloalkyl group, an optionally substituted C₃₋₁₀ cycloalkyloxy group, an optionally substituted C₆₋₁₄ aryl group, an optionally substituted C₆₋₁₄ aryloxy group, an optionally substituted nonaromatic heterocyclic group, an optionally substituted non-aromatic heterocyclyl-oxy group or an optionally substituted mono- or di-C₁₋₆ alkylamino group;
R³ is a hydrogen atom, a halogen atom, a cyano group, a hydroxy group, an optionally substituted C₁₋₆ alkyl group, an optionally substituted C₂₋₆ alkenyl group, an optionally substituted C₃₋₁₀ cycloalkyl group, an optionally substituted C₁₋₆ alkoxy group, an optionally substituted C₃₋₁₀ cycloalkyloxy group, an optionally substituted amino group, an optionally substituted carbamoyl group, an optionally substituted nonaromatic heterocyclic group or an optionally substituted 7-to 10-membered crosslinked heterocyclic group;
R⁴ is a hydrogen atom, a halogen atom, an optionally substituted C₁₋₆ alkyl group, an optionally substituted C₃₋₁₀ cycloalkyl group, an optionally substituted C₆₋₁₄ aryl group, an optionally substituted C₁₋₆ alkoxy group, an optionally substituted C₁₋₆ alkyl-carbonylamino group, an optionally substituted carbamoyl group, an optionally substituted nonaromatic heterocyclic group or an optionally substituted aromatic heterocyclic group; or
R⁴ and R³ are joined to form a non-aromatic heterocycle or an aromatic heterocycle, each of which is optionally substituted by 1 to 3 substituents selected from an optionally substituted C₁₋₆ alkyl group, an oxo group, an optionally substituted C₁₋₆ alkyl-carbonyl group and an optionally substituted C₁₋₆ alkylsulfonyl group;
ring A is an optionally further substituted benzene ring, or a pyridine ring, a pyrazine ring or a pyridazine ring, each of which is optionally further substituted; and
ring B is a furan ring, an imidazole ring, a pyrazole ring, a pyridine ring, a pyrimidine ring, a pyridazine ring or a pyrazine ring, each of which is optionally further substituted;
or a salt thereof.

3. The compound according to claim 2, wherein Z is N or CR¹;
R¹ is a hydrogen atom, a fluorine atom or a bromine atom;
R² is
(1) a C₁₋₆ alkyl group,
(2) a C₁₋₆ alkoxy group optionally substituted by 1 - 5 substituents selected from a halogen atom, a hydroxy group, a C₁₋₆ alkoxy group, a C₃₋₁₀ cycloalkyl group, a C₁₋₆ alkylcarbonyloxy group and a tri-C₁₋₆ alkylsilyl-oxy group,
(3) a C₃₋₁₀ cycloalkyl group,
(4) a C₃₋₁₀ cycloalkyloxy group optionally substituted by 1 - 5 substituents selected from a di-C₁₋₆ alkylamino group and a nonaromatic heterocyclic group,
(5) a C₆₋₁₄ aryl group,
(6) a C₆₋₁₄ aryloxy group,
(7) a nonaromatic heterocyclic group,
(8) a non-aromatic heterocyclyl-oxy group optionally substituted by a C₁₋₆ alkyl group, or
(9) a mono- or di-C₁₋₆ alkylamino group optionally substituted by a C₆₋₁₄ aryl group;
R³ is
(1) a hydrogen atom,
(2) a halogen atom,
(3) a cyano group,
(4) a hydroxy group,
(5) a C₁₋₆ alkyl group optionally substituted by 1 to 3 substituents selected from a halogen atom, a C₃₋₁₀ cycloalkyl group and an amino group,
(6) a C₂₋₆ alkenyl group,
(7) a C₃₋₁₀ cycloalkyl group,
(8) a C₁₋₆ alkoxy group optionally substituted by 1 to 3 substituents selected from a halogen atom, a C₁₋₆ alkoxy group and an amino group,
(9) a C₃₋₁₀ cycloalkyloxy group,
(10) an amino group,
(11) a mono- or di-C₁₋₆ alkylamino group optionally substituted by a mono- or di-C₁₋₆ alkylamino group,
(12) a (C₁₋₆ alkyl)(3- to 14-membered non-aromatic heterocyclyl)amino group,
(13) a mono- or di-C₇₋₁₆ aralkylamino group,
(14) a carbamoyl group,
(15) a nonaromatic heterocyclic group optionally substituted by 1 to 3 substituents selected from a hydroxy group, an oxo group, a C₁₋₆ alkyl group optionally substituted by a hydroxy group, a C₆₋₁₄ aryl group, a mono- or di-C₁₋₆ alkylamino group and a C₁₋₆ alkoxy-carbonyl group, or
(16) a 7- to 10-membered crosslinked heterocyclic group;
R⁴ is
(1) a hydrogen atom,
(2) a halogen atom,
(3) a C₁₋₆ alkyl group,
(4) a C₃₋₁₀ cycloalkyl group,
(5) a C₆₋₁₄ aryl group optionally substituted by 1 to 3 halogen atoms,
(6) a C₁₋₆ alkoxy group optionally substituted by 1 to 3 substituents selected from a halogen atom and a C₁₋₆ alkoxy group,
(7) a C₁₋₆ alkyl-carbonylamino group,
(8) a carbamoyl group,
(9) a nonaromatic heterocyclic group optionally substituted by an oxo group, or
(10) an aromatic heterocyclic group optionally substituted by 1 to 3 substituents selected from (i) a C₁₋₆ alkyl group optionally substituted by 1 to 3 substituents selected from a C₃₋₁₀ cycloalkyl group and an aromatic heterocyclic group, and (ii) a nonaromatic heterocyclic group; or
R⁴ and R³ are joined to form
(1) a non-aromatic heterocycle optionally substituted by 1 to 3 substituents selected from a C₁₋₆ alkyl group optionally substituted by a C₆₋₁₄ aryl group, an oxo group, a C₁₋₆ alkyl-carbonyl group and a C₁₋₆ alkylsulfonyl group, or
(2) an aromatic heterocycle optionally substituted by 1 to 3 substituents selected from a C₁₋₆ alkyl group optionally substituted by a hydroxy group;
ring A is a benzene ring optionally further substituted by 1 to 3 substituents selected from a halogen atom, a C₁₋₆ alkyl group and a C₃₋₁₀ cycloalkyl group, or a pyridine ring, a pyrazine ring or a pyridazine ring; and
ring B is a pyridine ring optionally further substituted by 1 or 2 substituents selected from a halogen atom, a hydroxy group, a C₁₋₆ alkyl group, a C₁₋₆ alkoxy group and an amino group, or a furan ring, an imidazole ring, a pyrazole ring, a pyrimidine ring, a pyridazine ring or a pyrazine ring;
or a salt thereof.

4. 7-Ethoxy-1-(4-methoxyquinolin-6-yl)-1,3-dihydro-2H-imidazo[4,5-b]pyridin-2-one or a salt thereof.

5. 7-Ethoxy-1-(8-fluoro-2.3-dihydro[1,4]dioxino[2,3-c]quinolin-9-yl)-1,3-dihydro-2H-imidazo[4,5-b]pyridin-2-one or a salt thereof.

6. 7-Ethoxy-1-(4-methoxy-3-(1-methyl-1H-pyrazol-4-yl)quinolin-6-yl)-1,3-dihydro-2H-imidazo[4,5-b]pyridin-2-one or a salt thereof.

7. A medicament comprising the compound according to claim 1 or a salt thereof.

8. The medicament according to claim 7, which is a protein kinase C inhibitor.

9. The medicament according to claim 7, which is a prophylactic or therapeutic agent for immune diseases and/or inflammatory diseases.

10. The medicament according to claim 7, which is a prophylactic or therapeutic agent for an inflammatory bowel disease, psoriasis or atopic dermatitis.

11. The compound according to claim 1 or a salt thereof for use in the prophylaxis or treatment of immune diseases and/or inflammatory diseases.

12. The compound according to claim 1 or a salt thereof for use in the prophylaxis or treatment of an inflammatory bowel disease, psoriasis or atopic dermatitis.

13. A method of inhibiting protein kinase C in a mammal, comprising administering an effective amount of the compound according to claim 1 or a salt thereof to the mammal.

14. A method for the prophylaxis or treatment of an immune disease and/or an inflammatory disease in a mammal, comprising administering an effective amount of the compound according to claim 1 or a salt thereof to the mammal.

15. A method for the prophylaxis or treatment of an inflammatory bowel disease, psoriasis or atopic dermatitis in a mammal, comprising administering an effective amount of the compound according to claim 1 or a salt thereof to the mammal.

16. Use of the compound according to claim 1 or a salt thereof in the production of a prophylactic or therapeutic agent for an immune disease and/or an inflammatory disease.

17. Use of the compound according to claim 1 or a salt thereof in the production of a prophylactic or therapeutic agent for an inflammatory bowel disease, psoriasis or atopic dermatitis.
